# EUROPEAN PATENT APPLICATION

(11) **EP 4 599 828 A1**
(43) Date of publication of application: **13.08.2025**
(21) Application number: 23875239.8
(22) Date of filing: 05.10.2023
(51) Int. Cl.: A61K 31/351, A61P 13/12, A61P 3/10

(54) **PHARMACEUTICAL COMPOSITION FOR PREVENTION OR TREATMENT OF NEPHROPATHY AND/OR DIABETES MELLITUS, COMPRISING ENAVOGLIFLOZIN**

(30) Priority: 05.10.2022 KR 20220127340
(71) Applicant: Daewoong Pharmaceutical Co., Ltd., Hwaseong-si, Gyenoggi-do 18623 (KR)
(72) Inventor: SONG, Hwa Rang, Seoul 06170 (KR); KIM, Young Hee, Seoul 06170 (KR); NAH, Jae Jin, Seoul 06170 (KR); HUH, Wan, Namyangju-si Gyeonggi-do 12111 (KR); CHO, Seung Ah, Seoul 07972 (KR); PARK, Mi Hee, Seoul 06762 (KR); LEE, Si Eun, Seoul 05245 (KR); KIM, Su Young, Seoul 05501 (KR); CHO, Bo Young, Seoul 05504 (KR); CHO, Jae Min, Seoul 06170 (KR); CHO, Seong In, Seoul 06170 (KR); CHOI, Ji-Soo, Yongin-si Gyeonggi-do 17028 (KR); JI, Hye Young, Yongin-si Gyeonggi-do 17028 (KR)
(74) Representative: Plasseraud IP
(86) International application number: PCT/KR2023/015349
(87) International publication number: WO 2024/076177

(57) **Abstract**

**The present invention relates to a pharmaceutical composition comprising enavogliflozin as an active ingredient for the prevention or treatment of diabetes and/or renal diseases in patients who have or are at risk of diabetes and/or renal diseases.**

## Description

### [Technical Field]

The present invention relates to a pharmaceutical composition for preventing or treating diabetes and/or nephropathy in patients who have or are at risk of nephropathy and/or diabetes, which includes enavogliflozin as an active ingredient.

### [Background Art]

Sodium glucose cotransporter 2 (SGLT-2) is a transporter that, along with sodium glucose cotransporter 1 (SGLT-1), is responsible for excessive blood sugar reabsorption in the kidneys, with SGLT-2 playing the major roles. Therefore, when an SGLT-2 inhibitor blocks the SGLT-2 transporter, the amount of blood sugar excreted in the urine increases, which ultimately lowers blood sugar levels and releases calories from the blood sugar, resulting in body weight loss.

One of the drugs developed as an SGLT-2 inhibitor that can be useful as a therapeutic agent for type 2 diabetes due to such effects is enavogliflozin represented by Chemical Formula 1 below, which is disclosed in Korean Unexamined Patent Application Publication No. 2014-0022086 (Patent Document 1).

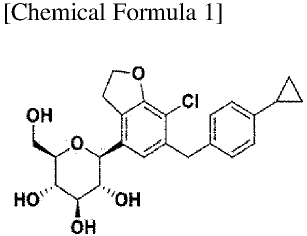

Name of compound: (2S,3R,4R,5S,6R)-2-(7-chloro-6(4-cyclopropylbenzyl)-2,3-dihydrobenzofuran-4-yl)-6-(hydroxymethyl)tetrahydro-2H-pyran-3,4,5-triol

Enavogliflozin (DWP16001) is a drug currently in Phase 3 clinical trials, and the Phase 2 clinical trial confirmed that enavogliflozin had a statistically significant blood glucoselowering effect in all groups treated with 0.1 mg, 0.3 mg, and 0.5 mg of enavogliflozin as compared to a placebo.

It is estimated that enavogliflozin had an excellent effect on urinary glucose secretion (glucose excreted in the urine) at a very low dose, 1/100^{th} of the dose of a drug in the same class.

However, it has not been reported that enavogliflozin has a therapeutic effect on nephropathy or is useful for treating diabetes in patients with or at risk of nephropathy.

### [Disclosure]

### [Technical Problem]

The present inventors administered enavogliflozin to patients who had or were at risk of diabetes and/or nephropathy in order to find a novel method for treating the patients and confirmed that enavogliflozin can prevent or treat nephropathy, thus completing the present invention.

Therefore, the present invention is directed to providing a pharmaceutical composition for preventing or treating nephropathy and/or diabetes in patients who have or are at risk of diabetes and/or nephropathy, which includes enavogliflozin as an active ingredient.

### [Technical Solution]

The present invention provides a pharmaceutical composition for preventing or treating nephropathy and/or diabetes in patients who have or are at risk of diabetes and/or nephropathy, which includes enavogliflozin as an active ingredient.

Hereinafter, the present invention will be described in detail.

The present invention provides a use of enavogliflozin for preventing or treating nephropathy, a pharmaceutical composition for preventing or treating nephropathy, and a method of preventing or treating nephropathy, which includes administering an effective amount of enavogliflozin to a subject in need thereof.

In the present invention, nephropathy may be, but is not limited to, a disease selected from diabetic nephropathy, renal failure, chronic kidney disease, glomerulonephritis, and proteinuria.

In one embodiment of the present invention, the pharmaceutical composition may be for reducing the risk of sustained decline in estimated glomerular filtration rate (eGFR), progression to end-stage kidney disease, cardiovascular death, and kidney-related death in patients with chronic kidney disease.

In another embodiment of the present invention, the nephropathy may be diabetic nephropathy.

In one embodiment, the pharmaceutical composition may be administered to a patient with diabetes or a pre-diabetic patient.

The pharmaceutical composition including enavogliflozin can be used in the form of monotherapy or combination therapy.

Enavogliflozin alone is sufficient to prevent or treat diabetes and/or nephropathy. However, for various purposes, therapeutic agents for diabetes or nephropathy are co-administered, and since these agents can exhibit complementary or synergistic effects when co-administered, the pharmaceutical composition including enavogliflozin can be co-administered with another therapeutic agent for diabetes or nephropathy.

In one embodiment, the pharmaceutical composition including enavogliflozin may be co-administered with metformin.

In another embodiment, the pharmaceutical composition including enavogliflozin may be co-administered with a DPP4 inhibitor.

For example, the DPP4 inhibitor may be, but is not limited to, gemigliptin.

In another embodiment, the combination therapy may be co-administered with two or more different drugs. For example, the pharmaceutical composition including enavogliflozin may be co-administered with metformin and a DPP4 inhibitor.

Alternatively, the pharmaceutical composition including enavogliflozin may be co-administered with a sulfonylurea-based insulin secretagogue.

In another embodiment, the pharmaceutical composition including enavogliflozin may be co-administered with insulin.

The present invention also provides a use of enavogliflozin for preventing or treating diabetes in a nephropathy patient, a pharmaceutical composition for preventing or treating diabetes in a nephropathy patient, which includes enavogliflozin, and a method of preventing or treating diabetes in a nephropathy patient, which includes administering an effective amount of enavogliflozin to a subject in need thereof.

In the present invention, nephropathy may include a disease selected from diabetic nephropathy, renal failure, chronic kidney disease, glomerulonephritis, and proteinuria, but the present invention is not limited thereto.

In another embodiment of the present invention, the nephropathy may be diabetic nephropathy.

In one embodiment, the pharmaceutical composition may be administered to a patient with diabetes or a prediabetic patient.

The pharmaceutical composition including enavogliflozin can be administered alone or in combination.

Enavogliflozin alone is sufficient to prevent or treat diabetes and/or nephropathy. However, for various purposes, therapeutic agents for diabetes or nephropathy are co-administered, and since these agents can exhibit complementary or synergistic effects when co-administered, the pharmaceutical composition including enavogliflozin can be co-administered with another therapeutic agent for diabetes or nephropathy.

In one embodiment, the pharmaceutical composition including enavogliflozin may be co-administered with metformin.

In another embodiment, the pharmaceutical composition including enavogliflozin may be co-administered with a DPP4 inhibitor.

For example, the DPP4 inhibitor may be gemigliptin, but the present invention is not limited thereto.

In another embodiment, the combination therapy may be administration in combination with two or more different drugs. For example, the pharmaceutical composition including enavogliflozin may be co-administered with metformin and a DPP4 inhibitor.

Alternatively, the pharmaceutical composition including enavogliflozin may be co-administered with a sulfonylurea-based insulin secretagogue.

In another embodiment, the pharmaceutical composition including enavogliflozin may be co-administered with insulin.

The term "prevention" used in the specification refers to all actions of inhibiting or delaying the occurrence of nephropathy and/or diabetes by the administration of the pharmaceutical composition according to the present invention.

The term "treatment" used in the specification refers to all actions involved in improving or beneficially changing nephropathy and/or diabetes by the administration of the pharmaceutical composition according to the present invention.

The dosage of enavogliflozin, which can be used for the prevention or treatment of diabetes and/or kidney disease in patients who have or are at risk of diabetes and/or kidney disease, is not specifically limited and can be appropriately adjusted based on the severity of a patient's disease, body weight, age, sex, and the presence or absence of other complications.

The duration of enavogliflozin administration can be appropriately adjusted by a clinician based on the preventive or therapeutic effect on diabetes and/or kidney disease in patients who have or are at risk of diabetes and/or kidney disease due to the administration of enavogliflozin.

In one embodiment of the present invention, the treatment method may be to administer a pharmaceutical composition for preventing or treating diabetes and/or kidney disease in combination with metformin and/or gemigliptin.

The method of treating diabetes and/or kidney disease uses a pharmaceutical composition for preventing or treating diabetes and/or kidney disease, and thus any overlapping content between these two is omitted to avoid excessive description in the specification.

Enavogliflozin used as an active ingredient in the present invention may be synthesized through the known related literatures. In the present invention, enavogliflozin may be crystalline or amorphous. For example, enavogliflozin may be crystalline form A, B, C, D, or E of enavogliflozin, or amorphous enavogliflozin, which are reported to have the following X-ray diffraction spectra according to Korean Unexamined Patent Application Publication No. 2017-0142904 or Korean Patent Application No. 2022-0123673.

Crystalline form A: the crystalline form with an X-ray diffraction (XRD) spectrum having peaks at 2[θ] values selected from 6.2° ± 0.2°, 7.2° ± 0.2°, 8.8° ± 0.2°, 17.6° ± 0.2°, 19.0° ± 0.2°, 22.5° ± 0.2°, and 25.1° ± 0.2°

Crystalline form B: the crystalline form with an X-ray diffraction (XRD) spectrum having peaks at 2[θ] values selected from 7.0° ± 0.2°, 14.9° ± 0.2°, 17.7° ± 0.2°, 18.8° ± 0.2°, 20.6° ± 0.2°, 21.8° ± 0.2°, and 23.5° ± 0.2°

Crystalline form C: the crystalline form with an X-ray diffraction (XRD) spectrum having peaks at 2[θ] values selected from 5.6° ± 0.2°, 7.3° ± 0.2°, 15.7° ± 0.2°, 17.2° ± 0.2°, 18.9° ± 0.2°, 21.2° ± 0.2°, and 21.9° ± 0.2°

Crystalline form D: the crystalline form with an X-ray diffraction (XRD) spectrum having peaks at 2[θ] values selected from 5.5° ± 0.2°, 7.2° ± 0.2°, 15.3° ± 0.2°, 17.2° ± 0.2°, 17.6° ± 0.2°, 18.9° ± 0.2°, and 21.1° ± 0.2°

Crystalline form E: the crystalline form with an X-ray diffraction (XRD) spectrum having peaks at 2[θ] values selected from 4.93°±0.2°, 6.12°±0.2°, 7.43°± 0.2°, 8.89°± 0.2°, 9.74°± 0.2°, 14.79°± 0.2°, 15.79°± 0.2°, 16.11°± 0.2°, 19.79°± 0.2° 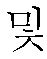 22.83°±0.2°

Each of the crystalline forms A, B, C, and D is specified by the X-ray diffraction spectra having four or more, e.g., 4, 5, 6, 7, 8 or more, peaks at the presented 2[θ] values.

The pharmaceutical composition including enavogliflozin according to the present invention may have the composition of the pharmaceutical composition of PCT/KR2022/014640.

For example, the pharmaceutical composition of the present invention may be a pharmaceutical composition which includes a compound of Chemical Formula 1 or a pharmaceutically acceptable salt thereof as an active ingredient, an excipient, a disintegrant, and a binder, and the average particle size of the compound of Chemical Formula 1 may be 15 µm or less.

In an exemplary embodiment of the present invention, the average particle size of enavogliflozin may be 15 µm or less, and preferably, 10 µm or less.

When the average particle size of enavogliflozin is greater than 15 µm, the 5-minute dissolution rate is very low at less than 40% of the total content of enavogliflozin, and the 30-minute dissolution rate is also less than 80% thereof, indicating that the final dissolution rate was inadequate.

When the micronization of drug particles is required, the drug particles may be crushed using a conventional mill that can micronize particles such as a Z-mill, a hammer mill, a ball mill, or a fluid energy mill. In addition, the drug particle size may be divided by a size classification method such as sieving using a sieve, or air current classification. Methods of controlling a desired particle size are well known in the art (e.g., refer to [Pharmaceutical dosage forms: volume 2, 2nd edition, Ed.: H. A. Lieberman, L. Lachman, J. B. Schwartz (Chapter 3: SIZE REDUCTION)].

In the specification, the particle size of a drug is expressed on the basis of a particle size distribution such as D(X) = Y (where X and Y are positive numbers). D(X) = Y means that when the particle size distribution of a drug obtained by measuring the particle diameter of any drug in the preparation is represented by a cumulative curve, the particle diameter at the point where the particle size becomes X% while being accumulated in the order of decreasing particle size (% is calculated on the basis of a number, volume, or weight) is Y. For example, D(10) represents the particle diameter at the point where the particle size becomes 10% while being accumulated in the order of decreasing particle size, D(50) represents the particle diameter at the point where the particle size becomes 50% while being accumulated in the order of decreasing particle size, and D(90) represents the particle diameter at the point where the particle size becomes 90% while being accumulated in the order of decreasing particle size.

Whether the particle size distribution D(X) is based on number, volume, or weight as a percentage of the total cumulative particles depends on the method used to measure the particle size distribution. Methods for measuring particle size distribution and types of % associated therewith are known in the art. For example, when measuring particle size distribution by the well-known laser diffraction method, the X value in D(X) represents the percentage calculated by the average volume. It is well known by those of ordinary skill in the art that the particle size distribution measurement result obtained by a particular method can be correlated with those obtained from other techniques based on an experience obtained from routine experiments. For example, laser diffraction is sensitive to the particle volume and provides a volume average particle size, which is equivalent to a weight average particle size at constant density.

In the present invention, the measurement of the particle size distribution of drug particles may be performed using a commercially available device based on the laser diffraction/scattering method based on the Mie theory. For example, the particle size distribution is measured using a commercial device such as the Mastersizer laser diffraction device (Malvern Instruments). In this device, a helium-neon laser beam and a blue lightemitting diode are applied on the particles, causing scattering and thus producing a light scattering pattern on a detector. By interpreting the light scattering pattern according to the Mie theory, the particle diameter distribution is obtained. The measurement method can be either dry or wet.

For reference, in an example of the present invention, the particle size of the drug was measured by volume average particle size using laser diffraction.

In one embodiment of the present invention, the compound of Chemical Formula 1 may be included at less than 1 part by weight with respect to 100 parts by weight of the total pharmaceutical composition.

The appropriate daily dose of enavogliflozin identified during clinical trials is 0.1 to 0.5 mg, and when the pharmaceutical composition is formulated as a unit dosage form, the content of the active ingredient in the pharmaceutical composition may be 0.1 to 0.5 mg.

The pharmaceutical composition according to the present invention includes pharmaceutically acceptable additives other than the compound of Chemical Formula 1 as an active ingredient.

The pharmaceutical composition of the present invention includes an excipient, a disintegrant, and a binder.

Examples of the excipient include lactose (including a hydrate), dextrin, mannitol, sorbitol, starch, microcrystalline cellulose (e.g., Celphere^{™}), silicified microcrystalline cellulose (e.g., Prosolv^{™}), calcium phosphate hydrate, anhydrous calcium phosphate, calcium carbonate, a saccharide, or a mixture thereof. In one embodiment of the present invention, a preferable excipient is microcrystalline cellulose.

Examples of the disintegrant include crospovidone, croscarmellose sodium, sodium starch glycolate, or low-substituted hydroxypropyl cellulose. In one embodiment of the present invention, a preferable excipient is croscarmellose sodium.

Examples of binders include polyvinylpyrrolidone, povidone, gelatin, starch, sucrose, methylcellulose, ethylcellulose, hydroxyethylcellulose, hydroxypropylcellulose, hydroxypropylalkylcelluloses (e.g., hydroxypropylmethylcellulose), and a mixture thereof. In one embodiment of the present invention, a preferable binder is hydroxypropylcellulose.

Examples of additives other than those listed above include a lubricant and a coloring agent.

The lubricant may be stearic acid, a stearate (e.g., magnesium stearate), light anhydrous silicic acid, talc, corn starch, carnauba wax, magnesium silicate, synthetic aluminum silicate, hydrogenated oil, white lead, titanium oxide, microcrystalline cellulose, Macrogol 4000 or 6000, isopropyl myristate, calcium hydrogen phosphate, or a mixture thereof.

In one embodiment of the present invention, the excipient may be included at 80 to 95 parts by weight with respect to 100 parts by weight of the total pharmaceutical composition.

In one embodiment of the present invention, the disintegrant may be included at 2 to 8 parts by weight with respect to 100 parts by weight of the total pharmaceutical composition. When the disintegrant is included at less than 2 parts by weight with respect to 100 parts by weight of the total pharmaceutical composition, the initial disintegration ability may be low and thus the dissolution rate may be delayed, affecting the Cmax in the body. In addition, when the disintegrant is included at more than 8 parts by weight with respect to 100 parts by weight of the total pharmaceutical composition, as the amount of disintegrant in the postmixed portion increases, the overall flowability of granules may decrease.

In one embodiment of the present invention, the binder may be included at 3 to 10 parts by weight with respect to 100 parts by weight of the total pharmaceutical composition. When the binder is included at less than 3 parts by weight with respect to 100 parts by weight of the total pharmaceutical composition, it may be difficult to form and maintain appropriate dry granules, affecting the maintenance of homogeneous dispersion of the main ingredient and the flowability of granules due to the generation of fine particles. In addition, when the binder is included at more than 10 parts by weight with respect to 100 parts by weight of the total pharmaceutical composition, granules with a strong binding strength are formed, affecting the solubility of the initially disintegrated granular particles during dissolution and also affecting the Cmax in the body.

The pharmaceutical composition according to the present invention may be an immediate-release formulation.

In one embodiment of the present invention, the pharmaceutical composition may have a dissolution rate after 5 minutes of 50% or more, and preferably, 60% or more of the total content of the active ingredient.

In one embodiment of the present invention, the pharmaceutical composition may have a dissolution rate after 15 minutes of 80% or more, and preferably, 80% or more of the total content of the active ingredient.

In one embodiment of the present invention, the pharmaceutical composition may have a dissolution rate after 30 minutes of 85% or more, and preferably, 90% or more of the total content of the active ingredient.

The dissolution rate of the active ingredient in the pharmaceutical composition affects the maximum blood concentration (Cmax) and the blood concentration-area under the blood concentration-time curve (AUC) when administering the drug, that is to say, in order to achieve appropriate Cmax and AUC, it is important to adjust the dissolution rate of the pharmaceutical composition. Since enavogliflozin has a Tmax of 1 to 2 hours, the drug absorption rate in the stomach is considered important. The above dissolution rate was measured under the condition of the dissolved solution 1.2 in accordance of the dissolution test method 2 of the Korean Pharmacopoeia (paddle method). For specific conditions, refer to the following experimental examples.

The present invention also provides a pharmaceutical composition including a granule mixture of premixed granules, which include the compound of Chemical Formula 1 or a pharmaceutically acceptable salt thereof, and a postmixed portion.

During the research on the formulation of the compound of Chemical Formula 1, the present inventors confirmed that preparing granules and formulating them into tablets is advantageous in terms of drug content uniformity and the uniformity of dosage units.

In the pharmaceutical composition, the granules are prepared by mixing premixed granules and a postmixed portion.

The premixed granules may include the compound of Chemical Formula 1 or a pharmaceutically acceptable salt thereof, an excipient, a binder, and a lubricant.

In addition, the postmixed portion may include an excipient, a disintegrant, and a lubricant.

Descriptions of the excipient, the binder, the disintegrant, and the lubricant are the same as those described above, and are therefore omitted to avoid duplicate description.

In one embodiment of the present invention, each of the premixed granules and the postmixed portion may include an excipient. More specifically, each of the premixed granules and the postmixed portion may include microcrystalline cellulose as the excipient.

According to the following examples, the microcrystalline cellulose included in the premixed granules and the postmixed portion was found to affect the content uniformity of a drug depending on its particle size and bulk density.

In one embodiment of the present invention, the particle size of the microcrystalline cellulose in the premixed granules may be 130 µm or less, and preferably, 60 to 130 µm. The bulk density of the microcrystalline cellulose in the premixed granules may be 0.26 to 0.33. When the particle size and bulk density of the microcrystalline cellulose in the premixed granules are the same as the above conditions, it is possible to obtain a formulation with low standard deviation (SD) of content uniformity. When the particle size of the microcrystalline cellulose in the premixed granules is 130 µm or less, all of the content uniformity of the premixed granules, the content uniformity of the final granules, and the formulation uniformity showed good levels, and Carr's index values, which indicate the physical properties of the final granules, were also good, confirming that the flowability of the formulation was also excellent. On the other hand, when the particle size of the microcrystalline cellulose in the premixed granules exceeded 130 µm, neither the content uniformity of the premixed granules nor the content uniformity of the final granules was suitable due to large variations, and the formulation uniformity was also found to be poor.

Meanwhile, the particle size of the microcrystalline cellulose in the postmixed portion may be 130 µm or more, and preferably, 130 to 250 µm. The bulk density of the excipient in the postmixed portion may be 0.28 to 0.37. When the particle size of the microcrystalline cellulose in the postmixed portion is less than 130 µm, the Carr's index value, which indicates the physical properties of the final granules, was not appropriate and the granule flowability was poor.

When comparing the microcrystalline cellulose in the premixed granules and the microcrystalline cellulose in the postmixed portion, it is preferable that the particle size of the microcrystalline cellulose in the premixed granules be small, whereas it is preferable that the particle size of the microcrystalline cellulose in the postmixed portion be relatively larger than the particle size of the microcrystalline cellulose in the premixed granules.

According to the following examples, not only the particle sizes of the microcrystalline cellulose in the premixed granules and the microcrystalline cellulose in the postmixed portion but also the weight ratio of the excipient in the premixed granules and the excipient in the postmixed portion is confirmed to affect the content uniformity of a drug.

In one embodiment of the present invention, the weight ratio of the excipient in the premixed granules and the excipient in the postmixed portion may be 4:1 to 1:1. As the proportion of the microcrystalline cellulose in the postmixed portion increases, the flowability of the granules is improved, but the content variation increases, and thus it was found that it is preferable to adjust the weight ratio within the appropriate range.

Meanwhile, in the pharmaceutical composition according to the present invention, the binder may be one or more selected from the group consisting of hydroxypropyl cellulose, povidone, copovidone, and hypromellose.

In one embodiment of the present invention, the binder may be hydroxypropyl cellulose,and have a weight average molecular weight of less than 200,000. When hydroxypropyl cellulose with a weight average molecular weight of 200,000 or more is used, both the 5-minute and 30-minute dissolution rates are low, which is not preferable in terms of bioavailability.

Meanwhile, regarding the Carr's index used as a measure of flowability in formulation, the Carr's index of the granules is preferably 21 to 25.

In the pharmaceutical composition of the present invention, the granules may be dry granules, but the present invention is not limited thereto. In another embodiment, the granules may be wet granules.

In the present invention, the pharmaceutical composition may have a dosage form for oral administration, such as a tablet or a capsule. In one embodiment of the present invention, the pharmaceutical composition may have a tablet formulation.

In an exemplary embodiment, the pharmaceutical composition may include 0.3 to 0.5 mg of the compound of Chemical Formula 1.

The pharmaceutical composition according to the present invention may be orally administered once a day, but the present invention is not limited thereto.

### [Advantageous Effects]

A pharmaceutical composition of the present invention which includes enavogliflozin as an active ingredient can be useful for preventing or treating nephropathy, or preventing or treating diabetes in patients with nephropathy.

### [Description of Drawings]

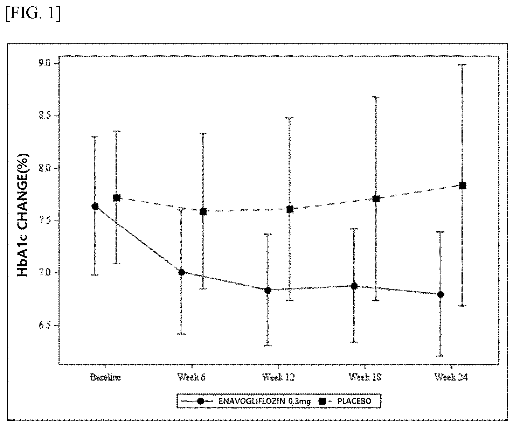

FIG. 1 shows results of measuring HbAlc at 6, 12, 18, and 24 weeks after monotherapy with enavogliflozin or a placebo of enavogliflozin for 24 weeks to patients with type 2 diabetes. The test group was administered enavogliflozin at a dose of 0.3 mg once a day for 24 weeks, and a control was administered an enavogliflozin placebo once a day for 24 weeks. The bar graph represents the mean of each variable, and the error bars represent S.D. The p value of less than 0.05 was considered statistically significant.

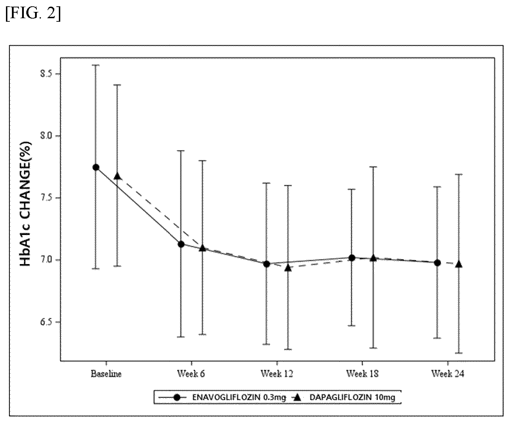

FIG. 2 shows results of measuring HbAlc at 6, 12, 18, and 24 weeks after administration of enavogliflozin or dapagliflozin for 24 weeks in patients with type 2 diabetes whose glycemic control was inadequate with metformin. The test group was administered enavogliflozin at a dose of 0.3 mg or a dapagliflozin placebo at a dose of 10 mg, and the control was administered an enavogliflozin placebo at a dose of 0.3 mg or dapagliflozin at a dose of 10 mg once a day for 24 weeks. Here, metformin was taken at the same dose as before. The bar graph represents the mean of each variable, and the error bars represent S.D. The p value of less than 0.05 was considered statistically significant.

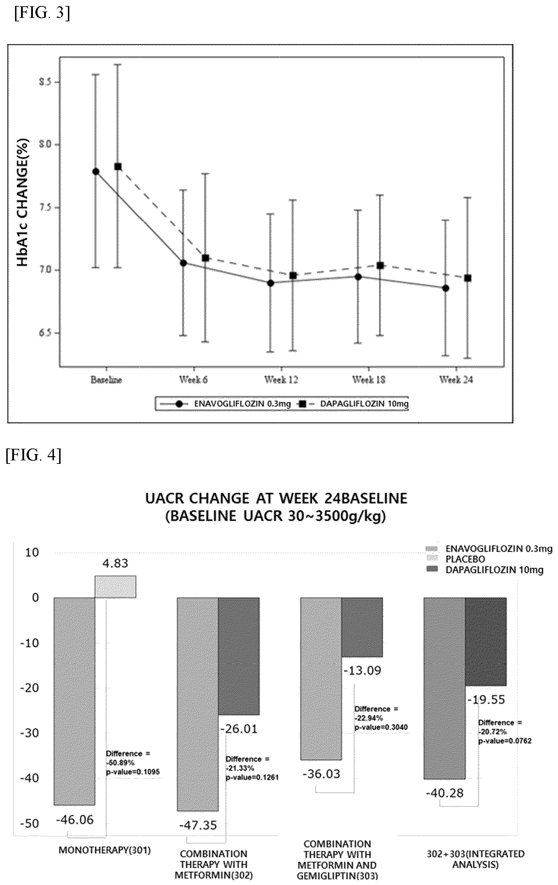

FIG. 3 shows results of measuring HbAlc at 6, 12, 18, and 24 weeks after administration of enavogliflozin or dapagliflozin for 24 weeks in patients with type 2 diabetes whose glycemic control was inadequate with metformin and gemigliptin. An experimental group was administered enavogliflozin at a dose of 0.3 mg or a dapagliflozin placebo 10 mg, and a control was administered an enavogliflozin placebo at a dose of 0.3 mg or dapagliflozin at a dose of 10 mg for 24 hours once a day for 24 weeks. Here, metformin and gemigliptin were taken at the same dose as before. The bar graph represents the mean of each variable, and the error bars represent S.D. The p value of less than 0.05 was considered statistically significant.
FIG. 4 is a graph showing a UACR change rate at week 24 by the administration of a placebo in patients with proteinuria (baseline value UACR of 30 to 3500 g/kg). All of the monotherapy of enavogliflozin, the combination therapy of enavogliflozin and metformin, and the combination therapy of enavogliflozin, metformin, and gemigliptin showed a significant decrease in the UACR change rate at week 24 based on the baseline value as compared to a control.

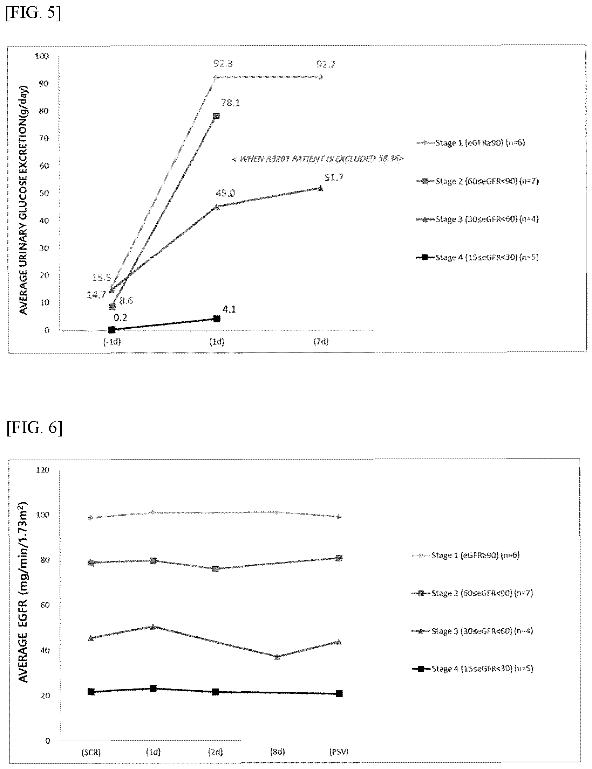

FIG. 5 is a graph showing the change in urinary glucose excretion following the administration of 0.5 mg enavogliflozin in patients satisfying eGFR≥90, 60≤eGFR<90, 30≤eGFR<60, or 15≤eGFR<30.
FIG. 6 is a graph showing the change in eGFR after the administration of 0.5 mg enavogliflozin in patients satisfying eGFR≥90, 60≤eGFR<90, 30≤eGFR<60, or 15≤eGFR<30.

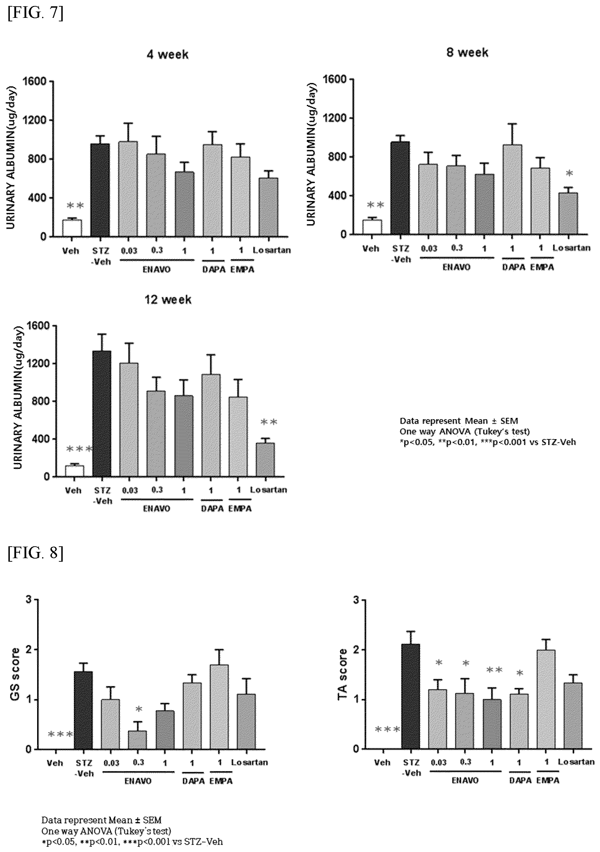

FIG. 7 is a graph showing the results of measuring a urinary albumin concentration at 4, 8, and 12 weeks after the administration of a test material to SD rats treated with streptozotocin.
FIG. 8 is a graph showing the results of measuring a GS score and a TA score at week 12 after the administration of a test material to SD rats treated with streptozotocin.

### [Modes of the Invention]

Hereinafter, one or more exemplary embodiments will be described in further detail with reference to examples. However, these examples are intended to illustrate one or more specific examples and the scope of the present invention is not limited to these examples.

### [Examples]

### Example 1: Single administration of enavogliflozin to patients with type 2 diabetes

### 1. Research process

When a subject voluntarily gave written consent to participate in this clinical trial, a screening test was conducted. Patients with type 2 diabetes who had inadequate glycemic control (7%≤ HbAlc ≤10%) at Visit 1 (screening) were eligible to participate, and patients with type 2 diabetes who were taking hypoglycemic agents at the time of screening were eligible to participate in the trial after a washout period of at least 8 weeks (56 days) prior to screening. However, when the administration of a hypoglycemic agentwas interrupted before the clinical trial Visit 1 (screening), at least an 8-week washout period, including the discontinuation period, was required, and during this period, a stable diet and exercise program was followed. Naïve patients were screened without a separate washout period, and the criteria for naïve patients were defined as patients who had not been administered hypoglycemic agents within 24 weeks prior to the date of obtaining written consent.

Subjects who met the selection/exclusion criteria^{[1]} were administered a placebo of the clinical trial drug in a single-blind (subject blinded) state for a 2-week run-in period. Subjects who met the final inclusion/exclusion criteria and had a medication compliance rate of 70 to 130% for a placebo during the run-in period were centrally randomized to either a test group (enavogliflozin 0.3 mg) or a control (placebo) at a ratio of 1:1 at Visit 2 (randomization). Here, the randomized subjects were stratified according to whether a hypoglycemic agent was administered (drug naïve or previously administered a hypoglycemic agent) within 24 weeks prior to obtaining written informed consent and by an HbAlc value (< 8% or ≥ 8%) at Visit 1 (screening) measured in the central laboratory, and the subjects were administered the clinical trial drug of the assigned group for 24 weeks.
^{[1]} [Inclusion criteria]

The randomized subjects were administered one tablet of the clinical trial drug corresponding to each administration group once a day, and then visited the test institution at 6, 12, 18, and 24 weeks during the 24-week administration period to conduct efficacy and safety assessments. In addition, they were instructed to maintain regular exercise and dietary control starting from 8 weeks prior to screening without changing their daily life patterns during the clinical trial period.

The results are shown in Tables 1 to 20 and FIG. 1.

### Inclusion criteria in Pre-visit (pre-screening) or Visit 1 (screening)

1. Adults aged 19 to 80 as of the date of obtaining written consent
2. Those with 7% ≤ HbA1c* ≤ 10% at Visit 1 (screening) and diagnosed with type 2 diabetes at least 8 weeks prior to Visit 1
   (For subjects who were administered a hypoglycemic agent within 8 weeks prior to Visit 1 (screening), at Pre-visit (pre-screening), the test result at the test institution must have been 6.5% ≤ HbAlc ≤ 10%, and the test result at Visit 1 (screening) after the drug washout period must have been 7% ≤ HbA1c* ≤ 10%.)
   *Test results from the test institution or the central test laboratory are available. The final decision was made based on the results from the central laboratory, but when the test results from the test institution were appropriate before confirming the results from the central laboratory for a run-in process, the run-in was able to proceed.
3. Those who satisfy FPG < 270 mg/dL at Pre-visit (pre-screening) and Visit 1 (screening)
4. Those who have BMI of 20 to 45 kg/m² at Pre-visit (pre-screening) and Visit 1 (screening)
5. Those who are given a stable diet and exercise program for at least 8 weeks prior to Visit 1 (screening)
6. Those who voluntarily decide to participate and give written consent after hearing an explanation of the purpose, method and effect of this clinical trial

### Inclusion criteria at Visit 2 (randomization)

1. Those who have HbAlc of 7% ≤ HbAlc ≤ 10% based on the test results at Visit 1 (screening)
2. Those who have placebo compliance of 70 to 130% during the run-in period confirmed at Visit 2 (randomization)

### [Exclusion criteria]

### Exclusion criteria at Pre-visit (pre-screening) or Visit 1 (screening)

1. Those who have a medical history of or hypersensitivity to drugs and components including the clinical trial drug in this test or any drug in the same class (e.g., a history of hypersensitivity to SGLT2 inhibitor-like drugs, etc.)
2. Those with the following medical history or surgical/therapeutic history

History of medically significant kidney disease: renal vascular occlusive disease, nephrectomy, kidney transplant, etc.

History of major gastrointestinal surgery: total gastrectomy, total colectomy, small bowel resection, gastroenterostomy, gastrointestinal bypass, etc.

History of acute pancreatitis or pancreatic surgery

History of bariatric surgery within the past 2 years

Diabetic ketoacidosis, diabetic coma or precoma within the past year

Urinary tract infection or genital infection within the past year

Alcohol or drug addiction within the past year

Occurrence of acute coronary syndrome, unstable angina, myocardial infarction requiring hospitalization, stroke, transient ischemic attack, or arrhythmia within the past 24 weeks

(However, those who had the disease more than 24 weeks prior and were currently cured or in a stable condition were allowed to enroll.)

Those who have had a history of major surgery resulting in electrolyte imbalance within the past 12 weeks, or who are scheduled for major surgery within 12 weeks after the end of the clinical trial

3. Those with the following diseases or symptoms

Types of diabetes other than type 2 diabetes (type 1 diabetes, secondary diabetes, or congenital renal diabetes)

Symptoms of urinary incontinence, anuria, oliguria, and urinary retention that are not controlled by medication due to stress incontinence, neurogenic bladder, and benign prostatic hyperplasia

Severe diabetic complications (proliferative diabetic retinopathy, kidney disease (nephropathy) of stage 4 or higher, or severe diabetic neuropathy)

Chronic disease requiring continued use (oral, injected, or inhaled) of diuretics, systemic steroids, or immunosuppressants

Severe infection (e.g., severe infection requiring continued use of antibiotics or immunotherapy), clinically significant major trauma

Unstable mental illness whose symptoms are not controlled by medication

Severe gastrointestinal disease: active ulcers, gastrointestinal/rectal bleeding, active inflammatory bowel syndrome, patients with biliary obstruction, active gastritis not controlled by medication, etc.

Uncontrolled high blood pressure (SBP > 180 mmHg or DBP > 110 mmHg)

Patients with moderate to severe nephropathy (eGFR < 60 mL/min/1.73 m²)

Patients with severe liver impairment who fall under one of the following categories:

-AST or ALT > three times the upper limit of the normal level

-Total bilirubin > two times the upper limit of the normal level

-Hepatitis or liver failure

Patients with severe hypertriglyceridemia (triglycerides > 500 mg/dL)

Acquired immunodeficiency syndrome

Severe heart failure (NYHA class III-IV)

Those who require treatment for dehydration due to persistent diarrhea or vomiting, or are at risk of fluid volume depletion

4. Patients who have used other clinical trial drugs/medical devices within the past 4 weeks (however, enrollment was permitted in the case of studies that, in the opinion of the investigator, were unlikely to affect the efficacy and safety of the test, such as observational or retrospective studies.)

5. Patients with a history of malignant tumor within the past 5 years

However, patients with appropriately controlled basal cell carcinoma, squamous cell carcinoma, or cervical intraepithelial carcinoma were allowed to participate

However, participation was possible when there had been no recurrence for more than 5 years after a complete cure (the time of complete removal of tumor through surgery or the completion of chemotherapy, etc.)

6. Pregnant or lactating women or those who do not agree to use appropriate contraception during the clinical trial period

*Appropriate contraception for a subject or his/her partner: should agree to one of the following:
① one of sterilization surgery (vasectomy, etc.), intrauterine device (copper loop, hormone-containing intrauterine system),
② combination of non-oral hormonal contraceptives or spermicides with a barrier method, or
③ combination of a cervical cap or diaphragm together with a male condom

7. Those who are judged by the investigator to be ineligible for this clinical trial

### Exclusion criteria at Visit 1 (screening)

1. Those who have a history of taking the following drugs or are expected to require continuous administration during the clinical trial period

Systemic steroids within the past 2 weeks (more than 30 mg of prednisolone per day)

Therapeutic agents for diabetes within the past 8 weeks

Those who have had a dose change of any drug for thyroid dysfunction within the past 6 weeks (combination use was allowed when dosing had been stable prior to enrollment in the clinical trial; dose reductions were permitted when stable.)

Weight loss drugs taken within the past 12 weeks

### 2. Experimental results

### Description of experimental results

1) Change in HbAlc at week 24 after administration of clinical trial drug compared to Visit 2 (randomization)

The primary efficacy endpoint of this clinical trial is the 'HbAlc change from baseline (Visit 2) at week 24 after administration of the clinical trial drug,' and as a result of performing the analysis of covariance (ANCOVA) using the baseline value and stratification factors (administration of hypoglycemic agent within 24 weeks prior to obtaining written consent, the HbAlc level (< 8% or ≥ 8%) at Visit 1 (screening)) as covariates, the least square means (LS mean (SE)) for the HbAlc change were -0.88(0.10)%p in the test group and 0.11(0.11)%p in the control, and the between-group difference (LS mean difference (95% CI)) was -0.99 (-1.24, -0.74)%p, which showed a statistically significant difference, demonstrating the superiority of the test group compared to the control (p<0.0001).

As a result of confirming the tendency of HbAlc changes over time of evaluation after administration of the clinical trial drug, the HbAlc level decreased in the test group over time, but decreased and then increased in the control.

**[Table 1]**

| **Change from baseline in HbaA1c at week 24 - LOCF (full analysis set)** | | |
|---|---|---|
| | Enavogliflozin 0.3 mg (N=82) | Placebo (N=79) |
| | | |
| Baseline | | |
| n | 82 | 79 |
| Mean (SD) | 7.64(0.66) | 7.72(0.63) |
| Median | 7.50 | 7.60 |
| Min, Max | 6.67, 9.52 | 6.70, 9.68 |
| | | |
| Week 24 | | |
| n | 82 | 79 |
| Mean (SD) | 6.80(0.59) | 7.84(1.15) |
| Median | 6.79 | 7.58 |
| Min, Max | 5.41, 8.59 | 5.76, 11.65 |
| | | |
| Change from baseline at week 24 | | |
| n | 82 | 79 |
| Mean (SD) | -0.84(0.66) | J.12(1.01) |
| Median | -0.78 | 0.06 |
| Min, Max | -2.72, 0.64 | -1.83, 4.08 |
| P-value [1] | <0.0001 (t) | 0.4836 (w) |
| | | |
| ANCOVA result | | |
| LS mean (SE) | -0.88(0.10) | J.11(0.11) |
| LS mean difference | -0.99 | |
| 95% confidence interval for difference | [-1.24, -0.74] | |
| P-value [2] | <0.0001 | |

2) Change in HbAlc at each time point of weeks 6, 12, and 18 after administration of clinical trial drug compared to Visit 2 (randomization)

As a result of performing ANCOVA using the baseline value and stratification factors as covariates, the between-group differences (LS mean difference (95% CI)) in HbAlc change were 0.52 (-0.65, -0.39)%p at week 6, -0.71 (-0.89, -0.54)%p at week 12, and -0.79 (-1.00, -0.58)%p at week 18, showing statistically significant between-group differences at all time points (p<0.0001).

**[Table 2]**

| **Change from baseline in HbA1c at weeks 6, 12 and 18 - LOCF (full analysis set)** | | |
|---|---|---|
| | Enavogliflozin 0.3 mg (N=82) | Placebo (N=79) |
| | | |
| Baseline | | |
| n | 82 | 79 |
| Mean (SD) | 7.64(0.66) | 7.72(0.63) |
| Median | 7.50 | 7.60 |
| Min, Max | 6.67, 9.52 | 6.70, 9.68 |
| | | |
| Week 6 | | |
| n | 81 | 78 |
| Mean (SD) | 7.01(0.59) | 7.59(0.74) |
| Median | 7.00 | 7.43 |
| Min, Max | 5.60, 8.74 | 6.07, 9.73 |
| | | |
| Change from baseline at week 6 | | |
| n | 81 | 78 |
| Mean (SD) | -0.63(0.39) | -0.12(0.51) |
| Median | -0.59 | -0.13 |
| Min, Max | -1.86, 0.17 | -1.61, 1.59 |
| P-value [1] | <0.0001 (t) | 0.0112 (w) |
| | | |
| ANCOVA result | | |
| LS mean (SE) | -0.67(0.05) | -0.16(0.05) |
| LS mean difference | -0.52 | |
| 95% confidence interval for difference | [-0.65, -0.39] | |
| P-value [2] | <0.0001 | |
| | | |
| Week 12 | | |
| n | 82 | 79 |
| Mean (SD) | 6.84(0.53) | 7.61(0.87) |
| Median | 6.81 | 7.48 |
| Min, Max | 5.66, 8.86 | 6.03, 9.75 |
| | | |
| Change from baseline at week 12 | | |
| n | 82 | 79 |
| Mean (SD) | -0.80(0.55) | -0.11 (0.67) |
| Median | -0.74 | -0.21 |
| Min, Max | -2.47, 0.29 | -2.23, 2.51 |
| P-value [1] | <0.0001 (w) | 0.0135 (w) |
| ANCOVA result | | |
| LS mean (SE) | -0.84(0.07) | -0.13(0.07) |
| LS mean difference | -0.71 | |
| 95% confidence interval for difference | [-0.89, -0.54] | |
| P-value [2] | <0.0001 | |
| | | |
| Week 18 | | |
| n | 82 | 79 |
| Mean (SD) | 6.88(0.54) | 7.71(0.97) |
| Median | 6.88 | 7.52 |
| Min, Max | 5.56, 8.78 | 6.02, 10.14 |
| | | |
| Change from baseline at week 18 | | |
| n | 82 | 79 |
| Mean (SD) | -0.77(0.67) | -0.01(0.80) |
| Median | -0.68 | 0.02 |
| Min, Max | -2.92, 0.55 | -2.09, 2.51 |
| P-value [1] | <0.0001 (w) | 0.8781 (t) |
| | | |
| ANCOVA result | | |
| LS mean (SE) | -0.81(0.09) | -0.02(0.09) |
| LS mean difference | -0.79 | |
| 95% confidence interval for difference | [-1.00, -0.58] | |
| P-value [2] | <0.0001 | |

3) Change in FPG at each time point of weeks 6, 12, 18, and 24 after administration of clinical trial drug compared to Visit 2 (randomization)

As a result of performing ANCOVA using the baseline value and stratification factors as covariates, the between-group differences (LS mean difference (95% CI)) in FPG change were -26.91 (-33.19, -20.63) mg/dL at week 6, -32.26 (-39.69, -24.82) mg/dL at week 12, -33.40 (-41.59, -25.22) mg/dL at week 18, and -40.08 (-49.39, -30.77) mg/dL at week 24, showing statistically significant between-group differences at all time points(p<0.0001).

**[Table 3]**

| **Change from baseline in FPG at weeks 6, 12, 18 and 24 - LOCF (full analysis set)** | | |
|---|---|---|
| | Enavogliflozin 0.3 mg (N=82) | Placebo (N=79) |
| | | |
| Baseline | | |
| n | 82 | 79 |
| Mean (SD) | 141.00(24.10) | 141.47(23.51) |
| Median | 137.50 | 140.00 |
| Min, Max | 97.00, 220.00 | 90.00, 214.00 |
| | | |
| Week 6 | | |
| n | 81 | 78 |
| Mean (SD) | 115.19(14.55) | 142.46(30.90) |
| Median | 116.00 | 139.00 |
| Min, Max | 78.00, 157.00 | 95.00, 278.00 |
| | | |
| Change from baseline at week 6 | | |
| n | 81 | 78 |
| Mean (SD) | -25.86(20.19) | 0.77(25.01) |
| Median | -22.00 | -1.00 |
| Min, Max | -97.00, 50.00 | -74.00, 108.00 |
| P-value [1] | <0.0001 (w) | 0.7975 (w) |
| | | |
| ANCOVA result | | |
| LS mean (SE) | -27.78(2.47) | -0.88(2.51) |
| LS mean difference | -26.91 | |
| 95% confidence interval for difference | [-33.19, -20.63] | |
| P-value [2] | <0.0001 | |
| | | |
| Week 12 | | |
| n | 82 | 79 |
| Mean (SD) | 111.66(13.55) | 144.18(36.21) |
| Median | 112.00 | 136.00 |
| Min, Max | 67.00, 151.00 | 89.00, 304.00 |
| | | |
| Change from baseline at week 12 | | |
| n | 82 | 79 |
| Mean (SD) | -29.34(20.90) | 2.71(30.34) |
| Median | -27.00 | -2.00 |
| Min, Max | -96.00, 10.00 | -59.00, 136.00 |
| P-value [1] | <0.0001 (w) | 0.8327 (w) |
| | | |
| ANCOVA result | | |
| LS mean (SE) | -30.66(2.93) | 1.60(2.97) |
| LS mean difference | -32.26 | |
| 95% confidence interval for difference | [-39.69, -24.82] | |
| P-value [2] | <0.0001 | |
| | | |
| Week 18 | | |
| n | 82 | 79 |
| Mean (SD) | 110.48(14.49) | 144.04(37.52) |
| Median | 112.50 | 137.00 |
| Min, Max | 64.00, 159.00 | 87.00, 304.00 |
| | | |
| Change from baseline at week 18 | | |
| n | 82 | 79 |
| Mean (SD) | -30.52(22.34) | 2.57(34.84) |
| Median | -27.00 | -3.00 |
| Min, Max | -106.00, 22.00 | -69.00, 151.00 |
| P-value [1] | <0.0001 (w) | 0.3378 (w) |
| | | |
| ANCOVA result | | |
| LS mean (SE) | -31.97(3.23) | 1.44(3.27) |
| LS mean difference | -33.40 | |
| 95% confidence interval for difference | [-41.59, -25.22] | |
| P-value [2] | <0.0001 | |
| | | |
| Week 24 | | |
| n | 82 | 79 |
| Mean (SD) | 111.00(14.56) | 151.34(44.39) |
| Median | 110.50 | 142.00 |
| Min, Max | 70.00, 150.00 | 87.00, 322.00 |
| | | |
| Change from baseline at week 24 | | |
| n | 82 | 79 |
| Mean (SD) | -30.00(20.73) | 9.87(39.42) |
| Median | -27.00 | 1.00 |
| Min, Max | -100.00, 36.00 | -47.00, 201.00 |
| P-value [1] | <0.0001 (w) | 0.1571 (w) |
| | | |
| ANCOVA result | | |
| LS mean (SE) | -31.66(3.68) | 8.42(3.72) |
| LS mean difference | -40.08 | |
| 95% confidence interval for difference | [-49.39, -30.77] | |
| P-value [2] | <0.0001 | |

4) Proportion of subjects achieving HbAlc < 7% at each time point of weeks 6, 12, 18, and 24 after administration of clinical trial drug

As a result of analyzing logistic regression corrected with a stratification factor, compared to the control, the odds ratio (95% CI) for achieving HbAlc < 7% in the test group was 6.55 (2.92, 14.70) at week 6, 6.53 (3.11, 13.70) at week 12, 4.85 (2.34, 10.07) at week 18, and 9.08 (4.27, 19.31) at week 24, and there were statistically significant between-group differences at all time points (p<0.0001).

**[Table 4]**

| **Proportion of subjects achieving HbAlc target of < 7% at weeks 6, 12, 18 and 24 - LOCF (full analysis set)** | | |
|---|---|---|
| | Enavogliflozin 0.3 mg (N=82) | Placebo (N=79) |
| | | |
| Proportion of subjects achieving HbA1c target of < 7% at week 6, N | 81 | 78 |
| Subjects achieving HbA1c target of < 7%, n(%) | 40(49.38) | 12(15.38) |
| | | |
| Logistic regression model | | |
| Odds ratio | 6.55 | |
| 95% confidence interval | [2.92, 14.70] | |
| P-value [1] | <0.0001 | |
| | | |
| Proportion of subjects achieving HbA1c target of < 7% at week 12, N | 82 | 79 |
| Subjects achieving HbA1c target of < 7%, n(%) | 54(65.85) | 21(26.58) |
| | | |
| Logistic regression model | | |
| Odds ratio | 6.53 | |
| 95% confidence interval | [3.11, 13.70] | |
| P-value [1] | <0.0001 | |
| | | |
| Proportion of subjects achieving HbA1c target of < 7% at week 18, N | 82 | 79 |
| Subjects achieving HbA1c target of < 7%, n(%) | 47(57.32) | 20(25.32) |
| | | |
| Logistic regression model | | |
| Odds ratio | 4.85 | |
| 95% confidence interval | [2.34, 10.07] | |
| P-value [1] | <0.0001 | |
| | | |
| Proportion of subjects achieving HbA1c target of < 7% at week 24, N | 82 | 79 |
| Subjects achieving HbA1c target of < 7%, n(%) | 58(70.73) | 19(24.05) |
| | | |
| Logistic regression model | | |
| Odds ratio | 9.08 | |
| 95% confidence interval | [4.27, 19.31] | |
| P-value [1] | <0.0001 | |

5) Proportion of subjects achieving HbAlc < 6.5% at each time point of weeks 6, 12, 18, and 24 after administration of clinical trial drug

As a result of logistic regression analysis corrected with the stratification factors, compared to the control, the odds ratios (95% CI) for achieving HbAlc < 6.5% in the test group were 3.29 (1.00, 10.83) at week 6, 7.58 (2.43, 23.65) at week 12, 5.58 (2.09, 14.90) at week 18, and 12.10 (3.90, 37.53) at week 24, showing that there were statistically significant between-group differences at all time points (week 6 p=0.0498, week 12 p=0.0005, week 18 p=0.0006, week 24 p<0.0001).

**[Table 5]**

| **Proportion of subjects achieving HbAlc target of < 6.5% at weeks 6, 12, 18 and 24 - LOCF (full analysis set)** | | |
|---|---|---|
| | Enavogliflozin 0.3 mg (N=82) | Placebo (N=79) |
| Proportion of subjects achieving HbA1c target of < 6.5% at week 6, N | 81 | 78 |
| Subjects achieving HbA1c target of < 6.5%, n(%) | 12(14.81) | 4(5.13) |
| Logistic regression model | | |
| Odds ratio | 3.29 | |
| 95% confidence interval | [1.00, 10.83] | |
| P-value [1] | 0.0498 | |
| Proportion of subjects achieving HbA1c target of < 6.5% at week 12, N | 82 | 79 |
| Subjects achieving HbA1c target of < 6.5%, n(%) | 22(26.83) | 4(5.06) |
| Logistic regression model | | |
| Odds ratio | 7.58 | |
| 95% confidence interval | [2.43, 23.65] | |
| P-value [1] | 0.0005 | |
| Proportion of subjects achieving HbA1c target of < 6.5% at week 18, N | 82 | 79 |
| Subjects achieving HbA1c target of < 6.5%, n(%) | 24(29.27) | 6(7.59) |
| Logistic regression model | | |
| Odds ratio | 5.58 | |
| 95% confidence interval | [2.09, 14.90] | |
| P-value [1] | 0.0006 | |
| Proportion of subjects achieving HbA1c target of < 6.5% at week 24, N | 82 | 79 |
| Subjects achieving HbA1c target of < 6.5%, n(%) | 29(35.37) | 4(5.06) |
| Logistic regression model | | |
| Odds ratio | 12.10 | |
| 95% confidence interval | [3.90, 37.53] | |
| P-value [1] | <0.0001 | |

6) **Proportion of subjects achieving therapeutic response (HbA1c change (HbAlc at Visit 2** - **HbAlc at each evaluation time point) > 0.5% or HbAlc < 7% at each evaluation time point compared to Visit 2 (randomization)) at each time point of weeks 6, 12, 18 and 24 after administration of clinical trial drug**

As a result of logistic regression analysis corrected with the stratification factors, compared to the control, the odds ratios (95% CI) for achieving therapeutic response in the test groups were 12.84(5.76, 28.63) at week 6, 14.77(6.57, 33.17) at week 12, 7.45(3.48, 15.95) at week 18, and 12.98(5.78, 29.15) at week 24, showing statistically significant between-group differences at all time points (p<0.0001).

**[Table 6]**

| **Proportion of Subjects achieving a therapeutic glycemic response at weeks 6, 12, 18 and 24 - LOCF (full analysis set)** | | |
|---|---|---|
| | Enavogliflozin 0.3 mg (N=82) | Placebo (N=79) |
| Proportion of subjects achieving therapeutic glycemic response at week 6, N | 81 | 78 |
| Subjects achieving therapeutic glycemic response, n(%) | 51(75.31) | 18(23.08) |
| Logistic regression model | | |
| Odds ratio | 12.84 | |
| 95% confidence interval | [5.76, 28.63] | |
| P-value [1] | <0.0001 | |
| Proportion of subjects achieving therapeutic glycemic response at week 12, N | 82 | 79 |
| Subjects achieving therapeutic glycemic response, n(%) | 70(85.37) | 24(30.38) |
| Logistic regression model | | |
| Odds ratio | 14.77 | |
| 95% confidence interval | [6.57, 33.17] | |
| P-value [1] | <0.0001 | |
| Proportion of subjects achieving therapeutic glycemic response at week 18, N | 82 | 79 |
| Subjects achieving therapeutic glycemic response, n(%) | 50(73.17) | 26(32.91) |
| Logistic regression model | | |
| Odds ratio | 7.45 | |
| 95% confidence interval | [3.48, 15.95] | |
| P-value [1] | <0.0001 | |
| Proportion of subjects achieving therapeutic glycemic response at week 24, N | 82 | 79 |
| Subjects achieving therapeutic glycemic response, n(%) | 58(82.93) | 25(31.65) |
| Logistic regression model | | |
| Odds ratio | 12.98 | |
| 95% confidence interval | [5.78, 29.15] | |
| P-value [1] | <0.0001 | |

7) Change in fasting C-peptide (FAS) at each time point of weeks 6, 12, 18, and 24 after administration of clinical trial drug compared to Visit 2 (randomization)

As a result of performing ANCOVA using the baseline value and stratification factors as covariates, the between-group differences (LS mean difference (95% CI)) in fasting C-peptide change between the control and the test group were -0.11(-0.17, -0.06) ng/dL at week 6, -0.12(-0.18, -0.06) ng/dL at week 12, -0.11(-0.16, -0.05) ng/dL at week 18, and -0.13(-0.18, -0.07) ng/dL at week 24, showing statistically significant differences at all time points.

**[Table 7]**

| **Change from baseline in fasting C-peptide at weeks 6, 12, 18 and 24 - LOCF (full analysis set)** | | |
|---|---|---|
| | Enavogliflozin 0.3 mg (N=82) | Placebo (N=79) |
| Baseline | | |
| n | 82 | 79 |
| Mean (SD) | J.75(0.30) | 0.76(0.30) |
| Median | 0.71 | 0.70 |
| Min, Max | 0.30, 1.75 | 0.33, 1.62 |
| Week 6 | | |
| n | 81 | 78 |
| Mean (SD) | J.66(0.25) | 0.78(0.33) |
| Median | 0.63 | 0.73 |
| Min, Max | 0.20, 1.39 | 0.30, 2.02 |
| Change from baseline at week 6 | | |
| n | 81 | 78 |
| Mean (SD) | -0.09(0.19) | 0.02(0.20) |
| Median | -0.10 | 0.03 |
| Min, Max | -0.83, 0.63 | -0.63, 0.76 |
| P-value [1] | <0.0001 (w) | 0.4517 (w) |
| ANCOVA result | | |
| LS mean (SE) | -0.10(0.02) | 0.01(0.02) |
| LS mean difference | -0.11 | |
| 95% confidence interval for difference | [-0.17, -0.06] | |
| P-value [2] | 0.0002 | |
| Week 12 | | |
| n | 82 | 79 |
| Mean (SD) | J.64(0.26) | 0.77(0.32) |
| Median | 0.60 | 0.70 |
| Min, Max | 0.30, 1.59 | 0.30, 1.82 |
| Change from baseline at week 12 | | |
| n | 82 | 79 |
| Mean (SD) | -0.11(0.17) | 0.01(0.22) |
| Median | -0.10 | 0.00 |
| Min, Max | -0.79, 0.23 | -0.73, 0.89 |
| P-value [1] | <0.0001 (w) | 0.9711 (w) |
| ANCOVA result | | |
| LS mean (SE) | -0.10(0.02) | 0.02(0.02) |
| LS mean difference | -0.12 | |
| 95% confidence interval for difference | [-0.18, -0.06] | |
| P-value [2] | <0.0001 | |
| Week 18 | | |
| n | 82 | 79 |
| Mean (SD) | 0.65(0.29) | 0.77(0.30) |
| Median | 0.56 | 0.70 |
| Min, Max | 0.26, 1.62 | 0.30, 1.56 |
| Change from baseline at week 18 | | |
| n | 82 | 79 |
| Mean (SD) | -0.10(0.16) | 0.01(0.21) |
| Median | -0.10 | 0.00 |
| Min, Max | -0.50, 0.36 | -0.46, 0.96 |
| P-value [1] | <0.0001 (t) | 0.9175 (w) |
| ANCOVA result | | |
| LS mean (SE) | -0.10(0.02) | 0.01(0.02) |
| LS mean difference | -0.11 | |
| 95% confidence interval for difference | [-0.16, -0.05] | |
| P-value [2] | 0.0001 | |
| Week 24 | | |
| n | 82 | 79 |
| Mean (SD) | J.64(0.27) | 0.78(0.30) |
| Median | 0.60 | 0.70 |
| Min, Max | 0.20, 1.56 | 0.30, 1.59 |
| Change from baseline at week 24 | | |
| n | 82 | 79 |
| Mean (SD) | -0.11(0.19) | 0.01(0.19) |
| Median | -0.10 | 0.00 |
| Min, Max | -0.86, 0.33 | -0.46, 0.73 |
| P-value [1] | <0.0001 (w) | 0.6740 (w) |
| ANCOVA result | | |
| LS mean (SE) | -0.10(0.02) | 0.02(0.02) |
| LS mean difference | -0.13 | |
| 95% confidence interval for difference | [-0.18, -0.07] | |
| P-value [2] | <0.0001 | |

8) Change in body weight (FAS) at each time point of weeks 6, 12, 18, and 24 after administration of the clinical trial drug compared to Visit 2 (randomization)

As a result of performing ANCOVA using the baseline value and stratification factors as covariates, the between-group differences (LS mean difference (95% CI)) in body weight change were -1.45(-1.93, -0.97) kg at week 6, -1.66(-2.35, -0.96) kg at week 12, -2.37(-3.14, -1.60) kg at week 18, and -2.47(-3.30, -1.63) kg at week 24, showing statistically significant between-group differences at all time points.

**[Table 8]**

| **Change from baseline in body weight at weeks 6, 12, 18 and 24 - LOCF (full analysis set)** | | |
|---|---|---|
| | Enavogliflozin 0.3 mg (N=82) | Placebo (N=79) |
| Baseline | | |
| n | 82 | 79 |
| Mean (SD) | 68.77(12.55) | 69.14(13.66) |
| Median | 68.50 | 68.20 |
| Min, Max | 42.00, 104.00 | 48.00, 116.20 |
| Week 6 | | |
| n | 81 | 78 |
| Mean (SD) | 56.81(11.92) | 68.48(13.73) |
| Median | 67.00 | 66.80 |
| Min, Max | 41.50, 95.00 | 46.30, 116.40 |
| Change from baseline at week 6 | | |
| n | 81 | 78 |
| Mean (SD) | -2.05(1.80) | -0.61(1.31) |
| Median | -1.90 | -0.45 |
| Min, Max | -9.00, 1.40 | -4.80, 6.10 |
| P-value [1] | <0.0001 (w) | <0.0001 (w) |
| ANCOVA result | | |
| LS mean (SE) | -2.03(0.19) | -0.58(0.19) |
| LS mean difference | -1.45 | |
| 95% confidence interval for difference | [-1.93, -0.97] | |
| P-value [2] | <0.0001 | |
| Week 12 | | |
| n | 82 | 79 |
| Mean (SD) | 66.12(12.10) | 68.14(13.53) |
| Median | 65.60 | 67.00 |
| Min, Max | 42.80, 100.00 | 47.10, 115.30 |
| Change from baseline at week 12 | | |
| n | 82 | 79 |
| Mean (SD) | -2.65(2.73) | -1.00(1.65) |
| Median | -2.60 | -1.10 |
| Min, Max | -12.80, 13.00 | -7.10, 4.70 |
| P-value [1] | <0.0001 (w) | <0.0001 (w) |
| ANCOVA result | | |
| LS mean (SE) | -2.59(0.27) | -0.93(0.27) |
| LS mean difference | -1.66 | |
| 95% confidence interval for difference | [-2.35, -0.96] | |
| P-value [2] | <0.0001 | |
| Week 18 | | |
| n | 82 | 79 |
| Mean (SD) | 65.32(11.77) | 68.04(13.90) |
| Median | 64.85 | 66.30 |
| Min, Max | 42.30, 99.40 | 47.00, 115.60 |
| Change from baseline at week 18 | | |
| n | 82 | 79 |
| Mean (SD) | -3.44(2.76) | -1.11 (2.28) |
| Median | -3.05 | -1.20 |
| Min, Max | -18.00, 1.00 | -6.10, 11.10 |
| P-value [1] | <0.0001 (w) | <0.0001 (w) |
| ANCOVA result | | |
| LS mean (SE) | -3.42(0.30) | -1.05(0.30) |
| LS mean difference | -2.37 | |
| 95% confidence interval for difference | [-3.14, -1.60] | |
| P-value [2] | <0.0001 | |
| Week 24 | | |
| n | 82 | 79 |
| Mean (SD) | 65.28(11.93) | 68.10(14.11) |
| Median | 65.25 | 66.00 |
| Min, Max | 42.70, 98.20 | 48.10, 118.10 |
| Change from baseline at week 24 | | |
| n | 82 | 79 |
| Mean (SD) | -3.48(2.96) | -1.04(2.42) |
| Median | -3.40 | -1.20 |
| Min, Max | -20.80, 1.00 | -5.70, 10.00 |
| P-value [1] | <0.0001 (w) | <0.0001 (w) |
| ANCOVA result | | |
| LS mean (SE) | -3.47(0.33) | -1.01(0.33) |
| LS mean difference | -2.47 | |
| 95% confidence interval for difference | [-3.30, -1.63] | |
| P-value [2] | <0.0001 | |

9) Change in fasting lipid concentration (triglycerides) at each time point of weeks 6, 12, 18, and 24 after administration of clinical trial drug compared to Visit 2 (randomization)

As a result of performing ANCOVA using the baseline value and stratification factors as covariates, the between-group differences (LS mean difference (95% CI)) in total cholesterol change were 1.95(-4.54, 8.43) mg/dL at week 6, -4.04(-11.86, 3.77) mg/dL at week 12, -0.99(-8.33, 6.34) mg/dL at week 18, and -6.40(-14.63, 1.83) mg/dL at week 24, showing no statistically significant differences at any time point.

**[Table 9]**

| **Change from baseline in fasting lipid parameters at weeks 6, 12, 18 and 24 - LOCF (modified full analysis set 1) [total cholesterol]** | | |
|---|---|---|
| | Enavogliflozin 0.3 mg (N=77) | Placebo (N=75) |
| | | |
| Baseline | | |
| n | 77 | 75 |
| Mean (SD) | 161.42(36.14) | 165.53(35.08) |
| Median | 158.00 | 170.00 |
| Min, Max | 101.00, 280.00 | 82.00, 249.00 |
| | | |
| Week 6 | | |
| n | 76 | 74 |
| Mean (SD) | 166.11(39.22) | 168.09(36.93) |
| Median | 159.00 | 168.00 |
| Min, Max | 104.00, 313.00 | 77.00, 263.00 |
| | | |
| Change from baseline at week 6 | | |
| n | 76 | 74 |
| Mean (SD) | 5.75(20.81) | 3.54(19.06) |
| Median | 3.50 | 1.00 |
| Min, Max | -39.00, 72.00 | -54.00, 68.00 |
| P-value [1] | 0.0185 (t) | 0.1145 (t) |
| | | |
| ANCOVA result | | |
| LS mean (SE) | 5.76(2.52) | 3.81(2.57) |
| LS mean difference | 1.95 | |
| 95% confidence interval for difference | [-4.54, 8.43] | |
| P-value [2] | 0.5537 | |
| | | |
| Week 12 | | |
| n | 77 | 75 |
| Mean (SD) | 167.40(37.13) | 174.99(40.86) |
| Median | 157.00 | 173.00 |
| Min, Max | 99.00, 310.00 | 81.00, 277.00 |
| | | |
| Change from baseline at week 12 | | |
| n | 77 | 75 |
| Mean (SD) | 5.99(23.92) | 9.45(25.57) |
| Median | 9.00 | 5.00 |
| Min, Max | -45.00, 90.00 | -50.00, 100.00 |
| P-value [1] | 0.0311 (t) | 0.0036 (w) |
| | | |
| ANCOVA result | | |
| LS mean (SE) | 4.75(3.04) | 8.79(3.09) |
| LS mean difference | -4.04 | |
| 95% confidence interval for difference | [-11.86, 3.77] | |
| P-value [2] | 0.3083 | |
| | | |
| Week 18 | | |
| n | 77 | 75 |
| Mean (SD) | 169.82(38.50) | 174.45(39.03) |
| Median | 161.00 | 173.00 |
| Min, Max | 107.00, 298.00 | 104.00, 303.00 |
| | | |
| Change from baseline at week 18 | | |
| n | 77 | 75 |
| Mean (SD) | 8.40(23.74) | 8.92(22.79) |
| Median | 5.00 | 6.00 |
| Min, Max | -53.00, 71.00 | -42.00, 84.00 |
| P-value [1] | 0.0027 (t) | 0.0030 (w) |
| | | |
| ANCOVA result | | |
| LS mean (SE) | 5.45(2.86) | 7.44(2.90) |
| LS mean difference | -0.99 | |
| 95% confidence interval for difference | [-8.33, 6.34] | |
| P-value [2] | 0.7890 | |
| | | |
| Week 24 | | |
| n | 77 | 75 |
| Mean (SD) | 168.00(36.92) | 177.92(42.51) |
| Median | 159.00 | 178.00 |
| Min, Max | 92.00, 289.00 | 94.00, 273.00 |
| | | |
| Change from baseline at week 24 | | |
| n | 77 | 75 |
| Mean (SD) | 5.58(21.82) | 12.39(30.02) |
| Median | 6.00 | 7.00 |
| Min, Max | -61.00, 71.00 | -49.00, 126.00 |
| P-value [1] | 0.0098 (t) | 0.0017 (w) |
| | | |
| ANCOVA result | | |
| LS mean (SE) | 4.84(3.21) | 11.24(3.25) |
| LS mean difference | -6.40 | |
| 95% confidence interval for difference | [-14.63, 1.83] | |
| P-value [2] | 0.1267 | |

10) Change in fasting lipid concentration (LDL-C) at each time point of weeks 6, 12, 18, and 24 after administration of clinical trial drug compared to Visit 2 (randomization)

As a result of performing ANCOVA using the baseline value and stratification factors as covariates, the between-group differences (LS mean difference (95% CI)) in LDL-C change were 0.64(-5.12, 6.41) mg/dL at week 6, -6.00(-12.58, 0.57) mg/dL at week 12, -2.35(-8.59, 3.89) mg/dL at week 18, and -6.91(-13.54, -0.28) mg/dL at week 24, showing a statistically significant difference at week 24.

**[Table 10]**

| **Change from baseline in fasting lipid parameters at weeks 6, 12, 18 and 24 - LOCF (modified full analysis set 1) [LDL-C]** | | |
|---|---|---|
| | Enavogliflozin 0.3 mg (N=77) | Placebo (N=75) |
| | | |
| Baseline | | |
| n | 77 | 75 |
| Mean (SD) | 95.47(33.86) | 99.01(33.01) |
| Median | 88.00 | 98.00 |
| Min, Max | 42.00, 204.00 | 42.00, 181.00 |
| | | |
| Week 6 | | |
| n | 76 | 74 |
| Mean (SD) | 96.18(36.96) | 98.96(34.55) |
| Median | 91.00 | 94.00 |
| Min, Max | 45.00, 231.00 | 25.00, 187.00 |
| | | |
| Change from baseline at week 6 | | |
| n | 76 | 74 |
| Mean (SD) | 1.55(18.30) | 0.74(17.15) |
| Median | 0.00 | -0.50 |
| Min, Max | -48.00, 46.00 | -51.00, 39.00 |
| P-value [1] | 0.4618 (t) | 0.7104 (t) |
| | | |
| ANCOVA result | | |
| LS mean (SE) | 1.23(2.24) | 0.59(2.29) |
| LS mean difference | 0.64 | |
| 95% confidence interval for difference | [-5.12, 6.41] | |
| P-value [2] | 0.8264 | |
| | | |
| Week 12 | | |
| n | 77 | 75 |
| Mean (SD) | 95.38(34.18) | 104.47(36.39) |
| Median | 89.00 | 101.00 |
| Min, Max | 42.00, 224.00 | 23.00, 205.00 |
| | | |
| Change from baseline at week 12 | | |
| n | 77 | 75 |
| Mean (SD) | -0.09(18.99) | 5.45(23.10) |
| Median | 0.00 | 3.00 |
| Min, Max | -54.00, 41.00 | -53.00, 97.00 |
| P-value [1] | 0.9666 (t) | 0.1281 (w) |
| | | |
| ANCOVA result | | |
| LS mean (SE) | -2.31(2.56) | 3.70(2.60) |
| LS mean difference | -6.00 | |
| 95% confidence interval for difference | [-12.58, 0.57] | |
| P-value [2] | 0.0733 | |
| | | |
| Week 18 | | |
| n | 77 | 75 |
| Mean (SD) | 97.16(34.89) | 102.68(35.85) |
| Median | 92.00 | 101.00 |
| Min, Max | 46.00, 213.00 | 33.00, 227.00 |
| | | |
| Change from baseline at week 18 | | |
| n | 77 | 75 |
| Mean (SD) | 1.69(18.80) | 3.67(20.91) |
| Median | -3.00 | -1.00 |
| Min, Max | -47.00, 64.00 | -43.00, 92.00 |
| P-value [1] | 0.8135 (w) | 0.3766 (w) |
| | | |
| ANCOVA result | | |
| LS mean (SE) | -0.32(2.43) | 2.03(2.47) |
| LS mean difference | -2.35 | |
| 95% confidence interval for difference | [-8.59, 3.89] | |
| P-value [2] | 0.4581 | |
| | | |
| Week 24 | | |
| n | 77 | 75 |
| Mean (SD) | 94.44(35.06) | 104.49(36.74) |
| Median | 87.00 | 101.00 |
| Min, Max | 39.00, 218.00 | 33.00, 185.00 |
| | | |
| Change from baseline at week 24 | | |
| n | 77 | 75 |
| Mean (SD) | -1.03(18.10) | 5.48(23.85) |
| Median | -1.00 | 3.00 |
| Min, Max | -55.00, 64.00 | -43.00, 102.00 |
| P-value [1] | 0.6204 (t) | 0.1453 (w) |
| | | |
| ANCOVA result | | |
| LS mean (SE) | -2.92(2.58) | 3.99(2.62) |
| LS mean difference | -6.91 | |
| 95% confidence interval for difference | [-13.54, -0.28] | |
| P-value [2] | 0.0412 | |

11) Change in fasting lipid concentration (HDL-C) at each time point of weeks 6, 12, 18, and 24 after administration of clinical trial drug compared to Visit 2 (randomization)

As a result of performing ANCOVA using the baseline value and stratification factors as covariates, the between-group differences (LS mean difference (95% CI)) in HDL-C change were 1.89(0.15, 3.64) mg/dL at week 6, 2.36(0.11, 4.62) mg/dL at week 12, 2.69(0.64, 4.74) mg/dL at week 18, and 4.05(1.71, 6.38) mg/dL at week 24, showing statistically significant between-group differences at all time points.

**[Table 11]**

| **Change from baseline in fasting lipid parameters at weeks 6, 12, 18 and 24 - LOCF (modified full analysis set 1) [HDL-C]** | | |
|---|---|---|
| | Enavogliflozin 0.3 mg (N=77) | Placebo (N=75) |
| Baseline | | |
| n | 77 | 75 |
| Mean (SD) | 51.92(10.64) | 49.20(11.90) |
| Median | 50.00 | 47.00 |
| Min, Max | 32.00, 84.00 | 29.00, 91.00 |
| | | |
| Week 6 | | |
| n | 76 | 74 |
| Mean (SD) | 53.79(11.10) | 49.68(11.52) |
| Median | 53.00 | 47.50 |
| Min, Max | 31.00, 85.00 | 28.00, 79.00 |
| | | |
| Change from baseline at week 6 | | |
| n | 76 | 74 |
| Mean (SD) | 2.07(5.44) | 0.43(5.56) |
| Median | 2.00 | 0.00 |
| Min, Max | -19.00, 16.00 | -17.00, 17.00 |
| P-value [1] | 0.0001 (w) | 0.5445 (w) |
| | | |
| ANCOVA result | | |
| LS mean (SE) | 2.40(0.67) | 0.50(0.69) |
| LS mean difference | 1.89 | |
| 95% confidence interval for difference | [0.15, 3.64] | |
| P-value [2] | 0.0335 | |
| | | |
| Week 12 | | |
| n | 77 | 75 |
| Mean (SD) | 54.14(12.02) | 49.41(12.03) |
| Median | 54.00 | 47.00 |
| Min, Max | 29.00, 100.00 | 30.00, 82.00 |
| | | |
| Change from baseline at week 12 | | |
| n | 77 | 75 |
| Mean (SD) | 2.22(7.64) | 0.21(6.44) |
| Median | 3.00 | 0.00 |
| Min, Max | -19.00, 30.00 | -16.00, 24.00 |
| P-value [1] | 0.0032 (w) | 0.8457 (w) |
| | | |
| ANCOVA result | | |
| LS mean (SE) | 2.35(0.87) | -0.01(0.89) |
| LS mean difference | 2.36 | |
| 95% confidence interval for difference | [0.11, 4.62] | |
| P-value [2] | 0.0404 | |
| | | |
| Week 18 | | |
| n | 77 | 75 |
| Mean (SD) | 55.55(10.83) | 50.68(11.07) |
| Median | 55.00 | 49.00 |
| Min, Max | 32.00, 82.00 | 30.00, 83.00 |
| | | |
| Change from baseline at week 18 | | |
| n | 77 | 75 |
| Mean (SD) | 3.62(7.86) | 1.48(5.43) |
| Median | 4.00 | 1.00 |
| Min, Max | -19.00, 22.00 | -10.00, 16.00 |
| P-value [1] | 0.0001 (t) | 0.0209 (t) |
| | | |
| ANCOVA result | | |
| LS mean (SE) | 4.18(0.79) | 1.50(0.81) |
| LS mean difference | 2.69 | |
| 95% confidence interval for difference | [0.64, 4.74] | |
| P-value [2] | 0.0106 | |
| | | |
| Week 24 | | |
| n | 77 | 75 |
| Mean (SD) | 56.05(11.72) | 49.76(11.76) |
| Median | 55.00 | 47.00 |
| Min, Max | 38.00, 86.00 | 29.00, 83.00 |
| | | |
| Change from baseline at week 24 | | |
| n | 77 | 75 |
| Mean (SD) | 4.13(8.36) | 0.56(6.35) |
| Median | 4.00 | 1.00 |
| Min, Max | -21.00, 36.00 | -19.00, 17.00 |
| P-value [1] | <0.0001 (w) | 0.4476 (t) |
| | | |
| ANCOVA result | | |
| LS mean (SE) | 4.39(0.91) | 0.35(0.93) |
| LS mean difference | 4.05 | |
| 95% confidence interval for difference | [1.71, 6.38] | |
| P-value [2] | 0.0008 | |

12) Change in fasting lipid concentration (triglycerides) at each time point of weeks 6, 12, 18, and 24 after administration of clinical trial drug compared to Visit 2 (randomization)

As a result of performing ANCOVA using the baseline value and stratification factors as covariates, the between-group differences in triglyceride change (LS mean difference (95% CI)) were -17.69(-34.78, -0.60) mg/dL at week 6, -10.37(-28.69, 7.95) mg/dL at week 12, -17.74(-39.45, 3.98) mg/dL at week 18, and -28.56(-54.04, -3.07) mg/dL at week 24, showing statistically significant differences at weeks 6 and 24.

**[Table 12]**

| **Change from baseline in fasting lipid parameters at weeks 6, 12, 18 and 24 - LOCF (modified full analysis set 1) [triglycerides]** | | |
|---|---|---|
| | Enavogliflozin 0.3 mg (N=77) | Placebo (N=75) |
| | | |
| Baseline | | |
| n | 77 | 75 |
| Mean (SD) | 117.44(49.60) | 137.88(75.62) |
| Median | 109.00 | 116.00 |
| Min, Max | 45.00, 262.00 | 45.00, 390.00 |
| | | |
| Week 6 | | |
| n | 76 | 74 |
| Mean (SD) | 116.13(49.64) | 145.61(79.75) |
| Median | 104.00 | 120.50 |
| Min, Max | 53.00, 261.00 | 41.00, 404.00 |
| | | |
| Change from baseline at week 6 | | |
| n | 76 | 74 |
| Mean (SD) | -1.21(39.88) | 10.97(68.10) |
| Median | -1.00 | 12.00 |
| Min, Max | -133.00, 119.00 | -276.00, 229.00 |
| P-value [1] | 0.8044 (w) | 0.1447 (w) |
| | | |
| ANCOVA result | | |
| LS mean (SE) | -5.05(6.60) | 12.64(6.74) |
| LS mean difference | -17.69 | |
| 95% confidence interval for difference | [-34.78, -0.60] | |
| P-value [2] | 0.0426 | |
| | | |
| Week 12 | | |
| n | 77 | 75 |
| Mean (SD) | 120.34(74.85) | 149.63(91.82) |
| Median | 96.00 | 125.00 |
| Min, Max | 40.00, 553.00 | 38.00, 642.00 |
| | | |
| Change from baseline at week 12 | | |
| n | 77 | 75 |
| Mean (SD) | 2.90(48.90) | 11.75(65.07) |
| Median | -5.00 | 3.00 |
| Min, Max | -131.00, 291.00 | -203.00, 264.00 |
| P-value [1] | 0.8603 (w) | 0.2939 (w) |
| | | |
| ANCOVA result | | |
| LS mean (SE) | 5.37(7.08) | 16.74(7.20) |
| LS mean difference | -10.37 | |
| 95% confidence interval for difference | [-28.69, 7.95] | |
| P-value [2] | 0.2649 | |
| | | |
| Week 18 | | |
| n | 77 | 75 |
| Mean (SD) | 113.30(52.73) | 146.80(106.25) |
| Median | 108.00 | 121.00 |
| Min, Max | 32.00, 321.00 | 44.00, 675.00 |
| | | |
| Change from baseline at week 18 | | |
| n | 77 | 75 |
| Mean (SD) | -4.14(39.69) | 8.92(88.46) |
| Median | -9.00 | -6.00 |
| Min, Max | -138.00, 74.00 | -254.00, 571.00 |
| P-value [1] | 0.4143 (w) | 1.0000 (w) |
| | | |
| ANCOVA result | | |
| LS mean (SE) | -8.82(8.40) | 8.92(8.54) |
| LS mean difference | -17.74 | |
| 95% confidence interval for difference | [-39.45, 3.98] | |
| P-value [2] | 0.1086 | |
| | | |
| Week 24 | | |
| n | 77 | 75 |
| Mean (SD) | 119.39(62.72) | 166.87(124.09) |
| Median | 101.00 | 131.00 |
| Min, Max | 44.00, 353.00 | 54.00, 741.00 |
| | | |
| **Change from baseline at week 24** | | |
| n | 77 | 75 |
| Mean (SD) | 1.95(50.74) | 28.99(98.32) |
| Median | -1.00 | 8.00 |
| Min, Max | -130.00, 221.00 | -232.00, 520.00 |
| P-value [1] | 0.7917 (w) | 0.0123 (w) |
| | | |
| **ANCOVA result** | | |
| LS mean (SE) | -0.08(9.86) | 28.48(10.02) |
| LS mean difference | -28.56 | |
| 95% confidence interval for difference | [-54.04, -3.07] | |
| P-value [2] | 0.0284 | |

13) Change in systolic blood pressure at each time point of weeks 6, 12, 18, and 24 after administration of clinical trial drug compared to Visit 2 (randomization)

As a result of performing ANCOVA using the baseline value and stratification factors as covariates, the between-group differences (LS mean difference (95% CI)) in the change of systolic blood pressure were -4.07(-6.90, -1.23) mmHg at week 6, -3.06(-6.21, 0.09) mmHg at week 12, -3.36(-6.62, -0.10) mmHg at week 18, and -5.75(-9.39, -2.11) mmHg at week 24, showing statistically significant between-group differences at all time points except week 12.

**[Table 13]**

| **Change from baseline in blood pressures at weeks 6, 12, 18 and 24 - LOCF (modified full analysis set 2) [systolic blood pressure]** | | |
|---|---|---|
| | Enavogliflozin 0.3 mg (N=78) | Placebo (N=74) |
| | | |
| Baseline | | |
| n | 78 | 74 |
| Mean (SD) | 127.85(13.39) | 129.47(12.31) |
| Median | 128.50 | 129.00 |
| Min, Max | 103.00, 168.00 | 104.00, 165.00 |
| | | |
| Week 6 | | |
| n | 77 | 73 |
| Mean (SD) | 123.32(11.13) | 128.14(10.75) |
| Median | 123.00 | 129.00 |
| Min, Max | 94.00, 151.00 | 107.00, 149.00 |
| | | |
| Change from baseline at week 6 | | |
| n | 77 | 73 |
| Mean (SD) | -4.81(10.69) | -1.41(10.63) |
| Median | -5.00 | -2.00 |
| Min, Max | -32.00, 30.00 | -23.00, 28.00 |
| P-value [1] | 0.0002 (t) | 0.2606 (t) |
| | | |
| ANCOVA result | | |
| LS mean (SE) | -5.38(1.10) | -1.31(1.13) |
| LS mean difference | -4.07 | |
| 95% confidence interval for difference | [-6.90, -1.23] | |
| P-value [2] | 0.0053 | |
| | | |
| Week 12 | | |
| n | 78 | 74 |
| Mean (SD) | 122.06(12.70) | 126.15(12.52) |
| Median | 122.50 | 125.00 |
| Min, Max | 94.00, 153.00 | 105.00, 157.00 |
| | | |
| Change from baseline at week 12 | | |
| n | 78 | 74 |
| Mean (SD) | -5.78(10.97) | -3.32(10.70) |
| Median | -5.00 | -2.50 |
| Min, Max | -39.00, 21.00 | -39.00, 18.00 |
| P-value [1] | <0.0001 (t) | 0.0093 (t) |
| | | |
| ANCOVA result | | |
| LS mean (SE) | -6.37(1.22) | -3.31(1.25) |
| LS mean difference | -3.06 | |
| 95% confidence interval for difference | [-6.21, 0.09] | |
| P-value [2] | 0.0565 | |
| | | |
| Week 18 | | |
| n | 78 | 74 |
| Mean (SD) | 121.63(12.18) | 126.00(13.40) |
| Median | 121.00 | 125.50 |
| Min, Max | 95.00, 147.00 | 100.00, 155.00 |
| | | |
| Change from baseline at week 18 | | |
| n | 78 | 74 |
| Mean (SD) | -6.22(11.60) | -3.47(10.83) |
| Median | -6.50 | -2.50 |
| Min, Max | -35.00, 23.00 | -30.00, 26.00 |
| P-value [1] | <0.0001 (t) | 0.0073 (t) |
| | | |
| ANCOVA result | | |
| LS mean (SE) | -6.56(1.27) | -3.20(1.30) |
| LS mean difference | -3.36 | |
| 95% confidence interval for difference | [-6.62, -0.10] | |
| P-value [2] | 0.0435 | |
| | | |
| Week 24 | | |
| n | 78 | 74 |
| Mean (SD) | 121.29(14.23) | 128.03(13.07) |
| Median | 122.50 | 127.50 |
| Min, Max | 90.00, 157.00 | 98.00, 167.00 |
| | | |
| Change from baseline at week 24 | | |
| n | 78 | 74 |
| Mean (SD) | -6.55(13.18) | -1.45(11.37) |
| Median | -7.00 | 0.00 |
| Min, Max | -35.00, 29.00 | -36.00, 22.00 |
| P-value [1] | <0.0001 (t) | 0.2776 (t) |
| | | |
| ANCOVA result | | |
| LS mean (SE) | -7.27(1.41) | -1.52(1.45) |
| LS mean difference | -5.75 | |
| 95% confidence interval for difference | [-9.39, -2.11] | |
| P-value [2] | 0.0022 | |

14) Change in diastolic blood pressure at each time point of weeks 6, 12, 18, and 24 after administration of clinical trial drug compared to Visit 2 (randomization)

As a result of performing ANCOVA using the baseline value and stratification factors as covariates, the between-group differences (LS mean difference (95% CI)) in the change of diastolic blood pressure were -2.43(-4.65, -0.22) mmHg at week 6, -2.33(-4.53, -0.14) mmHg at week 12, -1.90(-3.98, 0.18) mmHg at week 18, and -3.12(-5.46, -0.77) mmHg at week 24, showing statistically significant between-group differences at all time points except week 18.

**[Table 14]**

| **Change from baseline in blood pressures at weeks 6, 12, 18 and 24 - LOCF (modified full analysis set 2) [diastolic blood pressure]** | | |
|---|---|---|
| | Enavogliflozin 0.3 mg (N=78) | Placebo (N=74) |
| | | |
| Baseline | | |
| n | 78 | 74 |
| Mean (SD) | 76.23(8.12) | 76.80(8.61) |
| Median | 76.00 | 77.00 |
| Min, Max | 59.00, 98.00 | 59.00, 97.00 |
| | | |
| Week 6 | | |
| n | 77 | 73 |
| Mean (SD) | 73.81(7.93) | 76.34(8.11) |
| Median | 73.00 | 76.00 |
| Min, Max | 48.00, 99.00 | 62.00, 98.00 |
| | | |
| Change from baseline at week 6 | | |
| n | 77 | 73 |
| Mean (SD) | -2.65(8.76) | -0.29(6.90) |
| Median | -3.00 | 0.00 |
| Min, Max | -20.00, 35.00 | -18.00, 16.00 |
| P-value [1] | J.0008 (w) | 0.7226 (t) |
| | | |
| ANCOVA result | | |
| LS mean (SE) | -2.99(0.86) | -0.56(0.89) |
| LS mean difference | -2.43 | |
| 95% confidence interval for difference | [-4.65, -0.22] | |
| P-value [2] | 0.0316 | |
| | | |
| Week 12 | | |
| n | 78 | 74 |
| Mean (SD) | 72.83(8.28) | 75.58(9.88) |
| Median | 72.50 | 77.00 |
| Min, Max | 49.00, 90.00 | 55.00, 104.00 |
| | | |
| Change from baseline at week 12 | | |
| n | 78 | 74 |
| Mean (SD) | -3.40(7.62) | -1.22(6.76) |
| Median | -4.00 | -1.00 |
| Min, Max | -20.00, 21.00 | -25.00, 12.00 |
| P-value [1] | 0.0002 (t) | 0.1262 (t) |
| | | |
| ANCOVA result | | |
| LS mean (SE) | -3.82(0.86) | -1.49(0.87) |
| LS mean difference | -2.33 | |
| 95% confidence interval for difference | [-4.53, -0.14] | |
| P-value [2] | 0.0376 | |
| | | |
| Week 18 | | |
| n | 78 | 74 |
| Mean (SD) | 72.60(7.47) | 74.88(9.33) |
| Median | 73.00 | 76.00 |
| Min, Max | 56.00, 89.00 | 57.00, 93.00 |
| | | |
| Change from baseline at week 18 | | |
| n | 78 | 74 |
| Mean (SD) | -3.63(7.29) | -1.92(6.91) |
| Median | -4.00 | -1.50 |
| Min, Max | -22.00, 12.00 | -19.00, 10.00 |
| P-value [1] | <0.0001 (t) | 0.0194 (t) |
| | | |
| ANCOVA result | | |
| LS mean (SE) | -4.09(0.81) | -2.19(0.83) |
| LS mean difference | -1.90 | |
| 95% confidence interval for difference | [-3.98, 0.18] | |
| P-value [2] | 0.0725 | |
| | | |
| Week 24 | | |
| n | 78 | 74 |
| Mean (SD) | 72.68(8.44) | 76.16(9.59) |
| Median | 74.00 | 76.00 |
| Min, Max | 48.00, 92.00 | 56.00, 101.00 |
| | | |
| Change from baseline at week 24 | | |
| n | 78 | 74 |
| Mean (SD) | -3.55(8.02) | -0.64(7.68) |
| Median | -4.00 | 0.50 |
| Min, Max | -24.00, 29.00 | -19.00, 23.00 |
| P-value [1] | <0.0001 (w) | 0.4791 (t) |
| | | |
| ANCOVA result | | |
| LS mean (SE) | -3.92(0.91) | -0.80(0.93) |
| LS mean difference | -3.12 | |
| 95% confidence interval for difference | [-5.46, -0.77] | |
| P-value [2] | 0.0096 | |

15) Change in β-cell function measured by HOMA-β at each time point of weeks 6, 12, 18, and 24 after administration of clinical trial drug compared to Visit 2 (randomization)

As a result of performing ANCOVA using the baseline value and stratification factors as covariates, the between-group differences (LS mean difference (95% CI)) in HOMA-β change were 3.30(-3.49, 10.09) at week 6, 12.09(-0.52, 24.70) at week 12, 22.40(-7.93, 52.73) at week 18, and 5.88(-2.31, 14.06) at week 24, showing no statistically significant between-group differences at any time point.

**[Table 15]**

| **Change from baseline in beta-cell function at weeks 6, 12, 18 and 24 - LOCF (full analysis set) [HOMA-β]** | | |
|---|---|---|
| | Enavogliflozin 0.3 mg (N=82) | Placebo (N=79) |
| Baseline | | |
| n | 82 | 79 |
| Mean (SD) | 46.40(33.07) | 51.08(45.47) |
| Median | 36.25 | 37.90 |
| Min, Max | 4.40, 184.20 | 9.90, 269.00 |
| Week 6 | | |
| n | 81 | 78 |
| Mean (SD) | 50.65(33.40) | 50.34(38.83) |
| Median | 40.10 | 39.30 |
| Min, Max | 9.60, 181.60 | 8.30, 231.80 |
| Change from baseline at week 6 | | |
| n | 81 | 78 |
| Mean (SD) | 3.95(19.40) | -0.33(28.22) |
| Median | 3.10 | 0.85 |
| Min, Max | -69.50, 54.50 | -181.80, 63.60 |
| P-value [1] | 0.0116 (w) | 0.5064 (w) |
| ANCOVA result | | |
| LS mean (SE) | 3.20(2.65) | -0.10(2.68) |
| LS mean difference | 3.30 | |
| 95% confidence interval for difference | [-3.49, 10.09] | |
| P-value [2] | 0.3383 | |
| Week 12 | | |
| n | 82 | 79 |
| Mean (SD) | 58.83(60.44) | 50.84(39.63) |
| Median | 42.30 | 41.20 |
| Min, Max | 13.80, 477.00 | 7.30, 237.70 |
| Change from baseline at week 12 | | |
| n | 82 | 79 |
| Mean (SD) | 12.44(50.57) | -0.24(28.14) |
| Median | 7.15 | -0.60 |
| Min, Max | -49.20, 424.40 | -175.80, 83.60 |
| P-value [1] | 0.0002 (w) | 0.9383 (w) |
| ANCOVA result | | |
| LS mean (SE) | 13.51(4.93) | 1.42(4.96) |
| LS mean difference | 12.09 | |
| 95% confidence interval for difference | [-0.52, 24.70] | |
| P-value [2] | 0.0600 | |
| Week 18 | | |
| n | 82 | 79 |
| Mean (SD) | 70.05(138.49) | 53.01(46.64) |
| Median | 43.85 | 39.00 |
| Min, Max | 12.60, 1260.00 | 7.30, 237.70 |
| Change from baseline at week 18 | | |
| n | 82 | 79 |
| Mean (SD) | 23.65(133.90) | 1.93(27.37) |
| Median | 5.95 | 1.50 |
| Min, Max | -44.10, 1207.40 | -64.00, 133.80 |
| P-value [1] | 0.0001 (w) | 0.5691 (w) |
| ANCOVA result | | |
| LS mean (SE) | 28.64(11.87) | 6.24(11.93) |
| LS mean difference | 22.40 | |
| 95% confidence interval for difference | [-7.93, 52.73] | |
| P-value [2] | 0.1465 | |
| Week 24 | | |
| n | 82 | 79 |
| Mean (SD) | 54.64(39.03) | 53.24(52.52) |
| Median | 46.65 | 37.00 |
| Min, Max | 14.40, 232.90 | 5.70, 248.20 |
| Change from baseline at week 24 | | |
| n | 82 | 79 |
| Mean (SD) | 8.24(25.60) | 2.16(26.46) |
| Median | 6.10 | 1.00 |
| Min, Max | -52.90, 112.50 | -58.30, 132.30 |
| P-value [1] | 0.0026 (w) | 0.9575 (w) |
| ANCOVA result | | |
| LS mean (SE) | 7.97(3.20) | 2.09(3.22) |
| LS mean difference | 5.88 | |
| 95% confidence interval for difference | [-2.31, 14.06] | |
| P-value [2] | 0.1580 | |

16) Change in insulin resistance measured by HOMA-IR at each time point of weeks 6, 12, 18, and 24 after administration of clinical trial drug compared to Visit 2 (randomization)

As a result of performing ANCOVA using the baseline value and stratification factors as covariates, the between-group differences (LS mean difference (95% CI)) in HOMA-IR change were -1.81(-2.73, -0.89) at week 6, -1.72(-2.32, -1.13) at week 12, -1.49(-2.01, -0.96) at week 18, and -1.94(-2.52, -1.37) week 24, showing statistically significant between-group differences at all time points.

**[Table 16]**

| **Change from baseline in insulin resistance at weeks 6, 12, 18 and 24 - LOCF (full analysis set) [HOMA-IR]** | | |
|---|---|---|
| | Enavogliflozin 0.3 mg (N=82) | Placebo (N=79) |
| Baseline | | |
| n | 82 | 79 |
| Mean (SD) | 3.47(2.88) | 3.61(2.57) |
| Median | 2.67 | 2.73 |
| Min, Max | 0.30, 15.90 | 0.84, 12.11 |
| Week 6 | | |
| n | 81 | 78 |
| Mean (SD) | 1.96(1.17) | 3.84(4.61) |
| Median | 1.62 | 2.50 |
| Min, Max | 0.22, 6.05 | 0.81, 36.65 |
| Change from baseline at week 6 | | |
| n | 81 | 78 |
| Mean (SD) | -1.53(2.21) | 0.23(3.90) |
| Median | -0.96 | 0.11 |
| Min, Max | -9.85, 2.84 | -5.95, 28.25 |
| P-value [1] | <0.0001 (w) | 0.8860 (w) |
| ANCOVA result | | |
| LS mean (SE) | -1.75(0.36) | 0.06(0.36) |
| LS mean difference | -1.81 | |
| 95% confidence interval for difference | [-2.73, -0.89] | |
| P-value [2] | 0.0002 | |
| Week 12 | | |
| n | 82 | 79 |
| Mean (SD) | 1.97(1.59) | 3.79(3.33) |
| Median | 1.53 | 2.78 |
| Min, Max | 0.40, 11.14 | 0.77, 18.44 |
| Change from baseline at week 12 | | |
| n | 82 | 79 |
| Mean (SD) | -1.50(1.87) | 0.18(2.41) |
| Median | -1.09 | 0.01 |
| Min, Max | -8.39, 1.11 | -6.09, 13.30 |
| P-value [1] | <0.0001 (w) | 0.8884 (w) |
| ANCOVA result | | |
| LS mean (SE) | -1.60(0.23) | 0.12(0.24) |
| LS mean difference | -1.72 | |
| 95% confidence interval for difference | [-2.32, -1.13] | |
| P-value [2] | <0.0001 | |
| Week 18 | | |
| n | 82 | 79 |
| Mean (SD) | 1.99(1.61) | 3.55(2.54) |
| Median | 1.51 | 2.76 |
| Min, Max | 0.32, 10.10 | 0.80, 12.92 |
| Change from baseline at week 18 | | |
| n | 82 | 79 |
| Mean (SD) | -1.48(1.94) | -0.06(2.43) |
| Median | -1.10 | 0.05 |
| Min, Max | -8.60, 3.24 | -5.93, 10.61 |
| P-value [1] | <0.0001 (w) | 0.8200 (w) |
| ANCOVA result | | |
| LS mean (SE) | -1.55(0.20) | -0.06(0.21) |
| LS mean difference | -1.49 | |
| 95% confidence interval for difference | [-2.01, -0.96] | |
| P-value [2] | <0.0001 | |
| Week 24 | | |
| n | 82 | 79 |
| Mean (SD) | 1.94(1.49) | 3.97(3.03) |
| Median | 1.58 | 2.88 |
| Min, Max | 0.16, 8.26 | 0.79, 16.09 |
| Change from baseline at week 24 | | |
| n | 82 | 79 |
| Mean (SD) | -1.52(2.22) | 0.36(2.21) |
| Median | -0.99 | 0.19 |
| Min, Max | -9.18, 5.11 | -4.62, 8.88 |
| P-value [1] | <0.0001 (w) | 0.1820 (w) |
| ANCOVA result | | |
| LS mean (SE) | -1.57(0.23) | 0.38(0.23) |
| LS mean difference | -1.94 | |
| 95% confidence interval for difference | [-2.52, -1.37] | |
| P-value [2] | <0.0001 | |

17) Change in renal function-related indicator measured by UACR at each time point of weeks 6, 12, 18, and 24 after administration of clinical trial drug compared to Visit 2 (randomization)

As a result of performing ANCOVA using the baseline value and stratification factors as covariates, the between-group differences (LS mean difference (95% CI)) in UACR change were 7.96(-19.51, 35.43) g/kg at week 6, -15.16(-53.39, 23.06) g/kg at week 12, -13.39(-37.46, 10.68) g/kg at week 18, and -13.92(-41.03, 13.19) g/kg at week 24, showing no statistically significant between-group differences at any time point.

**[Table 17]**

| **Change from baseline in renal function parameters at weeks 6, 12, 18 and 24 - LOCF (full analysis set) [UACR]** | | |
|---|---|---|
| | Enavogliflozin 0.3 mg (N=82) | Placebo (N=79) |
| Baseline | | |
| n | 82 | 79 |
| Mean (SD) | 28.20(68.67) | 53.92(190.18) |
| Median | 10.50 | 11.20 |
| Min, Max | 2.90, 472.80 | 3.00, 1274.70 |
| Week 6 | | |
| n | 81 | 78 |
| Mean (SD) | 32.12(92.51) | 59.97(293.55) |
| Median | 11.30 | 8.80 |
| Min, Max | 2.80, 764.00 | 1.40, 2456.40 |
| Change from baseline at week 6 | | |
| n | 81 | 78 |
| Mean (SD) | 3.66(40.92) | 5.60(141.13) |
| Median | 0.80 | -1.80 |
| Min, Max | -98.50, 291.20 | -254.60, 1181.70 |
| P-value [1] | 0.3935 (w) | 0.0004 (w) |
| ANCOVA result | | |
| LS mean (SE) | 1.45(10.64) | -6.51(10.88) |
| LS mean difference | 7.96 | |
| 95% confidence interval for difference | [-19.51, 35.43] | |
| P-value [2] | 0.5678 | |
| Week 12 | | |
| n | 82 | 79 |
| Mean (SD) | 20.43(36.73) | 74.59(355.48) |
| Median | 11.15 | 9.90 |
| Min, Max | 2.00, 245.00 | 2.30, 3018.80 |
| Change from baseline at week 12 | | |
| n | 82 | 79 |
| Mean (SD) | -7.76(33.70) | 20.67(202.55) |
| Median | 0.60 | -0.90 |
| Min, Max | -227.80, 22.20 | -172.60, 1744.10 |
| P-value [1] | 0.8295 (w) | 0.0703 (w) |
| ANCOVA result | | |
| LS mean (SE) | -4.45(14.86) | 10.72(15.10) |
| LS mean difference | -15.16 | |
| 95% confidence interval for difference | [-53.39, 23.06] | |
| P-value [2] | 0.4345 | |
| Week 18 | | |
| n | 82 | 79 |
| Mean (SD) | 19.48(37.72) | 67.26(292.96) |
| Median | 10.50 | 10.20 |
| Min, Max | 2.20, 283.30 | 2.30, 2276.80 |
| Change from baseline at week 18 | | |
| n | 82 | 79 |
| Mean (SD) | -8.72(33.85) | 13.34(126.20) |
| Median | -0.75 | -1.00 |
| Min, Max | -189.50, 27.40 | -216.60, 1002.10 |
| P-value [1] | 0.0897 (w) | 0.0293 (w) |
| ANCOVA result | | |
| LS mean (SE) | -5.53(9.36) | 7.86(9.51) |
| LS mean difference | -13.39 | |
| 95% confidence interval for difference | [-37.46, 10.68] | |
| P-value [2] | 0.2734 | |
| Week 24 | | |
| n | 82 | 79 |
| Mean (SD) | 21.44(47.37) | 67.74(281.42) |
| Median | 9.00 | 10.20 |
| Min, Max | 2.10, 384.80 | 2.30, 2288.80 |
| Change from baseline at week 24 | | |
| n | 82 | 79 |
| Mean (SD) | -6.75(42.08) | 13.82(127.61) |
| Median | -0.15 | -0.20 |
| Min, Max | -306.20, 51.50 | -232.80, 1014.10 |
| P-value [1] | 0.3996 (w) | 0.7533 (w) |
| ANCOVA result | | |
| LS mean (SE) | -4.31(10.54) | 9.61(10.71) |
| LS mean difference | -13.92 | |
| 95% confidence interval for difference | [-41.03, 13.19] | |
| P-value [2] | 0.3120 | |

18) Change in renal function-related indicator measured by UGCR at each time point of weeks 6, 12, 18, and 24 after administration of clinical trial drug compared to Visit 2 (randomization)

As a result of performing ANCOVA using the baseline value and stratification factors as covariates, the between-group differences (LS mean difference (95% CI)) in UGCR change were 52.97(46.70, 59.25) mg/mg at week 6, 51.21(45.41, 57.00) mg/mg at week 12, 47.66(42.32, 53.01) mg/mg at week 18, and 47.36(42.25, 52.48) mg/mg at week 24, showing statistically significant between-group differences at all time points.

**[Table 18]**

| **Change from baseline in renal function parameters at weeks 6, 12, 18 and 24 - LOCF (full analysis set) [UGCR]** | | |
|---|---|---|
| | Enavogliflozin 0.3 mg (N=82) | Placebo (N=79) |
| Baseline | | |
| n | 82 | 79 |
| Mean (SD) | 1.42(7.89) | 0.18(0.25) |
| Median | 0.11 | 0.10 |
| Min, Max | 0.01, 70.44 | 0.01, 1.58 |
| Week 6 | | |
| n | 81 | 78 |
| Mean (SD) | 54.67(29.72) | 0.22(0.51) |
| Median | 51.06 | 0.10 |
| Min, Max | 0.03, 199.89 | 0.01, 4.10 |
| Change from baseline at week 6 | | |
| n | 81 | 78 |
| Mean (SD) | 53.23(28.14) | 0.04(0.43) |
| Median | 50.85 | 0.00 |
| Min, Max | -0.03, 199.14 | -1.45,3.14 |
| P-value [1] | <0.0001 (w) | 0.3750 (w) |
| ANCOVA result | | |
| LS mean (SE) | 55.38(2.44) | 2.40(2.47) |
| LS mean difference | 52.97 | |
| 95% confidence interval for difference | [46.70, 59.25] | |
| P-value [2] | <0.0001 | |
| Week 12 | | |
| n | 82 | 79 |
| Mean (SD) | 53.85(26.89) | 1.18(5.08) |
| Median | 51.70 | 0.11 |
| Min, Max | 0.02, 159.79 | 0.01, 38.30 |
| Change from baseline at week 12 | | |
| n | 82 | 79 |
| Mean (SD) | 52.43(25.29) | 1.00(5.08) |
| Median | 51.21 | 0.00 |
| Min, Max | -0.04, 159.73 | -0.32, 38.20 |
| P-value [1] | <0.0001 (w) | 0.0817 (w) |
| ANCOVA result | | |
| LS mean (SE) | 52.58(2.26) | 1.37(2.27) |
| LS mean difference | 51.21 | |
| 95% confidence interval for difference | [45.41, 57.00] | |
| P-value [2] | <0.0001 | |
| Week 18 | | |
| n | 82 | 79 |
| Mean (SD) | 52.38(25.25) | 2.71(11.15) |
| Median | 54.51 | 0.10 |
| Min, Max | 0.03, 157.08 | 0.01, 78.29 |
| Change from baseline at week 18 | | |
| n | 82 | 79 |
| Mean (SD) | 50.96(22.37) | 2.53(11.12) |
| Median | 54.29 | 0.00 |
| Min, Max | -0.04, 89.36 | -0.49, 77.75 |
| P-value [1] | <0.0001 (w) | 0.4498 (w) |
| ANCOVA result | | |
| LS mean (SE) | 51.70(2.08) | 4.04(2.09) |
| LS mean difference | 47.66 | |
| 95% confidence interval for difference | [42.32, 53.01] | |
| P-value [2] | <0.0001 | |
| Week 24 | | |
| n | 82 | 79 |
| Mean (SD) | 50.40(23.40) | 1.79(6.21) |
| Median | 50.80 | 0.12 |
| Min, Max | 0.05, 117.44 | 0.01, 43.67 |
| Change from baseline at week 24 | | |
| n | 82 | 79 |
| Mean (SD) | 48.97(21.97) | 1.61(6.20) |
| Median | 50.09 | 0.02 |
| Min, Max | -0.04, 105.40 | -1.35, 43.60 |
| P-value [1] | <0.0001 (t) | 0.0035 (w) |
| ANCOVA result | | |
| LS mean (SE) | 49.76(2.00) | 2.40(2.01) |
| LS mean difference | 47.36 | |
| 95% confidence interval for difference | [42.25, 52.48] | |
| P-value [2] | <0.0001 | |

19) Change in obesity-related indicator measured by adiponectin at each time point of weeks 6, 12, 18, and 24 after administration of clinical trial drug compared to Visit 2 (randomization)

As a result of performing ANCOVA using the baseline value and stratification factors as covariates, the between-group differences (LS mean difference (95% CI)) in adiponectin change were 0.26(-0.72, 1.24) µg/mL at week 6, 0.67(-0.50, 1.83) µg/mL at week 12, 1.04(0.04, 2.05) µg/mL at week 18, and 0.98(-0.05, 2.00) µg/mL at week 24, showing a statistically significant between-group difference at week 18.

**[Table 19]**

| **Change from baseline in obesity parameters at weeks 6, 12, 18 and 24 - LOCF (full analysis set) [adiponectin]** | | |
|---|---|---|
| | Enavogliflozin 0.3 mg (N=82) | Placebo (N=79) |
| Baseline | | |
| n | 82 | 78 |
| Mean (SD) | 7.79(4.94) | 8.31(4.79) |
| Median | 5.22 | 7.34 |
| Min, Max | 1.89, 23.95 | 1.59, 24.71 |
| Week 6 | | |
| n | 81 | 78 |
| Mean (SD) | 7.91(4.42) | 8.06(4.08) |
| Median | 5.67 | 7.29 |
| Min, Max | 2.17, 27.42 | 1.35, 20.20 |
| Change from baseline at week 6 | | |
| n | 81 | 77 |
| Mean (SD) | 0.31(3.50) | -0.23(3.63) |
| Median | 0.48 | -0.03 |
| Min, Max | -12.69, 11.26 | -12.97, 14.42 |
| P-value [1] | 0.1448 (w) | 0.8034 (w) |
| ANCOVA result | | |
| LS mean (SE) | 0.22(0.38) | -0.04(0.39) |
| LS mean difference | 0.26 | |
| 95% confidence interval for difference | [-0.72, 1.24] | |
| P-value [2] | 0.6046 | |
| Week 12 | | |
| n | 82 | 79 |
| Mean (SD) | 8.57(5.06) | 8.25(5.28) |
| Median | 6.59 | 7.29 |
| Min, Max | 2.14, 22.90 | 1.58, 35.29 |
| Change from baseline at week 12 | | |
| n | 82 | 78 |
| Mean (SD) | 0.78(4.03) | -0.01(3.80) |
| Median | 0.65 | 0.02 |
| Min, Max | 12.32 | -8.98, 18.18 |
| P-value [1] | 0.0073 (w) | 0.9253 (w) |
| ANCOVA result | | |
| LS mean (SE) | 0.94(0.46) | 0.28(0.46) |
| LS mean difference | 0.67 | |
| 95% confidence interval for difference | [-0.50, 1.83] | |
| P-value [2] | 0.2620 | |
| Week 18 | | |
| n | 82 | 79 |
| Mean (SD) | 8.62(4.86) | 7.92(4.07) |
| Median | 6.76 | 5.90 |
| Min, Max | 1.95, 24.63 | 1.43, 20.12 |
| Change from baseline at week 18 | | |
| n | 82 | 78 |
| Mean (SD) | 0.83(4.07) | -0.35(3.17) |
| Median | 0.61 | -0.09 |
| Min, Max | -10.98, 17.72 | -9.34, 9.51 |
| P-value [1] | 0.0045 (w) | 0.5192 (w) |
| ANCOVA result | | |
| LS mean (SE) | 0.97(0.39) | -0.08(0.39) |
| LS mean difference | 1.04 | |
| 95% confidence interval for difference | [0.04, 2.05] | |
| P-value [2] | 0.0423 | |
| Week 24 | | |
| n | 82 | 79 |
| Mean (SD) | 9.07(5.17) | 8.45(4.66) |
| Median | 7.57 | 6.80 |
| Min, Max | 2.47, 26.69 | 1.43, 26.74 |
| Change from baseline at week 24 | | |
| n | 82 | 78 |
| Mean (SD) | 1.28(3.53) | J.19(3.35) |
| Median | 1.08 | 0.45 |
| Min, Max | -12.64, 9.69 | -8.44, 11.42 |
| P-value [1] | <0.0001 (w) | 0.2453 (w) |
| ANCOVA result | | |
| LS mean (SE) | 1.25(0.40) | J.27(0.40) |
| LS mean difference | 0.98 | |
| 95% confidence interval for difference | [-0.05, 2.00] | |
| P-value [2] | 0.0610 | |

20) Change in obesity-related indicator measured by leptin at each time point of weeks 6, 12, 18, and 24 after administration of clinical trial drug compared to Visit 2 (randomization)

As a result of performing ANCOVA using the baseline value and stratification factors as covariates, the between-group differences (LS mean difference (95% CI)) in leptin change were -2.89(-4.30, -1.47) ng/mL at week 6, -3.17(-4.66, -1.68) ng/mL at week 12, -2.36(-3.58, -1.14) ng/mL at week 18, and -2.99(-4.30, -1.68) ng/mL at week 24, showing statistically significant between-group differences at all time points.

**[Table 20]**

| **Change from baseline in obesity parameters at weeks 6, 12, 18 and 24 - LOCF (full analysis set) [leptin]** | | |
|---|---|---|
| | Enavogliflozin 0.3 mg (N=82) | Placebo (N=79) |
| Baseline | | |
| n | 82 | 78 |
| Mean (SD) | 11.16(11.33) | 13.23(10.53) |
| Median | 6.94 | 10.31 |
| Min, Max | J.96, 62.35 | 1.85, 58.91 |
| Week 6 | | |
| n | 81 | 78 |
| Mean (SD) | 8.61(8.62) | 13.08(10.44) |
| Median | 5.75 | 10.59 |
| Min, Max | J.80, 44.18 | 1.75, 57.69 |
| Change from baseline at week 6 | | |
| n | 81 | 77 |
| Mean (SD) | -2.48(4.74) | -0.09(5.48) |
| Median | -1.13 | J.13 |
| Min, Max | -20.68, 9.27 | -17.24, 14.95 |
| P-value [1] | <0.0001 (w) | 0.7437 (w) |
| ANCOVA result | | |
| LS mean (SE) | -2.82(0.55) | J.07(0.56) |
| LS mean difference | -2.89 | |
| 95% confidence interval for difference | [-4.30, -1.47] | |
| P-value [2] | <0.0001 | |
| Week 12 | | |
| n | 82 | 79 |
| Mean (SD) | 8.78(9.16) | 13.68(11.82) |
| Median | 6.26 | 11.00 |
| Min, Max | 0.65, 51.85 | 2.07, 66.21 |
| Change from baseline at week 12 | | |
| n | 82 | 78 |
| Mean (SD) | -2.38(4.83) | J.52(5.03) |
| Median | -1.15 | -0.10 |
| Min, Max | -23.85, 9.64 | -15.51, 25.24 |
| P-value [1] | <0.0001 (w) | J.6533 (w) |
| ANCOVA result | | |
| LS mean (SE) | -2.35(0.58) | J.82(0.58) |
| LS mean difference | -3.17 | |
| 95% confidence interval for difference | [-4.66, -1.68] | |
| P-value [2] | <0.0001 | |
| Week 18 | | |
| n | 82 | 79 |
| Mean (SD) | 8.96(9.20) | 12.81(9.27) |
| Median | 6.05 | 11.40 |
| Min, Max | 0.24, 60.49 | 2.41, 57.25 |
| Change from baseline at week 18 | | |
| n | 82 | 78 |
| Mean (SD) | -2.20(4.87) | -0.36(4.36) |
| Median | -1.21 | J.28 |
| Min, Max | -18.55, 12.75 | -15.51, 9.72 |
| P-value [1] | <0.0001 (w) | J.8378 (w) |
| ANCOVA result | | |
| LS mean (SE) | -2.70(0.48) | -0.34(0.48) |
| LS mean difference | -2.36 | |
| 95% confidence interval for difference | [-3.58, -1.14] | |
| P-value [2] | 0.0002 | |
| Week 24 | | |
| n | 82 | 79 |
| Mean (SD) | 9.09(8.95) | 13.70(10.82) |
| Median | 6.89 | 10.71 |
| Min, Max | 0.61, 56.42 | 2.10, 57.25 |
| Change from baseline at week 24 | | |
| n | 82 | 78 |
| Mean (SD) | -2.07(4.73) | J.53(4.40) |
| Median | -0.84 | 0.54 |
| Min, Max | -20.86, 3.70 | -15.51, 12.34 |
| P-value [1] | 0.0005 (w) | 0.0596 (w) |
| ANCOVA result | | |
| LS mean (SE) | -2.36(0.51) | J.63(0.51) |
| LS mean difference | -2.99 | |
| 95% confidence interval for difference | [-4.30, -1.68] | |
| P-value [2] | <0.0001 | |

### 3. Conclusion

As a result of the efficacy evaluation, the test group was proven to be superior to the control group in terms of the change in HbAlc at week 24 after administration of the clinical trial drug from the baseline (Visit 2), which is the primary efficacy endpoint. In addition, the items that showed statistically significant differences in the test group compared to the control group at all time points after administration of the clinical trial drug from the baseline were all items of the secondary endpoints, and changes in fasting C-peptide, body weight, fasting HDL-C, HOMA-IR, UGCR, and leptin of the exploratory endpoints. Moreover, among the exploratory endpoints, LDL-C change (at week 24), triglyceride change (at weeks 6 and 12), systolic blood pressure change (at weeks 6, 18, and 24), diastolic blood pressure change (at weeks 6, 12, and 24), and adiponectin change (at week 18) showed statistically significant differences in the test group compared to the control at some time points.

To sum up the efficacy evaluation results, it was confirmed that after administration, enavogliflozin had a glycemic control effect through HbAlc and FPG evaluations, the proportion of subjects who achieved HbAlc < 7% or HbAlc < 6.5%, which is the treatment goal recommended by the diabetes treatment guidelines, was also statistically significant, showing that enavogliflozin as monotherapy has the potential to be an effective therapeutic agent for diabetes. In addition, some items evaluated exploratively showed statistical significance, indicating that there is an indirect improvement effect on insulin resistance and obesity-related indicators.

In the safety evaluation results, there were no statistically significant differences in the occurrence rates of adverse events or adverse drug reactions, serious adverse events or serious adverse drug reactions, or adverse events that have to be carefully monitored compared to the control, and there were no adverse events or adverse drug reactions leading to discontinuation of the clinical trial drug, and no adverse events or adverse drug reactions leading to death. All adverse drug reactions of the adverse drug reactions (PT) occurring in this clinical trial, except "periodontitis," "myelosuppression," and "skin ulcer," were adverse events previously reported for the test drug enavogliflozin and similar drugs, and among them, "pyelonephritis" and "skin ulcer" were adverse events that occurred in the control, not in the test group.

Based on the above, the efficacy and safety of enavogliflozin as monotherapy in patients with type 2 diabetes were confirmed, identifying enavogliflozin as an effective therapeutic agent for diabetes for monotherapy.

### Example 2: Combined administration of enavogliflozin and metformin to patients with type 2 diabetes

### 1. Research process

Patients with type 2 diabetes showing inadequate glycemic control (7%<HbA1c≤10.5% in screening) despite receiving metformin at a constant dose (≥1,000 mg/day) for at least 8 weeks prior to Visit 1 (screening) were able to participate, and patients with type 2 diabetes who were taking metformin and other hypoglycemic agents in screening were able to participate in the trial after a washout period of at least 8 weeks (56 days), including a discontinuation period, for hypoglycemic agents other than metformin.

Subjects who met the inclusion/exclusion criteria were administered a placebo of the clinical trial drug under single-blind (subject blinding) conditions during a 2-week run-in period while continuing to receive metformin. Subjects who met the final inclusion/exclusion criteria and had compliance with both placebo and metformin of 70 to 130% during the run-in period were centrally randomized to either the test group (enavogliflozin 0.3 mg) or the control (dapagliflozin, 10 mg) at a ratio of 1:1 at Visit 2 (randomization). The randomized subjects were administered two tablets of the clinical trial drug for each administration group once a day, and then visited the institution at weeks 6, 12, 18, and 24 during the 24-week administration period to conduct efficacy and safety evaluations.

### [Inclusion criteria]

Inclusion criteria at Pre-visit (pre-screening) or Visit 1 (screening)
1. Patients with type 2 diabetes aged 19 to 80 years as of the date of obtaining written consent
2. Those who received fixed dose of metformin (≥1,000 mg/day) as monotherapy for at least 8 weeks prior to Visit 1 (screening) and had 7%≤ HbA1c* ≤ 10.5% at Visit 1 (screening)
   (subjects who were taking a fixed dose of metformin (≥1,000 mg/day) and other hypoglycemic agents had to be 6.5% ≤ HbAlc ≤ 10% at Pre-visit (pre-screening) and 7% ≤ HbA1c* ≤ 10.5% at Visit 1 (screening) after the washout period)
   (* Results from the test institution or central laboratory are available. The final decision was made based on the results from the central laboratory, but when the results from the test institution were appropriate before confirming the results from the central laboratory for the run-in, the run-in was allowed to proceed.)
3. Those with FPG < 270 mg/dL at Pre-visit (pre-screening) and Visit 1 (screening)
4. Those with BMI of 20 to 45 kg/m² at Pre-visit (pre-screening) and Visit 1 (screening)
5. Those who voluntarily decided to participate and gave written consent after hearing an explanation of the purpose, method, and effects of this clinical trial

Inclusion criteria at Visit 2 (randomization)
1. Those with 7% ≤ HbAlc ≤ 10.5% as tested at the central laboratory at Visit 1 (screening)
2. Those with placebo and metformin compliances of 70 to 130% during the run-in period confirmed at Visit 2 (randomization)

### [Exclusion criteria]

Exclusion criteria at Pre-visit (pre-screening) or Visit 1 (screening)
1. Those who have a medical history of or hypersensitivity to drugs and components including the clinical trial drug in this test, metformin, or any drug in the same class (e.g., a history of hypersensitivity to biguanide and SGLT2 inhibitor-like drugs, etc.)
2. Those with the following medical history or surgical/therapeutic history
   History of medically significant kidney disease: renal vascular occlusive disease, nephrectomy, kidney transplant, etc.
   History of major gastrointestinal surgery: total gastrectomy, total colectomy, small bowel resection, gastroenterostomy, gastrointestinal bypass, etc.
   History of acute pancreatitis or pancreatic surgery
   History of bariatric surgery within the past 2 years
   Diabetic ketoacidosis, diabetic coma or precoma within the past year
   Urinary tract infection or genital infection within the past year
   Alcohol or drug addiction within the past year
   Occurrence of acute coronary syndrome, unstable angina, myocardial infarction requiring hospitalization, stroke, transient ischemic attack, or arrhythmia within the past 24 weeks
   (However, those who had the disease more than 24 weeks prior and were currently cured or in a stable condition were allowed to enroll)
   Those who have a history of major surgery resulting in electrolyte imbalance within the past 12 weeks, or who are scheduled for major surgery within 12 weeks after the end of the clinical trial
   Those with lactic acidosis or a history of lactic acidosis
3. Those with the following diseases or symptoms
   Types of diabetes other than type 2 diabetes (type 1 diabetes, secondary diabetes, or congenital renal diabetes)
   Symptoms of urinary incontinence, anuria, oliguria, and urinary retention that are not controlled by medication due to stress incontinence, neurogenic bladder, and benign prostatic hyperplasia
   Severe diabetic complications (proliferative diabetic retinopathy, kidney disease (nephropathy) of stage 4 or higher, or severe diabetic neuropathy)
   Chronic disease requiring continued use (oral administration, injection, or inhalation) of diuretics, systemic steroids, or immunosuppressants
   Pituitary insufficiency or adrenal insufficiency
   Severe infection (e.g., severe infection requiring continued use of antibiotics or immunotherapy), clinically significant major trauma
   Unstable mental illness whose symptoms are not controlled by medication
   Severe gastrointestinal disease: active ulcers, gastrointestinal/rectal bleeding, active inflammatory bowel syndrome, patients with biliary obstruction, active gastritis not controlled by medication, etc.
   Uncontrolled high blood pressure (SBP > 180 mmHg or DBP > 110 mmHg)
   Patients with moderate to severe nephropathy (eGFR < 60 mL/min/1.73 m²)
   Patients with severe liver impairment who fall under one of the following categories:
   - AST or ALT > three times the upper limit of the normal level
   -Total bilirubin > two times the upper limit of the normal level
   - Hepatitis or liver failure
   Patients with severe hypertriglyceridemia (triglycerides > 500 mg/dL)
   Acquired immunodeficiency syndrome
   Severe heart failure (NYHA class III/IV)
   Patients with pulmonary infarction, severe pulmonary dysfunction, and other conditions prone to hypoxemia
   Those who require treatment for dehydration due to persistent diarrhea or vomiting, or are at risk of fluid volume depletion
   Those with genetic problems such as galactose intolerance, Lapp lactase deficiency, or glucose-galactose malabsorption
4. Patients who have used other clinical trial drugs/medical devices within the past 4 weeks (however, enrollment was permitted in the case of studies that, in the opinion of the investigator, were unlikely to affect the validity and safety of the study, such as observational or retrospective studies.)
5. Patients with a history of malignant tumor within the past 5 years
   - However, patients with appropriately controlled basal cell carcinoma, squamous cell carcinoma, or cervical intraepithelial carcinoma were allowed to participate.
   - However, participation was possible only when there had been no recurrence for more than 5 years after a complete cure (the time of complete removal of tumor through surgery or the completion of chemotherapy, etc.)
6. Pregnant or lactating women or those who do not agree to use appropriate contraception during the clinical trial period

Appropriate contraception for a subject or his/her partner: should agree to one of the following:
① one of sterilization surgery (vasectomy, etc.), intrauterine device (copper loop, hormone-containing intrauterine system),
② combination of non-oral hormonal contraceptives or spermicides with a barrier method, or
③ combination of a cervical cap or diaphragm together with a male condom

7. Those who are judged by the investigator to be ineligible for this clinical trial

### Exclusion criteria at Visit 1 (screening)

1. Those who have a history of taking the following drugs or are expected to require continuous administration during the clinical trial period

Systemic steroids within the past 2 weeks (more than 30 mg of prednisolone per day)

Those who have had a dose change of any drug for thyroid dysfunction within the past 6 weeks (combination use was allowed when dosing had been stable prior to enrollment in the clinical trial; dose reductions were permitted when stable)

Therapeutic agents for diabetes except metformin within the past 8 weeks

Weight loss drugs taken within the past 12 weeks

### Validation variables

### [Primary efficacy endpoint]

1. Change in HbAlc at week 24 after administration of clinical trial drug compared to Visit 2 (randomization)

### [Secondary efficacy endpoints]

1. Change in HbAlc at each time point of weeks 6, 12, and 18 after administration of clinical trial drug compared to Visit 2 (randomization)
2. Change in FPG at each time point of weeks 6, 12, 18, and 24 after administration of clinical trial drug compared to Visit 2 (randomization)
3. Proportion of subjects achieving HbAlc < 7% at each time point of weeks 6, 12, 18, and 24 after administration of clinical trial drug
4. Proportion of subjects achieving HbAlc < 6.5% at each time point of weeks 6, 12, 18, and 24 after administration of clinical trial drug
5. Proportion of subjects achieving therapeutic response (HbA1c change (HbAlc at Visit 2 - HbAlc at each evaluation time point) > 0.5% or HbAlc < 7% at each evaluation time point compared to Visit 2 (randomization)) at each time point of weeks 6, 12, 18 and 24 after administration of clinical trial drug

### [Exploratory endpoints]

1. Change in fasting c-peptide at each time point of weeks 6, 12, 18, and 24 after administration of clinical trial drug compared to Visit 2 (randomization)
2. Change in body weight at each time point of weeks 6, 12, 18, and 24 after administration of clinical trial drug compared to Visit 2 (randomization)
3. Change in fasting lipid concentration (total cholesterol, LDL-C, HDL-C, and triglycerides) at each time point of weeks 6, 12, 18, and 24 after administration of clinical trial drug compared to Visit 2 (randomization)
4. Changes in systolic or diastolic blood pressure at each time point of weeks 6, 12, 18, and 24 after administration of clinical trial drug compared to Visit 2 (randomization)
5. Changes in β-cell function and insulin resistance measured by HOMA-β and HOMA-IR at each time point of weeks 6, 12, 18, and 24 after administration of clinical trial drug compared to Visit 2 (randomization)
6. Changes in renal function-related indicators (UACR, UGCR) measured by UACR at each time point of weeks 6, 12, 18, and 24 after administration of clinical trial drug compared to Visit 2 (randomization)
7. Changes in obesity-related indicators (adiponectin, leptin) at each time point of weeks 6, 12, 18, and 24 after administration of clinical trial drug compared to Visit 2 (randomization)

### 1. Experimental results

### 1.1.1.1. Primary efficacy endpoint

### 1) Change in HbAlc at week 24 after administration of clinical trial drug compared to Visit 2 (randomization)

The result of summarizing the change in HbAlc in PPS at week 24 after administration of the clinical trial drug, which is the primary efficacy endpoint in this clinical trial, is shown in Table 21.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the HbAlc change from baseline at week 24 after administration of the clinical trial drug were -0.80(0.06)%p in the test group (enavogliflozin, 0.3 mg) and -0.75(0.06)%p in the control (10 mg of dapagliflozin), and the between-group difference (LS mean difference (95% CI)) was -0.04(-0.21, 0.12)%p. The upper limit of the 95% confidence interval for [test group-control] was 0.12%p, which was less than the non-inferiority margin of 0.35, demonstrating that the test group was not inferior to the control.

The HbAlc changes (SD) between administration groups were -0.78(0.73)%p in the test group and -0.71(0.76)%p in the control, and there were statistically significant differences in both groups (p<0.0001).

**[Table 21]**

| **Change from baseline in HbA1c at week 24 (per-protocol set)** | | |
|---|---|---|
| | Enavogliflozin 0.3 mg (N=95) | Dapagliflozin 10 mg (N=90) |
| Baseline | | |
| N | 95 | 90 |
| Mean (SD) | 7.75(0.82) | 7.68(0.73) |
| Median | 7.49 | 7.49 |
| Min, Max | 5.67, 10.21 | 5.69, 10.83 |
| Week 24 | | |
| N | 95 | 90 |
| Mean (SD) | 5.98(0.61) | 5.97(0.72) |
| Median | 6.88 | 6.84 |
| Min, Max | 5.78,9.26 | 5.85,9.85 |
| Change from baseline at week 24 | | |
| N | 95 | 90 |
| Mean (SD) | -0.78(0.73) | -0.71(0.76) |
| Median | -0.65 | -0.74 |
| Min, Max | -2.83, 1.01 | -2.85, 0.97 |
| P-value [1] | <0.0001 (w) | <0.0001 (t) |
| ANCOVA result [2] | | |
| LS mean (SE) | -0.80(0.06) | -0.75(0.06) |
| LS mean difference | -0.04 | |
| 95% confidence interval for difference | [-0.21, 0.12] | |
| Non-inferiority (UCI < 0.35%) | Yes | |

| | | |
|---|---|---|
| SD = standard deviation, Min = minimum, Max = maximum, ANCOVA = analysis of covariance, LS mean = least square mean, SE = standard error, UCI = upper limit confidence interval. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

As a result of examining the change in HbAlc at each evaluation time point after administration of the clinical trial drug from the baseline (Visit 2) in PPS, both groups showed a tendency to decrease or maintain over time.

### Secondary efficacy endpoints

### 1) Change in HbA1c at each time point of weeks 6, 12, and 18 after administration of clinical trial drug compared to Visit 2 (randomization)

The results of summarizing the HbAlc changes from baseline in PPS at weeks 6, 12, and 18 after administration of the clinical trial drug are presented in Table 22.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the HbAlc changes from the baseline at different time points after administration of the clinical trial drug were -0.68(0.04)%p in the test group (enavogliflozin, 0.3 mg) and -0.63(0.04)%p in the control (dapagliflozin, 10 mg) at week 6, -0.83(0.06)%p in the test group and -0.79(0.05)%p in the control at week 12, and -0.74(0.06)%p in the test group and -0.70(0.06)%p in the control at week 18. The between-group differences (LS mean difference/test group-control (95% CI)) were -0.05(-0.16, 0.06)%p at week 6, -0.03(-0.17, 0.11)%p at week 12, and -0.05(-0.21, 0.11)%p at week 18, showing that no statistically significant between-group differences were noted at any time point (p = 0.3806 at week 6, p = 0.6604 at week 12, and p = 0.5606 at week 18).

The HbAlc changes (SD) over time were -0.62(0.39)%p in the test group and -0.58(0.51)%p in the control at week 6, -0.77(0.55)%p in the test group and -0.74(0.69)%p in the control at week 12, and -0.71(0.67)%p in the test group and -0.66(0.76)%p in the control at week 18, showing statistically significant between-group differences at all time points and in all administration groups (p<0.0001).

**[Table 22]**

| **Change from baseline in HbA1c at weeks 6, 12 and 18 (per-protocol set)** | | |
|---|---|---|
| | Enavogliflozin 0.3 mg (N=95) | Dapagliflozin 10 mg (N=90) |
| Baseline | | |
| N | 95 | 90 |
| Mean (SD) | 7.75(0.82) | 7.68(0.73) |
| Median | 7.49 | 7.49 |
| Min, Max | 6.67, 10.21 | 5.69, 10.83 |
| Week 6 | | |
| N | 95 | 89 |
| Mean (SD) | 7.13(0.75) | 7.10(0.70) |
| Median | 5.98 | 6.98 |
| Min, Max | 5.09, 9.67 | 5.08, 10.06 |
| Change from baseline at week 6 | | |
| N | 95 | 89 |
| Mean (SD) | -0.62(0.39) | -0.58(0.51) |
| Median | -0.57 | -0.59 |
| Min, Max | -1.79, 0.24 | -2.10, 2.04 |
| P-value [1] | <0.0001 (w) | <0.0001 (w) |
| ANCOVA result | | |
| LS mean (SE) | -0.68(0.04) | -0.63(0.04) |
| LS mean difference | -0.05 | |
| 95% confidence interval for difference | [-0.16, 0.06] | |
| P-value [2] | 0.3806 | |
| Week 12 | | |
| N | 94 | 88 |
| Mean (SD) | 5.97(0.65) | 5.94(0.66) |
| Median | 5.83 | 6.83 |
| Min, Max | 5.74,9.24 | 5.74,9.73 |
| Change from baseline at week 12 | | |
| N | 94 | 88 |
| Mean (SD) | -0.77(0.55) | -0.74(0.69) |
| Median | -0.65 | -0.66 |
| Min, Max | -2.33,0.47 | -2.89, 1.71 |
| P-value [1] | <0.0001 (w) | <0.0001 (w) |
| ANCOVA result | | |
| LS mean (SE) | -0.83(0.06) | -0.79(0.05) |
| LS mean difference | -0.03 | |
| 95% confidence interval for difference | [-0.17, 0.11] | |
| P-value [2] | 0.6604 | |
| Week 18 | | |
| N | 91 | 87 |
| Mean (SD) | 7.02(0.55) | 7.02(0.73) |
| Median | 5.89 | 5.90 |
| Min, Max | 5.74, 8.80 | 5.79, 9.78 |
| Change from baseline at week 18 | | |
| N | 91 | 87 |
| Mean (SD) | -0.71(0.67) | -0.66(0.76) |
| Median | -0.56 | -0.61 |
| Min, Max | -2.72, 0.64 | -2.99, 1.67 |
| P-value [1] | <0.0001 (w) | <0.0001 (t) |
| ANCOVA result | | |
| LS mean (SE) | -0.74(0.06) | -0.70(0.06) |
| LS mean difference | -0.05 | |
| 95% confidence interval for difference | [-0.21, 0.11] | |
| P-value [2] | 0.5606 | |

| | | |
|---|---|---|
| SD = standard deviation, Min = minimum, Max = maximum, ANCOVA = analysis of covariance, LS mean = least square mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

### 2) FPG change at each time point of weeks 6, 12, 18, and 24 after administration of clinical trial drug compared to Visit 2 (randomization)

The result of summarizing FPG changes from baseline in PPS at weeks 6, 12, 18, and 24 after administration of the clinical trial drug is shown in Table 23.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the FPG changes from the baseline at different time points after administration of the clinical trial drug were -28.07(2.14) mg/dL in the test group (enavogliflozin, 0.3 mg) and -23.81(2.23) mg/dL in the control (dapagliflozin, 10 mg) at week 6, -29.70(1.96) mg/dL in the test group and -30.11(2.03) mg/dL in the control at week 12, -33.44(1.60) mg/dL in the test group and -31.13(1.63) mg/dL in the control at week 18, and -32.53(1.76) mg/dL in the test group and -29.14(1.82) mg/dL in the control at week 24. The between-group differences (LS mean difference/test group-control (95% CI)) were -4.27(-10.08, 1.55) mg/dL at week 6, 0.42(-4.90, 5.74) mg/dL at week 12, -2.31(-6.62, 2.00) mg/dL at week 18, and -3.38(-8.15, 1.39) mg/dL at week 24, showing no statistically significant between-group differences at any time point (p = 0.1491 at week 6, p = 0.8765 at week 12, p = 0.2911 at week 18, and p = 0.1633 at week 24).

The FPG changes (SD) over time were -26.91(22.00) mg/dL in the test group and -24.90(28.02) mg/dL in the control at week 6, -27.74(24.23) mg/dL in the test group and -31.89(31.22) mg/dL in the control at week 12, -31.78(23.46) mg/dL in the test group and -32.55(26.20) mg/dL in the control at week 18, and -31.77(23.03) mg/dL in the test group and -31.42(30.03) mg/dL in the control at week 24, showing statistically significant between-group differences at all time points and in all administration groups (p<0.0001).

**[Table 23]**

| **Change from baseline in FPG at weeks 6, 12, 18 and 24 (per-protocol set)** | | |
|---|---|---|
| | **Enavogliflozin 0.3 mg (N=95)** | **Dapagliflozin 10 mg (N=90)** |
| **Baseline** | | |
| N | 95 | 90 |
| Mean (SD) | 145.35(26.60) | 149.33(31.85) |
| Median | 147.00 | 142.00 |
| Min, Max | 70.00, 235.00 | 98.00, 240.00 |

| **Week 6** | | |
|---|---|---|
| N | 95 | 89 |
| Mean (SD) | 118.44(22.53) | 124.37(26.88) |
| Median | 115.00 | 122.00 |
| Min, Max | 77.00, 217.00 | 60.00, 271.00 |

| **Change from baseline at week 6** | | |
|---|---|---|
| N | 95 | 89 |
| Mean (SD) | -26.91(22.00) | -24.90(28.02) |
| Median | -27.00 | -24.00 |
| Min, Max | -112.00, 36.00 | -137.00, 42.00 |
| P-value [1] | <0.0001 (w) | <0.0001 (w) |

| **ANCOVA result** | | |
|---|---|---|
| LS mean (SE) | -28.07(2.14) | -23.81(2.23) |
| LS mean difference | -4.27 | |
| 95% confidence interval for difference | [-10.08, 1.55] | |
| P-value [2] | 0.1491 | |

| **Week 12** | | |
|---|---|---|
| N | 94 | 88 |
| Mean (SD) | 117.00(20.52) | 117.78(19.08) |
| Median | 115.50 | 116.50 |
| Min, Max | 76.00, 207.00 | 70.00, 185.00 |

| **Change from baseline at week 12** | | |
|---|---|---|
| N | 94 | 88 |
| Mean (SD) | -27.74(24.23) | -31.89(31.22) |
| Median | -26.00 | -23.50 |
| Min, Max | -113.00, 73.00 | -170.00, 25.00 |
| P-value [1] | <0.0001 (w) | <0.0001 (w) |

| **ANCOVA result** | | |
|---|---|---|
| LS mean (SE) | -29.70(1.96) | -30.11(2.03) |
| LS mean difference | 0.42 | |
| 95% confidence interval for difference | [-4.90, 5.74] | |
| P-value [2] | 0.8765 | |

| **Week 18** | | |
|---|---|---|
| N | 91 | 87 |
| Mean (SD) | 112.95(15.78) | 116.53(17.58) |
| Median | 113.00 | 115.00 |
| Min, Max | 75.00, 150.00 | 72.00, 182.00 |

| **Change from baseline at week 18** | | |
|---|---|---|
| N | 91 | 87 |
| Mean (SD) | -31.78(23.46) | -32.55(26.20) |
| Median | -31.00 | -26.00 |
| Min, Max | -126.00, 21.00 | -114.00, 5.00 |
| P-value [1] | <0.0001 (w) | <0.0001 (w) |

| **ANCOVA result** | | |
|---|---|---|
| LS mean (SE) | -33.44(1.60) | -31.13(1.63) |
| LS mean difference | -2.31 | |
| 95% confidence interval for difference | [-6.62, 2.00] | |
| P-value [2] | 0.2911 | |

| **Week 24** | | |
|---|---|---|
| N | 95 | 90 |
| Mean (SD) | 113.58(17.21) | 117.91(19.38) |
| Median | 113.00 | 116.50 |
| Min, Max | 73.00, 179.00 | 73.00, 176.00 |

| **Change from baseline at week 24** | | |
|---|---|---|
| N | 95 | 90 |
| Mean (SD) | -31.77(23.03) | -31.42(30.03) |
| Median | -31.00 | -24.00 |
| Min, Max | -127.00, 14.00 | -160.00, 15.00 |
| P-value [1] | <0.0001 (w) | <0.0001 (w) |

| **ANCOVA result** | | |
|---|---|---|
| LS mean (SE) | -32.53(1.76) | -29.14(1.82) |
| LS mean difference | -3.38 | |
| 95% confidence interval for difference | [-8.15, 1.39] | |
| P-value [2] | 0.1633 | |

| | | |
|---|---|---|
| FPG = fasting plasma glucose, SD = standard deviation, Min = minimum, Max = maximum, ANCOVA = analysis of covariance, LS mean = least square mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

### 3) Proportion of subjects achieving HbAlc < 7% at each time point of weeks 6, 12, 18, and 24 after administration of clinical trial drug

The result of summarizing the proportions of subjects achieving HbAlc < 7% in PPS at weeks 6, 12, 18, and 24 after administration of the clinical trial drug is shown in Table 24.

As a result of logistic regression corrected with stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) in PPS, compared to the control (dapagliflozin, 10 mg), the odds ratios (95% CI) for achieving HbAlc < 7% in the test group (enavogliflozin, 0.3 mg) at different time points were 1.06(0.54, 2.09) at week 6, 1.21(0.63, 2.34) at week 12, 1.29(0.67, 2.46) at week 18, and 0.97(0.52, 1.83) at week 24, showing no statistically significant between-group differences at all time points (p = 0.8592 at week 6, p = 0.5678 at week 12, p = 0.4413 at week 18, p = 0.9366 at week 24).

The proportions of subjects achieving HbAlc < 7% over time were 53.68%(51/95) in the test group and 51.69%(46/89) in the control at week 6, 68.09%(64/94) in the test group and 63.64%(56/88) in the control at week 12, 62.64%(57/91) in the test group and 57.47%(50/87) in the control at week 18, and 61.05%(58/95) in the test group and 62.22%(56/90) in the control at week 24.

**[Table 24]**

| **Proportion of subjects achieving HbAlc target of < 7% at weeks 6, 12, 18 and 24 (per-protocol set)** | | |
|---|---|---|
| | Enavogliflozin 0.3 mg (N=95) | Dapagliflozin 10 mg (N=90) |
| Proportion of subjects achieving HbA1c target of< 7% at week 6, N | 95 | 89 |
| Subjects achieving HbA1c target of< 7%, n(%) | 51(53.68) | 46(51.69) |
| Logistic regression model | | |
| Odds ratio | 1.06 | |
| 95% confidence interval | [0.54, 2.09] | |
| P-value [1] | 0.8592 | |
| Proportion of subjects achieving HbA1c target of< 7% at week 12, N | 94 | 88 |
| Subjects achieving HbA1c target of< 7%, n(%) | 64(68.09) | 56(63.64) |
| Logistic regression model | | |
| Odds ratio | 1.21 | |
| 95% confidence interval | [0.63, 2.34] | |
| P-value [1] | 0.5678 | |
| Proportion of subjects achieving HbA1c target of< 7% at week 18, N | 91 | 87 |
| Subjects achieving HbA1c target of< 7%, n(%) | 57(62.64) | 50(57.47) |
| Logistic regression model | | |
| Odds ratio | 1.29 | |
| 95% confidence interval | [0.67, 2.46] | |
| P-value [1] | 0.4413 | |
| Proportion of subjects achieving HbA1c target of< 7% at week 24, N | 95 | 90 |
| Subjects achieving HbA1c target of< 7%, n(%) | 58(61.05) | 56(62.22) |
| Logistic regression model | | |
| Odds ratio | 0.97 | |
| 95% confidence interval | [0.52, 1.83] | |
| P-value [1] | 0.9366 | |

| | | |
|---|---|---|
| Proportion = the number of subjects with HbAlc less than 7% at week n / the number of subjects with available data at week n in the efficacy analysis set * 100, n = 6, 12, 18, 24. [1] Testing for difference between DWP16001 and dapagliflozin (logistic regression model with treatment group as a factor and stratification factors (regimen [monotherapy or combination] and HbA1c level at Visit 1 [screening][< 8% or ≥ 8%])) as covariates). | | |

Data source: Listing 16.2.3:1, Listing 16.2.6:1.

### 4) Proportion of subjects achieving HbAlc < 6.5% at each time point of weeks 6, 12, 18, and 24 after administration of clinical trial drug

The result of summarizing the proportions of subjects achieving HbAlc **<** 6.5% in PPS at weeks 6, 12, 18, and 24 after administration of the clinical trial drug is shown in Table 25.

As a result of logistic regression corrected with stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)), compared to the control (dapagliflozin, 10 mg), the odds ratio (95% CI) for achieving HbAlc **<** 6.5% at different time points in the test group (enavogliflozin, 0.3 mg) was 0.83(0.35, 1.97) at week 6, 0.51(0.24, 1.09) at week 12, 0.31(0.13, 0.72) at week 18, and 0.40(0.19, 0.86) at week 24, showing statistically significant between-group differences at weeks 18 and 24 (p = 0.6733 at week 6, p = 0.0819 at week 12, p = 0.0068 at week 18, and p = 0.0188 at week 24).

The proportions of subjects achieving HbAlc **<** 6.5% at each time point were 12.63% (12/95) in the test group and 14.61% (13/89) in the control at week 6, 15.96% (15/94) in the test group and 26.14% (23/88) in the control at week 12, 9.89% (9/91) in the test group and 25.29% (22/87) in the control at week 18, and 13.68% (13/95) in the test group and 27.78% (25/90) in the control at week 24.

**[Table 25]**

| **Proportion of subjects achieving HbA1c target of < 6.5%** at **weeks 6, 12, 18 and 24 (per-protocol set)** | | |
|---|---|---|
| | Enavogliflozin 0.3 mg (N=95) | Dapagliflozin 10 mg (N=90) |
| Proportion of subjects achieving HbA1c target of< 6.5% at week 6, N | 95 | 89 |
| Subjects achieving HbA1c target of< 6.5%, n(%) | 12(12.63) | 13(14.61) |
| Logistic regression model | | |
| Odds ratio | 0.83 | |
| 95% confidence interval | [0.35, 1.97] | |
| P-value [1] | 0.6733 | |
| Proportion of subjects achieving HbA1c target of< 6.5% at week 12, N | 94 | 88 |
| Subjects achieving HbA1c target of< 6.5%, n(%) | 15(15.96) | 23(26.14) |
| Logistic regression model | | |
| Odds ratio | 0.51 | |
| 95% confidence interval | [0.24, 1.09] | |
| P-value [1] | 0.0819 | |
| Proportion of subjects achieving HbA1c target of< 6.5% at week 18, N | 91 | 87 |
| Subjects achieving HbA1c target of< 6.5%, n(%) | 9(9.89) | 22(25.29) |
| Logistic regression model | | |
| Odds ratio | 0.31 | |
| 95% confidence interval | [0.13, 0.72] | |
| P-value [1] | 0.0068 | |
| Proportion of subjects achieving HbA1c target of< 6.5% at week 24, N | 95 | 90 |
| Subjects achieving HbA1c target of< 6.5%, n(%) | 13(13.68) | 25(27.78) |
| Logistic regression model | | |
| Odds ratio | 0.40 | |
| 95% confidence interval | [0.19, 0.86] | |
| P-value [1] | 0.0188 | |

| | | |
|---|---|---|
| Proportion = the number of subjects with HbAlc less than 7% at week n / the number of subjects with available data at week n in the efficacy analysis set * 100, n = 6, 12, 18, 24. [1] Testing for difference between DWP16001 and dapagliflozin (logistic regression model with treatment group as a factor and stratification factors (regimen [monotherapy or combination] and HbA1c level at Visit 1 [screening][< 8% or ≥ 8%])) as covariates). | | |

Data source: Listing 16.2.3:1, Listing 16.2.6:1.

### 5) Proportion of subjects achieving therapeutic response (HbA1c change (HbAlc at Visit 2 - HbA1c at each evaluation time point) > 0.5% or HbAlc < 7% at each evaluation time point compared to Visit 2 (randomization)) at each time point of weeks 6, 12, 18 and 24 after administration of clinical trial drug

The result of summarizing the proportions of subjects achieving therapeutic response in PPS at weeks 6, 12, 18, and 24 after administration of the clinical trial drug is shown in Table 26.

As a result of logistic regression corrected with stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)), compared to the control (dapagliflozin, 10 mg), the odds ratio (95% CI) for achieving therapeutic response at different time points in the test group (enavogliflozin, 0.3 mg) was 0.98(0.47, 2.02) at week 6, 2.04(0.89, 4.66) at week 12, 1.58(0.76, 3.28) at week 18, and 1.30(0.63, 2.68) at week 24, showing no statistically significant between-group differences at any time point (p = 0.9484 at week 6, p = 0.0917 at week 12, p = 0.2197 at week 18, and p = 0.4776 at week 24).

The proportions of subjects achieving therapeutic response over time were 78.95% (75/95) in the test group and 79.78% (71/89) in the control at week 6, 88.30% (83/94) in the test group and 79.55% (70/88) in the control at week 12, 80.22% (73/91) in the test group and 73.56% (64/87) in the control at week 18, and 78.95% (75/95) in the test group and 75.56% (68/90) in the control at week 24.

**[Table 26]**

| **Proportion of Subjects achieving a therapeutic glycemic response at weeks 6, 12, 18 and 24 (per-protocol set)** | | |
|---|---|---|
| | Enavogliflozin 0.3 mg (N=95) | Dapagliflozin 10 mg (N=90) |
| Proportion of subjects achieving therapeutic glycemic response at week 5, N | 95 | 89 |
| Subjects achieving therapeutic glycemic response, n(%) | 75(78.95) | 71(79.78) |
| Logistic regression model | | |
| Odds ratio | J.98 | |
| 95% confidence interval | [0.47, 2.02] | |
| P-value [1] | 0.9484 | |
| Proportion of subjects achieving therapeutic glycemic response at week 12, N | 94 | 88 |
| Subjects achieving therapeutic glycemic response, n(%) | 83(88.30) | 70(79.55) |
| Logistic regression model | | |
| Odds ratio | 2.04 | |
| 95% confidence interval | [0.89, 4.66] | |
| P-value [1] | 0.0917 | |
| Proportion of subjects achieving therapeutic glycemic response at week 18, N | 91 | 87 |
| Subjects achieving therapeutic glycemic response, n(%) | 73(80.22) | 64(73.56) |
| Logistic regression model | | |
| Odds ratio | 1.58 | |
| 95% confidence interval | [0.76, 3.28] | |
| P-value [1] | 0.2197 | |
| Proportion of subjects achieving therapeutic glycemic response at week 24, N | 95 | 90 |
| Subjects achieving therapeutic glycemic response, n(%) | 75(78.95) | 68(75.56) |
| Logistic regression model | | |
| Odds ratio | 1.30 | |
| 95% confidence interval | [0.63, 2.68] | |
| P-value [1] | 0.4776 | |

| | | |
|---|---|---|
| Proportion = the number of subjects achieving a therapeutic glycemic response at week n / the number of subjects with available data at week n in the efficacy analysis set * 100, n = 6, 12, 18, 24. [1] Testing for difference between DWP16001 and dapagliflozin (logistic regression model with treatment group as a factor and stratification factors (regimen [monotherapy or combination] and HbAlc level at Visit 1 [screening][< 8% or ≥ 8%])) as covariates). | | |

Data source: Listing 16.2.3:1, Listing 16.2.6:1.

### Exploratory endpoints

**1) Change in fasting c-peptide at each time point of weeks 6, 12, 18, and 24 after administration of clinical trial drug compared to Visit 2 (randomization)**

The result of summarizing changes in fasting c-peptide from baseline in PPS at weeks 6, 12, 18, and 24 after administration of the clinical trial drug is shown in Table 27.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the change in fasting c-peptide from the baseline at different time points after administration of the clinical trial drug were -0.07(0.02) ng/mL in the test group (enavogliflozin, 0.3 mg) and -0.07(0.02) ng/mL in the control (dapagliflozin, 10 mg) at week 6, -0.08(0.02) ng/mL in the test group and -0.09(0.02) ng/mL in the control at week 12, -0.07(0.18) ng/mL in the test group and -0.06(0.19) ng/mL in the control at week 18, and -0.10(0.16) ng/mL in the test group and -0.04(0.26) ng/mL in the control at week 24. The between-group differences (LS mean difference/test group-control (95% CI)) were 0.00(-0.05, 0.04) ng/mL at week 6, 0.01(-0.05, 0.06) ng/mL at week 12, -0.02(-0.07, 0.03) ng/mL at week 18, and -0.06(-0.12, 0.00) ng/mL at week 24, showing a statistically significant between-group difference only at week 24 (p = 0.9601 at week 6, p = 0.8086 at week 12, p = 0.3689 at week 18, and p = 0.0355 at week 24).

The changes in fasting c-peptide (SD) over time were -0.06(0.19) ng/mL in the test group and -0.07(0.18) ng/mL in the control at week 6, -0.07(0.21) ng/mL in the test group and -0.08(0.18) ng/mL in the control at week 12, -0.07(0.18) in the test group and -0.06(0.19) in the control at week 18, and -0.10(0.16) ng/mL in the test group and -0.04(0.26) ng/mL in the control at week 24, showing statistically significant between-group differences at all time points and in all administration groups (p=0.0001 in the test group and the control at week 6, p<0.0001 in the test group and the control at week 12, p=0.0002 in the test group and p=0.0003 in the control at week 18, and p<0.0001 in the test group and the p=0.0016 in the control at week 24).

**[Table 27]**

| **Change from baseline in fasting c-peptide at weeks 6, 12, 18 and 24 (per-protocol set)** | | |
|---|---|---|
| | Enavogliflozin 0.3 mg (N=95) | Dapagliflozin 10 mg (N=90) |
| Baseline | | |
| n | 95 | 90 |
| Mean (SD) | 0.72(0.29) | 0.74(0.23) |
| Median | 0.66 | 0.71 |
| Min, Max | 0.30, 1.79 | 0.33, 1.39 |
| Week 6 | | |
| n | 95 | 89 |
| Mean (SD) | 0.66(0.24) | 0.66(0.20) |
| Median | 0.60 | 0.63 |
| Min, Max | 0.23, 1.46 | 0.26, 1.09 |
| Change from baseline at week 6 | | |
| n | 95 | 89 |
| Mean (SD) | -0.06(0.19) | -0.07(0.18) |
| Median | -0.03 | -0.07 |
| Min, Max | -0.86, 0.60 | -0.53, 0.46 |
| P-value [1] | 0.0001 (w) | 0.0001 (w) |
| ANCOVA result | | |
| LS mean (SE) | -0.07(0.02) | -0.07(0.02) |
| LS mean difference | 0.00 | |
| 95% confidence interval for difference | [-0.05, 0.04] | |
| P-value [2] | 0.9601 | |
| Week 12 | | |
| n | 94 | 88 |
| Mean (SD) | 0.65(0.28) | J.66(0.23) |
| Median | 0.61 | 0.65 |
| Min, Max | 0.23, 1.92 | 0.07, 1.49 |
| Change from baseline at week 12 | | |
| n | 94 | 88 |
| Mean (SD) | -0.07(0.21) | -0.08(0.18) |
| Median | -0.07 | -0.10 |
| Min, Max | -0.89, 0.93 | -0.50, 0.53 |
| P-value [1] | <0.0001 (w) | <0.0001 (t) |
| ANCOVA result | | |
| LS mean (SE) | -0.08(0.02) | -0.09(0.02) |
| LS mean difference | 0.01 | |
| 95% confidence interval for difference | [-0.05, 0.06] | |
| P-value [2] | 0.8086 | |
| Week 18 | | |
| n | 90 | 87 |
| Mean (SD) | 0.65(0.24) | J.69(0.24) |
| Median | 0.61 | 0.66 |
| Min, Max | 0.26, 1.42 | 0.13, 1.46 |
| Change from baseline at week 18 | | |
| n | 90 | 87 |
| Mean (SD) | -0.07(0.18) | -0.06(0.19) |
| Median | -0.07 | -0.03 |
| Min, Max | -0.73, 0.33 | -0.56, 0.83 |
| P-value [1] | 0.0002 (w) | 0.0003 (w) |
| ANCOVA result | | |
| LS mean (SE) | -0.08(0.02) | -0.06(0.02) |
| LS mean difference | -0.02 | |
| 95% confidence interval for difference | [-0.07, 0.03] | |
| P-value [2] | 0.3689 | |
| Week 24 | | |
| n | 95 | 90 |
| Mean (SD) | 0.62(0.22) | J.70(0.30) |
| Median | 0.56 | 0.66 |
| Min, Max | 0.26, 1.26 | 0.13, 2.09 |
| Change from baseline at week 24 | | |
| n | 95 | 90 |
| Mean (SD) | -0.10(0.16) | -0.04(0.26) |
| Median | -0.07 | -0.07 |
| Min, Max | -0.66, 0.17 | -0.63, 1.26 |
| P-value [1] | <0.0001 (w) | 0.0016 (w) |
| ANCOVA result | | |
| LS mean (SE) | -0.10(0.02) | -0.04(0.02) |
| LS mean difference | -0.06 | |
| 95% confidence interval for difference | [-0.12, 0.00] | |
| P-value [2] | 0.0355 | |

| | | |
|---|---|---|
| SD = standard deviation, Min = minimum, Max = maximum, ANCOVA = analysis of covariance, LS mean = least square mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

**2) Change in body weight at each time point of weeks 6, 12, 18, and 24 after administration of clinical trial drug compared to Visit 2 (randomization)**

The result of summarizing changes in body weight from baseline in PPS at weeks 6, 12, 18, and 24 after administration of the clinical trial drug is shown in Table 28.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the change in body weight from the baseline at different time points after administration of the clinical trial drug were -1.56(0.20) kg in the test group (enavogliflozin, 0.3 mg) and -1.61(0.21) kg in the control (dapagliflozin, 10 mg) at week 6, -2.81(0.23) kg in the test group and -2.63(0.23) kg in the control at week 12, -3.14(0.26) kg in the test group and -3.16(0.26) kg in the control at week 18, and -3.77(0.33) kg in the test group and -3.58(0.34) kg in the control at week 24. The between-group differences (LS mean difference/test group-control (95% CI)) were 0.04(-0.51, 0.59) kg at week 6, -0.19(-0.81, 0.44) kg at week 12, 0.02(-0.68, 0.71) kg at week 18, and -0.18(-1.08, 0.71) kg at week 24, showing no statistically significant between-group differences at any time point (p = 0.8846 at week 6, p = 0.5548 at week 12, p = 0.9590 a t week 18, p = 0.6840 at week 24).

The changes in body weight (SD) over time were -1.56(2.30) kg in the test group and -1.60(1.25) kg in the control at week 6, -2.78(2.48) kg in the test group and -2.61(1.66) kg in the control at week 12, -3.17(2.68) kg in the test group and -3.19(1.93) kg in the control at week 18, and -3.78(3.77) kg in the test group and -3.60(2.14) kg in the control at week 24, showing statistically significant between-group differences at all time points and in all administration groups (p<0.0001).

**[Table 28]**

| **Change from baseline in body weight at weeks 6, 12, 18 and 24 (per-protocol set)** | | |
|---|---|---|
| | Enavogliflozin 0.3 mg (N=95) | Dapagliflozin 10 mg (N=90) |
| Baseline | | |
| N | 95 | 90 |
| Mean (SD) | 70.17(11.06) | 70.52(11.81) |
| Median | 67.40 | 59.70 |
| Min, Max | 49.00, 97.00 | 44.60, 105.00 |
| Week 6 | | |
| N | 95 | 89 |
| Mean (SD) | 68.61(11.27) | 58.88(11.86) |
| Median | 67.30 | 56.90 |
| Min, Max | 45.00, 95.00 | 42.90, 103.00 |
| Change from baseline at week 6 | | |
| N | 95 | 89 |
| Mean (SD) | -1.56(2.30) | -1.60(1.25) |
| Median | -1.70 | -1.50 |
| Min, Max | -5.10, 16.20 | -6.40, 2.10 |
| P-value [1] | <0.0001 (w) | <0.0001 (w) |
| ANCOVA result | | |
| LS mean (SE) | -1.56(0.20) | -1.61(0.21) |
| LS mean difference | 0.04 | |
| 95% confidence interval for difference | [-0.51, 0.59] | |
| P-value [2] | 0.8846 | |
| Week 12 | | |
| N | 94 | 88 |
| Mean (SD) | 67.44(11.05) | 58.19(11.65) |
| Median | 55.70 | 56.90 |
| Min, Max | 44.60, 93.00 | 42.80, 100.20 |
| Change from baseline at week 12 | | |
| N | 94 | 88 |
| Mean (SD) | -2.78(2.48) | -2.61(1.66) |
| Median | -2.75 | -2.55 |
| Min, Max | -9.20, 15.20 | -10.00, 0.70 |
| P-value [1] | <0.0001 (w) | <0.0001 (w) |
| ANCOVA result | | |
| LS mean (SE) | -2.81(0.23) | -2.63(0.23) |
| LS mean difference | -0.19 | |
| 95% confidence interval for difference | [-0.81, 0.44] | |
| P-value [2] | 0.5548 | |
| Week 18 | | |
| N | 92 | 87 |
| Mean (SD) | 66.72(11.26) | 57.54(11.96) |
| Median | 64.30 | 57.00 |
| Min, Max | 44.60, 95.00 | 41.10, 98.00 |
| Change from baseline at week 18 | | |
| N | 92 | 87 |
| Mean (SD) | -3.17(2.68) | -3.19(1.93) |
| Median | -3.20 | -3.00 |
| Min, Max | -12.00, 15.20 | -12.20, 1.00 |
| P-value [1] | <0.0001 (w) | <0.0001 (w) |
| ANCOVA result | | |
| LS mean (SE) | -3.14(0.26) | -3.16(0.26) |
| LS mean difference | 0.02 | |
| 95% confidence interval for difference | [-0.68, 0.71] | |
| P-value [2] | 0.9590 | |
| Week 24 | | |
| N | 95 | 90 |
| Mean (SD) | 66.39(10.90) | 56.92(11.89) |
| Median | 64.40 | 56.05 |
| Min, Max | 44.80, 94.00 | 41.20, 99.80 |
| Change from baseline at week 24 | | |
| N | 95 | 90 |
| Mean (SD) | -3.78(3.77) | -3.60(2.14) |
| Median | -3.60 | -3.40 |
| Min, Max | -27.00, 15.20 | -16.00, 1.10 |
| P-value [1] | <0.0001 (w) | <0.0001 (w) |
| ANCOVA result | | |
| LS mean (SE) | -3.77(0.33) | -3.58(0.34) |
| LS mean difference | -0.18 | |
| 95% confidence interval for difference | [-1.08, 0.71] | |
| P-value [2] | 0.6840 | |

| | | |
|---|---|---|
| SD = standard deviation, Min = minimum, Max = maximum, ANCOVA = analysis of covariance, LS mean = least square mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

**3) Change in fasting lipid concentrations (total cholesterol, LDL-C, HDL-C, and triglycerides) at each time point of weeks 6, 12, 18, and 24 after administration of clinical trial drug compared to Visit 2 (randomization)**

### ① Total cholesterol

The result of summarizing the changes in total cholesterol from baseline in modified per-protocol set 1 (mPPS1) at weeks 6, 12, 18, and 24 after administration of the clinical trial drug is shown in Table 29.

As a result of analysis of covariance (ANCOVA) on mPPS1 with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the total cholesterol change from the baseline at different time points after administration of the clinical trial drug were 4.77(2.19) mg/dL in the test group (enavogliflozin, 0.3 mg) and 6.87(2.27) mg/dL in the control (dapagliflozin, 10 mg) at week 6, 7.26(1.98) mg/dL in the test group and 4.25(2.04) mg/dL in the control at week 12, 7.80(2.16) mg/dL in the test group and 9.57(2.20) mg/dL in the control at week 18, and 9.13(2.22) mg/dL in the test group and 6.74(2.30) mg/dL in the control at week 24. The between-group differences (LS mean difference/test group-control (95% CI)) were -2.10(-8.16, 3.95) mg/dL at week 6, 3.01(-2.45, 8.46) mg/dL at week 12, -1.77(-7.69, 4.15) mg/dL at week 18, and 2.40(-3.74, 8.53) mg/dL at week 24, showing no statistically significant between-group differences at any time point (p = 0.4940 at week 6, p = 0.2784 at week 12, p = 0.5560 at week 18, p = 0.4422 at week 24).

The changes in total cholesterol (SD) over time were 4.34(17.75) mg/dL in the test group and 7.71(22.44) mg/dL in the control at week 6, 5.74(18.70) mg/dL in the test group and 5.47(18.88) mg/dL in the control at week 12, 6.23(16.54) mg/dL in the test group and 10.18(22.74) mg/dL in the control at week 18, and 7.99(20.78) mg/dL in the test group and 9.15(22.59) mg/dL in the control at week 24, showing statistically significant between-group differences at all time points and in all administration groups (p = 0.0371 in the test group and p=0.0027 in the control at week 6, p = 0.0010 in the test group and p = 0.0003 in the control at week 12, p = 0.0007 in the test group and p <0.0001 in the control at week 18, and p <0.0001 in the test group and the control).

**[Table 29]**

| **Change from baseline in fasting lipid parameters at weeks 6, 12, 18 and 24 (modified per-protocol set 1) [Total cholesterol]** | | |
|---|---|---|
| | Enavogliflozin 0.3 mg (N=93) | Dapagliflozin 10 mg (N=87) |
| Baseline | | |
| n | 93 | 87 |
| Mean (SD) | 151.43(32.91) | 137.87(26.64) |
| Median | 150.00 | 139.00 |
| Min, Max | 98.00, 244.00 | 89.00, 212.00 |
| Week 6 | | |
| n | 93 | 86 |
| Mean (SD) | 155.77(35.37) | 145.62(31.91) |
| Median | 147.00 | 145.50 |
| Min, Max | 93.00, 255.00 | 65.00, 221.00 |
| Change from baseline at week 6 | | |
| n | 93 | 86 |
| Mean (SD) | 4.34(17.75) | 7.71(22.44) |
| Median | 3.00 | 7.00 |
| Min, Max | -43.00, 66.00 | -35.00, 118.00 |
| P-value [1] | 0.0371 (w) | 0.0027 (w) |
| ANCOVA result | | |
| LS mean (SE) | 4.77(2.19) | 6.87(2.27) |
| LS mean difference | -2.10 | |
| 95% confidence interval for difference | [-8.16, 3.95] | |
| P-value [2] | 0.4940 | |
| Week 12 | | |
| n | 92 | 85 |
| Mean (SD) | 157.39(31.50) | 143.19(28.59) |
| Median | 150.00 | 138.00 |
| Min, Max | 101.00, 257.00 | 84.00, 208.00 |
| Change from baseline at week 12 | | |
| n | 92 | 85 |
| Mean (SD) | 5.74(18.70) | 5.47(18.88) |
| Median | 6.00 | 5.00 |
| Min, Max | -56.00, 78.00 | -86.00, 38.00 |
| P-value [1] | 0.0010 (w) | 0.0003 (w) |
| ANCOVA result | | |
| LS mean (SE) | 7.26(1.98) | 4.25(2.04) |
| LS mean difference | 3.01 | |
| 95% confidence interval for difference | [-2.45, 8.46] | |
| P-value [2] | 0.2784 | |
| Week 18 | | |
| n | 88 | 84 |
| Mean (SD) | 156.99(32.54) | 148.48(30.10) |
| Median | 149.00 | 151.50 |
| Min, Max | 101.00, 250.00 | 85.00, 225.00 |
| Change from baseline at week 18 | | |
| n | 88 | 84 |
| Mean (SD) | 5.23(16.54) | 10.18(22.74) |
| Median | 5.00 | 9.00 |
| Min, Max | -28.00, 64.00 | -79.00, 88.00 |
| P-value [1] | 0.0007 (t) | <0.0001 (w) |
| ANCOVA result | | |
| LS mean (SE) | 7.80(2.16) | 9.57(2.20) |
| LS mean difference | -1.77 | |
| 95% confidence interval for difference | [-7.69, 4.15] | |
| P-value [2] | 0.5560 | |
| Week 24 | | |
| n | 93 | 87 |
| Mean (SD) | 159.42(32.17) | 147.02(27.55) |
| Median | 157.00 | 145.00 |
| Min, Max | 109.00, 267.00 | 84.00, 221.00 |
| Change from baseline at week 24 | | |
| n | 93 | 87 |
| Mean (SD) | 7.99(20.78) | 9.15(22.59) |
| Median | 11.00 | 10.00 |
| Min, Max | -91.00, 67.00 | -78.00, 118.00 |
| P-value [1] | <0.0001 (w) | <0.0001 (w) |
| ANCOVA result | | |
| LS mean (SE) | 9.13(2.22) | 6.74(2.30) |
| LS mean difference | 2.40 | |
| 95% confidence interval for difference | [-3.74, 8.53] | |
| P-value [2] | 0.4422 | |

| | | |
|---|---|---|
| SD = standard deviation, Min = minimum, Max = maximum, ANCOVA = analysis of covariance, LS mean = least square mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

### ② LDL-C

The result of summarizing the LDL-C changes from baseline in mPPS1 at weeks 6, 12, 18, and 24 after administration of the clinical trial drug is shown in Table 30.

As a result of analysis of covariance (ANCOVA) on mPPS1 with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the LDL-C change from the baseline at different time points after administration of the clinical trial drug were -0.09(1.89) mg/dL in the test group (enavogliflozin, 0.3 mg) and 4.20(1.95) mg/dL in the control (dapagliflozin, 10 mg) at week 6, 1.86(1.68) mg/dL in the test group and 0.56(1.73) mg/dL in the control at week 12, 1.42(1.86) mg/dL in the test group and 2.58(1.89) mg/dL in the control at week 18, and 1.64(1.92) mg/dL in the test group and 0.93(1.98) mg/dL in the control at week 24. The between-group differences (LS mean difference/test group-control (95% CI)) were -4.29(-9.49, 0.90) mg/dL at week 6, 1.30(-3.31, 5.92) mg/dL at week 12, -1.15(-6.22, 3.91) mg/dL at week 18, and 0.71(-4.56, 5.99) mg/dL at week 24, showing no statistically significant between-group differences at any time point (p = 0.1045 at week 6, p = 0.5783 at week 12, p = 0.6536 at week 18, p = 0.7898 at week 24).

The LDL-C changes (SD) over time were -0.67(15.12) mg/dL in the test group and 4.87(19.92) mg/dL in the control at week 6, 0.78(16.26) mg/dL in the test group and 1.33(16.02) mg/dL in the control at week 12, 0.50(14.20) mg/dL in the test group and 3.27(20.01) mg/dL in the control at week 18, and 0.88(17.04) mg/dL in the test group and 2.68(20.64) mg/dL in the control at week 24, showing statistically significant between-group differences only in the control at week 6 (p = 0.3026 in the test group and p = 0.0469 in the control at week 6, p = 0.5116 in the test group and p = 0.1260 in the control at week 12, p = 0.9665 in the test group and p = 0.0798 in the control at week 18, and p = 0.2948 in the test group and p = 0.1171 in the control at week 24).

**[Table 30]**

| **Change from baseline in fasting lipid parameters at weeks 6, 12, 18 and 24 (modified per-protocol set 1) [LDL-C]** | | |
|---|---|---|
| | Enavogliflozin 0.3 mg (N=93) | Dapagliflozin 10 mg (N=87) |
| Baseline | | |
| n | 93 | 87 |
| Mean (SD) | 86.52(30.53) | 76.16(26.27) |
| Median | 83.00 | 75.00 |
| Min, Max | 32.00, 182.00 | 19.00, 157.00 |
| Week 6 | | |
| n | 93 | 86 |
| Mean (SD) | 85.85(31.51) | 81.20(28.87) |
| Median | 79.00 | 76.50 |
| Min, Max | 28.00, 184.00 | 23.00, 155.00 |
| Change from baseline at week 6 | | |
| n | 93 | 86 |
| Mean (SD) | -0.67(15.12) | 4.87(19.92) |
| Median | -2.00 | 3.00 |
| Min, Max | -45.00, 55.00 | -37.00, 107.00 |
| P-value [1] | 0.3026 (w) | 0.0469 (w) |
| ANCOVA result | | |
| LS mean (SE) | -0.09(1.89) | 4.20(1.95) |
| LS mean difference | -4.29 | |
| 95% confidence interval for difference | [-9.49, 0.90] | |
| P-value [2] | 0.1045 | |
| Week 12 | | |
| n | 92 | 85 |
| Mean (SD) | 87.58(30.26) | 77.54(25.39) |
| Median | 79.00 | 77.00 |
| Min, Max | 38.00, 181.00 | 32.00, 130.00 |
| Change from baseline at week 12 | | |
| n | 92 | 85 |
| Mean (SD) | 0.78(16.26) | 1.33(16.02) |
| Median | 1.00 | 2.00 |
| Min, Max | -68.00, 58.00 | -97.00, 27.00 |
| P-value [1] | 0.5116 (w) | 0.1260 (w) |
| ANCOVA result | | |
| LS mean (SE) | 1.86(1.68) | J.56(1.73) |
| LS mean difference | 1.30 | |
| 95% confidence interval for difference | [-3.31, 5.92] | |
| P-value [2] | 0.5783 | |
| Week 18 | | |
| n | 88 | 84 |
| Mean (SD) | 85.75(29.78) | 79.71(26.69) |
| Median | 77.50 | 80.00 |
| Min, Max | 37.00, 166.00 | 30.00, 142.00 |
| Change from baseline at week 18 | | |
| n | 88 | 84 |
| Mean (SD) | 0.50(14.20) | 3.27(20.01) |
| Median | -1.00 | 3.00 |
| Min, Max | -26.00, 60.00 | -94.00, 74.00 |
| P-value [1] | 0.9665 (w) | 0.0798 (w) |
| ANCOVA result | | |
| LS mean (SE) | 1.42(1.86) | 2.58(1.89) |
| LS mean difference | -1.15 | |
| 95% confidence interval for difference | [-6.22, 3.91] | |
| P-value [2] | 0.6536 | |
| Week 24 | | |
| n | 93 | 87 |
| Mean (SD) | 87.40(29.52) | 78.84(25.81) |
| Median | 84.00 | 76.00 |
| Min, Max | 40.00, 174.00 | 32.00, 153.00 |
| Change from baseline at week 24 | | |
| n | 93 | 87 |
| Mean (SD) | 0.88(17.04) | 2.68(20.64) |
| Median | 3.00 | 2.00 |
| Min, Max | -84.00, 58.00 | -93.00, 117.00 |
| P-value [1] | 0.2948 (w) | 0.1171 (w) |
| ANCOVA result | | |
| LS mean (SE) | 1.64(1.92) | J.93(1.98) |
| LS mean difference | 0.71 | |
| 95% confidence interval for difference | [-4.56, 5.99] | |
| P-value [2] | 0.7898 | |

| | | |
|---|---|---|
| SD = standard deviation, Min = minimum, Max = maximum, ANCOVA = analysis of covariance, LS mean = least square mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

### ③ HDL-C

The result of summarizing the HDL-C changes from baseline in mPPS1 at weeks 6, 1,2 18, and 24 after administration of the clinical trial drug is shown in Table 31.

As a result of analysis of covariance (ANCOVA) on mPPS1 with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the HDL-C changes from the baseline at different time points after administration of the clinical trial drug were 1.11(0.58) mg/dL in the test group (enavogliflozin, 0.3 mg) and 1.60(0.61) mg/dL in the control (dapagliflozin, 10 mg) at week 6, 2.31(0.60) mg/dL in the test group and 2.25(0.62) mg/dL in the control at week 12, 2.46(0.66) mg/dL in the test group and 3.08(0.67) mg/dL in the control at week 18, and 3.06(0.64) mg/dL in the test group and 3.19(0.66) mg/dL in the control at week 24. The between-group differences (LS mean difference/test group-control (95% CI)) were -0.49(-2.10, 1.12) mg/dL at week 6, 0.06(-1.59, 1.72) mg/dL at week 12, -0.62(-2.42, 1.19) mg/dL at week 18, and -0.14(-1.90, 1.63) mg/dL at week 24, showing no statistically significant between-group differences at any time point (p = 0.5500 at week 6, p = 0.9405 at week 12, p = 0.4996 at week 18, p = 0.8788 at week 24).

The HDL-C changes over time were 1.18(5.14) mg/dL in the test group and 1.94(5.62) mg/dL in the control at week 6, 2.08(5.60) mg/dL in the test group and 2.65(5.61) mg/dL in the control at week 12, 1.89(6.20) mg/dL in the test group and 3.17(5.82) mg/dL in the control at week 18, and 2.75(6.70) mg/dL in the test group and 3.55(5.30) mg/dL in the control at week 24, showing statistically significant between-group differences at all time points and in all administration groups (p = 0.0290 in the test group and p = 0.0037 in the control at week 6, p = 0.0006 in the test group and p <0.0001 in the control at week 12, p = 0.0054 in the test group and p <0.0001 in the control at week 18, p = 0.0001 in the test group and p <0.0001 in the control at week 24).

**[Table 31]**

| **Change from baseline in fasting lipid parameters at weeks 6, 12, 18 and 24 (modified per-protocol set 1) [HDL-C]** | | |
|---|---|---|
| | Enavogliflozin 0.3 mg (N=93) | Dapagliflozin 10 mg (N=87) |
| Baseline | | |
| n | 93 | 87 |
| Mean (SD) | 50.94(10.85) | 46.90(9.17) |
| Median | 50.00 | 46.00 |
| Min, Max | 28.00, 84.00 | 26.00, 68.00 |
| Week 6 | | |
| n | 93 | 86 |
| Mean (SD) | 52.12(11.10) | 48.83(10.55) |
| Median | 51.00 | 48.50 |
| Min, Max | 29.00, 85.00 | 28.00, 81.00 |
| Change from baseline at week 6 | | |
| n | 93 | 86 |
| Mean (SD) | 1.18(5.14) | 1.94(5.62) |
| Median | 1.00 | 1.00 |
| Min, Max | -11.00, 14.00 | -10.00, 21.00 |
| P-value [1] | 0.0290 (t) | 0.0037 (w) |
| ANCOVA result | | |
| LS mean (SE) | 1.11(0.58) | 1.60(0.61) |
| LS mean difference | -0.49 | |
| 95% confidence interval for difference | [-2.10, 1.12] | |
| P-value [2] | 0.5500 | |
| Week 12 | | |
| n | 92 | 85 |
| Mean (SD) | 53.08(10.89) | 49.29(9.21) |
| Median | 52.50 | 49.00 |
| Min, Max | 29.00, 91.00 | 29.00, 78.00 |
| Change from baseline at week 12 | | |
| n | 92 | 85 |
| Mean (SD) | 2.08(5.60) | 2.65(5.61) |
| Median | 2.00 | 2.00 |
| Min, Max | -12.00, 16.00 | -10.00, 16.00 |
| P-value [1] | 0.0006 (t) | <0.0001 (t) |
| ANCOVA result | | |
| LS mean (SE) | 2.31(0.60) | 2.25(0.62) |
| LS mean difference | 0.06 | |
| 95% confidence interval for difference | [-1.59, 1.72] | |
| P-value [2] | 0.9405 | |
| Week 18 | | |
| n | 88 | 84 |
| Mean (SD) | 53.49(11.50) | 49.93(9.16) |
| Median | 52.50 | 49.00 |
| Min, Max | 28.00, 95.00 | 28.00, 70.00 |
| Change from baseline at week 18 | | |
| n | 88 | 84 |
| Mean (SD) | 1.89(6.20) | 3.17(5.82) |
| Median | 2.50 | 2.00 |
| Min, Max | -10.00, 17.00 | -11.00, 24.00 |
| P-value [1] | 0.0054 (t) | <0.0001 (w) |
| ANCOVA result | | |
| LS mean (SE) | 2.46(0.66) | 3.08(0.67) |
| LS mean difference | -0.62 | |
| 95% confidence interval for difference | [-2.42, 1.19] | |
| P-value [2] | 0.4996 | |
| Week 24 | | |
| n | 93 | 87 |
| Mean (SD) | 53.69(10.72) | 50.45(9.72) |
| Median | 53.00 | 50.00 |
| Min, Max | 30.00, 95.00 | 30.00, 77.00 |
| Change from baseline at week 24 | | |
| n | 93 | 87 |
| Mean (SD) | 2.75(6.70) | 3.55(5.30) |
| Median | 3.00 | 3.00 |
| Min, Max | -15.00, 20.00 | -8.00, 19.00 |
| P-value [1] | 0.0001 (t) | <0.0001 (t) |
| ANCOVA result | | |
| LS mean (SE) | 3.06(0.64) | 3.19(0.66) |
| LS mean difference | -0.14 | |
| 95% confidence interval for difference | [-1.90, 1.63] | |
| P-value [2] | 0.8788 | |

| | | |
|---|---|---|
| SD = standard deviation, Min = minimum, Max = maximum, ANCOVA = analysis of covariance, LS mean = least square mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

### ④ Triglycerides

The result of summarizing the triglyceride changes from baseline in mPPS1 at weeks 6, 12, 18, and 24 after administration of the clinical trial drug is shown in Table 32.

As a result of analysis of covariance (ANCOVA) on mPPS1 with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the triglyceride changes from the baseline at different time points after administration of the clinical trial drug were 8.62(6.88) mg/dL in the test group (enavogliflozin, 0.3 mg) and -10.14(7.09) mg/dL in the control (dapagliflozin, 10 mg) at week 6, -7.53(4.80) mg/dL in the test group and -10.10(4.92) mg/dL in the control at week 12, -4.99(5.99) mg/dL in the test group and 1.88(6.04) mg/dL in the control at week 18, and -5.07(5.14) mg/dL in the test group and -6.70(5.28) mg/dL in the control at week 24. The between-group differences (LS mean difference/test group-control (95% CI)) were 18.76(-0.01, 37.53) mg/dL at week 6, 2.57(-10.49, 15.62) mg/dL at week 12, -6.87(-23.09, 9.34) mg/dL at week 18, and 1.63(-12.36, 15.63) mg/dL at week 24, showing no statistically significant between-group differences at any time point (p = 0.0501 at week 6, p = 0.6984 at week 12, p = 0.4038 at week 18, and p = 0.8181 at week 24).

The triglyceride changes (SD) over time were 9.49(73.63) mg/dL in the test group and -11.86(54.93) mg/dL in the control at week 6, -5.00(57.85) mg/dL in the test group and -14.35(70.12) mg/dL in the control at week 12, -2.70(78.86) mg/dL in the test group and -4.10(62.62) mg/dL in the control at week 18, and -2.47(76.88) mg/dL in the test group and -10.64(52.51) mg/dL in the control at week 24, showing no statistically significant between-group differences at any time point or in any administration group (p = 0.8181 in the test group and p = 0.1343 in the control at week 6, p = 0.7179 in the test group and p = 0.0965 in the control at week 12, p = 0.9181 in the test group and p = 0.3730 in the control at week 18, and p = 0.7295 in the test group and p = 0.0749 in the control at week 24).

**[Table 32]**

| **Change from baseline in fasting lipid parameters at weeks 6, 12, 18 and 24 (modified per-protocol set 1) [triglycerides]** | | |
|---|---|---|
| | Enavogliflozin 0.3 mg (N=93) | Dapagliflozin 10 mg (N=87) |
| Baseline | | |
| N | 93 | 87 |
| Mean (SD) | 122.10(85.91) | 132.92(75.63) |
| Median | 101.00 | 115.00 |
| Min, Max | 53.00, 742.00 | 47.00, 580.00 |

| Week 6 | | |
|---|---|---|
| N | 93 | 86 |
| Mean (SD) | 131.59(114.93) | 120.41(53.12) |
| Median | 97.00 | 104.50 |
| Min, Max | 39.00, 799.00 | 52.00, 364.00 |

| Change from baseline at week 6 | | |
|---|---|---|
| N | 93 | 86 |
| Mean (SD) | 9.49(73.63) | -11.86(54.93) |
| Median | -3.00 | -2.00 |
| Min, Max | -101.00, 547.00 | -310.00, 135.00 |
| P-value [1] | 0.8181 (w) | 0.1343 (w) |

| ANCOVA result | | |
|---|---|---|
| LS mean (SE) | 8.62(6.88) | -10.14(7.09) |
| LS mean difference | 18.76 | |
| 95% confidence interval for difference | [-0.01, 37.53] | |
| P-value [2] | 0.0501 | |

| Week 12 | | |
|---|---|---|
| N | 92 | 85 |
| Mean (SD) | 116.23(59.46) | 119.06(50.67) |
| Median | 100.00 | 104.00 |
| Min, Max | 47.00, 429.00 | 46.00, 269.00 |

| Change from baseline at week 12 | | |
|---|---|---|
| N | 92 | 85 |
| Mean (SD) | -5.00(57.85) | -14.35(70.12) |
| Median | 3.00 | -6.00 |
| Min, Max | -313.00, 116.00 | -477.00, 124.00 |
| P-value [1] | 0.7179 (w) | 0.0965 (w) |

| ANCOVA result | | |
|---|---|---|
| LS mean (SE) | -7.53(4.80) | -10.10(4.92) |
| LS mean difference | 2.57 | |
| 95% confidence interval for difference | [-10.49, 15.62] | |
| P-value [2] | 0.6984 | |

| Week 18 | | |
|---|---|---|
| N | 88 | 84 |
| Mean (SD) | 118.10(55.93) | 130.88(70.92) |
| Median | 108.00 | 110.00 |
| Min, Max | 49.00, 402.00 | 47.00, 408.00 |

| Change from baseline at week 18 | | |
|---|---|---|
| N | 88 | 84 |
| Mean (SD) | -2.70(78.86) | -4.10(62.62) |
| Median | -2.50 | 1.00 |
| Min, Max | -485.00, 334.00 | -277.00, 232.00 |
| P-value [1] | 0.9181 (w) | 0.3730 (w) |

| ANCOVA result | | |
|---|---|---|
| LS mean (SE) | -4.99(5.99) | 1.88(6.04) |
| LS mean difference | -6.87 | |
| 95% confidence interval for difference | [-23.09, 9.34] | |
| P-value [2] | 0.4038 | |

| Week 24 | | |
|---|---|---|
| N | 93 | 87 |
| Mean (SD) | 119.62(58.93) | 122.28(58.14) |
| Median | 104.00 | 110.00 |
| Min, Max | 54.00, 404.00 | 46.00, 351.00 |

| Change from baseline at week 24 | | |
|---|---|---|
| N | 93 | 87 |
| Mean (SD) | -2.47(76.88) | -10.64(52.51) |
| Median | J.00 | -7.00 |
| Min, Max | -509.00, 196.00 | -312.00, 111.00 |
| P-value [1] | 0.7295 (w) | 0.0749 (w) |

| ANCOVA result | | |
|---|---|---|
| LS mean (SE) | -5.07(5.14) | -6.70(5.28) |
| LS mean difference | 1.63 | |
| 95% confidence interval for difference | [-12.36, 15.63] | |
| P-value [2] | 0.8181 | |

| | | |
|---|---|---|
| SD = standard deviation, Min = minimum, Max = maximum, ANCOVA = analysis of covariance, LS mean = least square mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

### 4) Changes in systolic or diastolic blood pressure at each of weeks 6, 12, 18, and 24 after administration of clinical trial drug compared to Visit 2 (randomization)

### ① Systolic blood pressure (SBP)

The result of summarizing the changes in systolic blood pressure from baseline in modified per-protocol set 2 (mPPS2) at weeks 6, 12, 18, and 24 after administration of the clinical trial drug is shown in Table 33.

As a result of analysis of covariance (ANCOVA) on mPPS2 with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the changes in systolic blood pressure from the baseline at different time points after administration of the clinical trial drug were -3.29(1.02) mmHg in the test group (enavogliflozin, 0.3 mg) and -2.71(1.06) mmHg in the control (dapagliflozin, 10 mg) at week 6, -5.86(1.04) mmHg in the test group and -6.12(1.07) mmHg in the control at week 12, -6.69(1.12) mmHg in the test group and -5.97(1.15) mmHg in the control at week 18, and -5.93(1.01) mmHg in the test group and -6.57(1.04) mmHg in the control at week 24. The between-group differences (LS mean difference/test group-control (95% CI)) were -0.58(-3.40, 2.23) mmHg at week 6, 0.26(-2.59, 3.10) mmHg at week 12, -0.72(-3.78, 2.34) mmHg at week 18, and 0.63(-2.13, 3.39) mmHg at week 24, showing no statistically significant between-group differences at any time point (p=0.6824 at week 6, p=0.8582 at week 12, p=0.6433 at week 18, and p=0.6512 at week 24).

The changes in systolic blood pressure (SD) over time were -3.28(9.34) mmHg in the test group and -1.98(11.14) mmHg in the control at week 6, -6.42(9.73) mmHg in the test group and -5.86(11.46) mmHg in the control at week 12, -7.04(10.51) mmHg in the test group and -5.43(12.97) mmHg in the control at week 18, and -6.34(9.75) mmHg in the test group and -6.03(12.13) mmHg in the control at week 24, showing statistically significant between-group differences at all time points and in all administration groups except the control at week 6 (p=0.0010 in the test group and p=0.1035 in the control at week 6, p<0.0001 in the test group and the control at week 12, p<0.0001 in the test group and p=0.0002 in the control at week 18, and p<0.0001 in the test group and the control at week 24).

**[Table 33]**

| **Change from baseline in blood pressures at weeks 6, 12, 18 and 24 (modified per-protocol set 2) [systolic blood pressure]** | | |
|---|---|---|
| | Enavogliflozin 0.3 mg (N=93) | Dapagliflozin 10 mg (N=87) |
| Baseline | | |
| n | 93 | 87 |
| Mean (SD) | 129.72(13.64) | 127.44(14.12) |
| Median | 130.00 | 127.00 |
| Min, Max | 91.00, 168.00 | 87.00, 168.00 |

| Week 6 | | |
|---|---|---|
| n | 93 | 86 |
| Mean (SD) | 126.44(14.45) | 125.24(13.01) |
| Median | 128.00 | 126.00 |
| Min, Max | 83.00, 169.00 | 89.00, 157.00 |

| Change from baseline at week 6 | | |
|---|---|---|
| n | 93 | 86 |
| Mean (SD) | -3.28(9.34) | -1.98(11.14) |
| Median | -4.00 | -1.50 |
| Min, Max | -24.00, 25.00 | -34.00, 27.00 |
| P-value [1] | 0.0010 (t) | 0.1035 (t) |

| ANCOVA result | | |
|---|---|---|
| LS mean (SE) | -3.29(1.02) | -2.71(1.06) |
| LS mean difference | -0.58 | |
| 95% confidence interval for difference | [-3.40, 2.23] | |
| P-value [2] | 0.6824 | |

| Week 12 | | |
|---|---|---|
| n | 92 | 85 |
| Mean (SD) | 123.29(13.73) | 121.46(12.64) |
| Median | 123.00 | 123.00 |
| Min, Max | 87.00, 153.00 | 84.00, 152.00 |

| Change from baseline at week 12 | | |
|---|---|---|
| n | 92 | 85 |
| Mean (SD) | -6.42(9.73) | -5.86(11.46) |
| Median | -6.00 | -6.00 |
| Min, Max | -30.00, 18.00 | -42.00, 19.00 |
| P-value [1] | <0.0001 (t) | <0.0001 (t) |

| ANCOVA result | | |
|---|---|---|
| LS mean (SE) | -5.86(1.04) | -6.12(1.07) |
| LS mean difference | 0.26 | |
| 95% confidence interval for difference | [-2.59, 3.10] | |
| P-value [2] | 0.8582 | |

| Week 18 | | |
|---|---|---|
| n | 91 | 84 |
| Mean (SD) | 122.70(12.89) | 122.27(12.91) |
| Median | 122.00 | 122.00 |
| Min, Max | 81.00, 162.00 | 91.00, 154.00 |

| Change from baseline at week 18 | | |
|---|---|---|
| n | 91 | 84 |
| Mean (SD) | -7.04(10.51) | -5.43(12.97) |
| Median | -9.00 | -5.00 |
| Min, Max | -26.00, 26.00 | -44.00, 18.00 |
| P-value [1] | <0.0001 (w) | 0.0002 (t) |

| ANCOVA result | | |
|---|---|---|
| LS mean (SE) | -6.69(1.12) | -5.97(1.15) |
| LS mean difference | -0.72 | |
| 95% confidence interval for difference | [-3.78, 2.34] | |
| P-value [2] | 0.6433 | |

| Week 24 | | |
|---|---|---|
| n | 93 | 87 |
| Mean (SD) | 123.38(12.55) | 121.40(11.90) |
| Median | 122.00 | 122.00 |
| Min, Max | 101.00, 159.00 | 86.00, 153.00 |

| Change from baseline at week 24 | | |
|---|---|---|
| n | 93 | 87 |
| Mean (SD) | -6.34(9.75) | -6.03(12.13) |
| Median | -5.00 | -7.00 |
| Min, Max | -27.00, 13.00 | -34.00, 25.00 |
| P-value [1] | <0.0001 (t) | <0.0001 (t) |

| ANCOVA result | | |
|---|---|---|
| LS mean (SE) | -5.93(1.01) | -6.57(1.04) |
| LS mean difference | 0.63 | |
| 95% confidence interval for difference | [-2.13, 3.39] | |
| P-value [2] | 0.6512 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

### ② Diastolic blood pressure (DBP)

The result of summarizing the changes in diastolic blood pressure from baseline in modified per-protocol set 2 (mPPS2) at weeks 6, 12, 18, and 24 after administration of the clinical trial drug is shown in Table 34.

As a result of analysis of covariance (ANCOVA) on mPPS2 with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for changes in diastolic blood pressure from the baseline at different time points after administration of the clinical trial drug were -2.75(0.69) mmHg in the test group (enavogliflozin, 0.3 mg) and -1.47(0.72) mmHg in the control (dapagliflozin, 10 mg) at week 6, -4.19(0.79) mmHg in the test group and -4.35(0.81) mmHg in the control at week 12, -4.80(0.78) mmHg in the test group and -4.10(0.81) mmHg in the control at week 18, and -5.41(0.65) mmHg in the test group and -4.26(0.67) mmHg in the control at week 24. The between-group differences (LS mean difference/test group-control (95% CI)) were -1.28(-3.18, 0.63) mmHg at week 6, 0.16(-2.00, 2.32) mmHg at week 12, -0.70(-2.85, 1.45) mmHg at week 18, and -1.15(-2.94, 0.63) mmHg at week 24, showing no statistically significant between-group differences at any time point (p = 0.1879 at week 6, p = 0.8819 at week 12, p =0.5201 at week 18, and p =0.2044 at week 24).

The changes in diastolic blood pressure (SD) over time were -2.80(6.99) mmHg in the test group and -1.10(7.02) mmHg in the control at week 6, -4.33(7.69) mmHg in the test group and -4.06(8.78) mmHg in the control at week 12, -5.05(6.71) mmHg in the test group and -3.94(9.82) mmHg in the control at week 18, and -5.40(5.94) mmHg in the test group and -3.87(7.93) mmHg in the control at week 24, showing statistically significant between-group differences at all time points and in all administration groups except the control at week 6 (p = 0.0002 in the test group and p=0.2694 in the control at week 6, p<0.0001 in the test group and the control at week 12, p<0.0001 in the test group and p=0.0004 in the control at week 18, and p<0.0001 in the test group and the control at week 24).

**[Table 34]**

| **Change from baseline in blood pressures at weeks 6, 12, 18 and 24 (modified per-protocol set 2) [diastolic blood pressure]** | | |
|---|---|---|
| | Enavogliflozin 0.3 mg (N=93) | Dapagliflozin 10 mg (N=87) |
| Baseline | | |
| n | 93 | 87 |
| Mean (SD) | 77.04(9.72) | 75.92(10.50) |
| Median | 77.00 | 75.00 |
| Min, Max | 55.00, 103.00 | 49.00, 107.00 |

| Week 6 | | |
|---|---|---|
| n | 93 | 86 |
| Mean (SD) | 74.25(10.12) | 74.57(9.02) |
| Median | 74.00 | 75.00 |
| Min, Max | 36.00, 104.00 | 50.00, 102.00 |

| Change from baseline at week 6 | | |
|---|---|---|
| n | 93 | 86 |
| Mean (SD) | -2.80(6.99) | -1.10(7.02) |
| Median | -3.00 | 0.00 |
| Min, Max | -19.00, 10.00 | -26.00, 14.00 |
| P-value [1] | 0.0002 (t) | 0.2694 (w) |

| ANCOVA result | | |
|---|---|---|
| LS mean (SE) | -2.75(0.69) | -1.47(0.72) |
| LS mean difference | -1.28 | |
| 95% confidence interval for difference | [-3.18, 0.63] | |
| P-value [2] | 0.1879 | |

| Week 12 | | |
|---|---|---|
| n | 92 | 85 |
| Mean (SD) | 72.66(10.22) | 71.86(8.61) |
| Median | 72.00 | 72.00 |
| Min, Max | 48.00, 96.00 | 50.00, 95.00 |

| Change from baseline at week 12 | | |
|---|---|---|
| n | 92 | 85 |
| Mean (SD) | -4.33(7.69) | -4.06(8.78) |
| Median | -4.00 | -3.00 |
| Min, Max | -23.00, 15.00 | -26.00, 19.00 |
| P-value [1] | <0.0001 (t) | <0.0001 (w) |

| ANCOVA result | | |
|---|---|---|
| LS mean (SE) | -4.19(0.79) | -4.35(0.81) |
| LS mean difference | 0.16 | |
| 95% confidence interval for difference | [-2.00, 2.32] | |
| P-value [2] | 0.8819 | |

| Week 18 | | |
|---|---|---|
| n | 91 | 84 |
| Mean (SD) | 71.85(9.37) | 72.08(9.06) |
| Median | 71.00 | 71.00 |
| Min, Max | 44.00, 98.00 | 45.00, 94.00 |

| Change from baseline at week 18 | | |
|---|---|---|
| n | 91 | 84 |
| Mean (SD) | -5.05(6.71) | -3.94(9.82) |
| Median | -5.00 | -2.50 |
| Min, Max | -19.00, 15.00 | -34.00, 14.00 |
| P-value [1] | <0.0001 (t) | 0.0004 (t) |

| ANCOVA result | | |
|---|---|---|
| LS mean (SE) | -4.80(0.78) | -4.10(0.81) |
| LS mean difference | -0.70 | |
| 95% confidence interval for difference | [-2.85, 1.45] | |
| P-value [2] | 0.5201 | |

| Week 24 | | |
|---|---|---|
| n | 93 | 87 |
| Mean (SD) | 71.65(9.39) | 72.05(8.44) |
| Median | 71.00 | 71.00 |
| Min, Max | 55.00, 101.00 | 45.00, 89.00 |

| Change from baseline at week 24 | | |
|---|---|---|
| n | 93 | 87 |
| Mean (SD) | -5.40(5.94) | -3.87(7.93) |
| Median | -6.00 | -4.00 |
| Min, Max | -18.00, 11.00 | -22.00, 14.00 |
| P-value [1] | <0.0001 (t) | <0.0001 (t) |

| ANCOVA result | | |
|---|---|---|
| LS mean (SE) | -5.41(0.65) | -4.26(0.67) |
| LS mean difference | -1.15 | |
| 95% confidence interval for difference | [-2.94, 0.63] | |
| P-value [2] | 0.2044 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

**5) Changes in β-cell function and insulin resistance measured by HOMA-β and HOMA-IR at each of weeks 6, 12, 18, and 24 after administration of clinical trial drug compared to Visit 2 (randomization)**

### ① β-cell function measured by HOMA-β

The result of summarizing the changes in HOMA-β from baseline in PPS at weeks 6, 12, 18, and 24 after administration of the clinical trial drug is shown in Table 35.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for changes in HOMA-β from the baseline at different time points after administration of the clinical trial drug were 7.22(5.44) in the test group (enavogliflozin, 0.3 mg) and -1.79(5.59) in the control (dapagliflozin, 10 mg) at week 6, 5.57(3.08) in the test group and 8.75(3.15) in the control at week 12, 10.97(3.06) in the test group and 13.49(3.08) in the control at week 18, and 6.94(3.17) in the test group and 11.93(3.25) in the control at week 24. The between-group differences (LS mean difference/test group-control (95% CI)) were 9.01(-5.78, 23.80) at week 6, -3.17(-11.52, 5.17) at week 12, -2.53(-10.76, 5.71) at week 18, and -4.99(-13.60, 3.62) at week 24, showing no statistically significant between-group differences at any time point (p=0.2307 at week 6, p=0.4542 at week 12, p=0.5459 at week 18, and p=0.2545 at week 24).

The HOMA-β changes (SD) over time were 5.87(22.04) in the test group and -2.83(69.39) in the control at week 6, 5.59(26.08) in the test group and 8.97(32.68) in the control at week 12, 10.63(28.28) in the test group and 13.46(27.25) in the control at week 18, and 6.80(28.34) in the test group and 12.23(31.72) in the control at week 24, showing statistically significant between-group differences at all time points and in all administration groups except the control at week 6 (p<0.0001 in the test group and p=0.1818 in the control at week 6, p=0.0003 in the test group and p=0.0027 in the control at week 12, p<0.0001 in the test group and the control at week 18, and p=0.0069 in the test group and p=0.0002 in the control at week 24).

**[Table 35]**

| **Change from baseline in beta-cell function at weeks 6, 12, 18 and 24 (per-protocol set) [HOMA-β]** | | |
|---|---|---|
| | Enavogliflozin 0.3 mg (N=95) | Dapagliflozin 10 mg (N=90) |
| Baseline | | |
| n | 95 | 90 |
| Mean (SD) | 45.53(36.34) | 44.44(25.03) |
| Median | 35.00 | 38.75 |
| Min, Max | 7.70, 210.90 | 13.70, 178.60 |
| Week 6 | | |
| n | 95 | 89 |
| Mean (SD) | 51.39(36.66) | 41.10(71.67) |
| Median | 43.00 | 42.20 |
| Min, Max | 12.30, 236.60 | -576.00, 154.10 |
| Change from baseline at week 6 | | |
| n | 95 | 89 |
| Mean (SD) | 5.87(22.04) | -2.83(69.39) |
| Median | 8.10 | 1.10 |
| Min, Max | -72.30, 71.80 | -627.00, 74.70 |
| P-value [1] | <0.0001 (w) | 0.1818 (w) |
| ANCOVA result | | |
| LS mean (SE) | 7.22(5.44) | -1.79(5.59) |
| LS mean difference | 9.01 | |
| 95% confidence interval for difference | [-5.78, 23.80] | |
| P-value [2] | 0.2307 | |
| Week 12 | | |
| n | 94 | 88 |
| Mean (SD) | 51.45(35.30) | 53.77(37.80) |
| Median | 41.60 | 45.50 |
| Min, Max | 7.10, 192.30 | 12.20, 288.00 |
| Change from baseline at week 12 | | |
| n | 94 | 88 |
| Mean (SD) | 5.59(26.08) | 8.97(32.68) |
| Median | 5.10 | 4.55 |
| Min, Max | -97.10, 95.10 | -41.10, 252.00 |
| P-value [1] | 0.0003 (w) | 0.0027 (w) |
| ANCOVA result | | |
| LS mean (SE) | 5.57(3.08) | 8.75(3.15) |
| LS mean difference | -3.17 | |
| 95% confidence interval for difference | [-11.52, 5.17] | |
| P-value [2] | 0.4542 | |
| Week 18 | | |
| n | 90 | 87 |
| Mean (SD) | 56.39(44.68) | 58.55(35.39) |
| Median | 40.45 | 51.80 |
| Min, Max | 9.80, 223.40 | 10.00, 168.50 |
| Change from baseline at week 18 | | |
| n | 90 | 87 |
| Mean (SD) | 10.63(28.28) | 13.46(27.25) |
| Median | 5.75 | 9.80 |
| Min, Max | -83.70, 102.10 | -53.10, 128.90 |
| P-value [1] | <0.0001 (w) | <0.0001 (w) |
| ANCOVA result | | |
| LS mean (SE) | 10.97(3.06) | 13.49(3.08) |
| LS mean difference | -2.53 | |
| 95% confidence interval for difference | [-10.76, 5.71] | |
| P-value [2] | 0.5459 | |
| Week 24 | | |
| n | 95 | 90 |
| Mean (SD) | 52.32(40.75) | 56.67(38.17) |
| Median | 42.00 | 46.75 |
| Min, Max | 9.90, 189.00 | 14.00, 228.30 |
| Change from baseline at week 24 | | |
| n | 95 | 90 |
| Mean (SD) | 5.80(28.34) | 12.23(31.72) |
| Median | 4.00 | 5.30 |
| Min, Max | -79.80, 127.90 | -50.50, 148.70 |
| P-value [1] | 0.0069 (w) | 0.0002 (w) |
| ANCOVA result | | |
| LS mean (SE) | 5.94(3.17) | 11.93(3.25) |
| LS mean difference | -4.99 | |
| 95% confidence interval for difference | [-13.60, 3.62] | |
| P-value [2] | 0.2545 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

### ② Insulin resistance detected by HOMA-IR

The result of summarizing the changes in HOMA-IR from baseline in PPS at weeks 6, 12, 18, and 24 after administration of the clinical trial drug is shown in Table 36.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for HOMA-IR changes from the baseline at different time points after administration of the clinical trial drug were -1.47(0.14) in the test group (enavogliflozin, 0.3 mg) and -1.31(0.15) in the control (dapagliflozin, 10 mg) at week 6, -1.58(0.17) in the test group and -1.60(0.17) in the control at week 12, -1.79(0.11) in the test group and -1.47(0.12) in the control at week 18, and -1.85(0.13) in the test group and -1.31(0.14) in the control at week 24. The between-group differences (LS mean difference/test group-control (95% CI)) were -0.16(-0.55, 0.22) at week 6, 0.02(-0.43, 0.47) at week 12, -0.32(-0.63, -0.01) at week 18, and -0.53(-0.90, -0.17) at week 24, showing statistically significant between-group differences only at week 18 and 24 (p=0.4053 at week 6, p=0.9442 at week 12, p=0.0420 at week 18, and p=0.0041 at week 24).

The HOMA-IR changes (SD) over time were -1.35(3.16) in the test group and -1.46(1.88) in the control at week 6, -1.36(3.30) in the test group and -1.74(2.16) in the control at week 12, -1.58(3.10) in the test group and -1.61(2.09) in the control at week 18, and -1.69(2.98) in the test group and -1.47(2.30) in the control at week 24, showing statistically significant between-group differences at all time points and in all administration groups (p<0.0001).

**[Table 36]**

| **Change from baseline in insulin resistance at weeks 6, 12, 18 and 24 (per-protocol set) [HOMA-IR]** | | |
|---|---|---|
| | Enavogliflozin 0.3 mg (N=95) | Dapagliflozin 10 mg (N=90) |
| Baseline | | |
| n | 95 | 90 |
| Mean (SD) | 3.45(3.42) | 3.85(2.49) |
| Median | 2.67 | 3.05 |
| Min, Max | 0.38, 29.59 | 0.68, 11.24 |
| Week 6 | | |
| n | 95 | 89 |
| Mean (SD) | 2.10(1.51) | 2.34(1.48) |
| Median | 1.72 | 2.07 |
| Min, Max | 0.41, 8.55 | 0.55, 8.97 |
| Change from baseline at week 6 | | |
| n | 95 | 89 |
| Mean (SD) | -1.35(3.16) | -1.46(1.88) |
| Median | -0.91 | -1.03 |
| Min, Max | -26.74, 6.38 | -8.53, 3.61 |
| P-value [1] | <0.0001 (w) | <0.0001 (w) |
| ANCOVA result | | |
| LS mean (SE) | -1.47(0.14) | -1.31(0.15) |
| LS mean difference | -0.16 | |
| 95% confidence interval for difference | [-0.55, 0.22] | |
| P-value [2] | 0.4053 | |
| Week 12 | | |
| n | 94 | 88 |
| Mean (SD) | 2.10(1.97) | 2.17(1.29) |
| Median | 1.61 | 1.89 |
| Min, Max | 0.37, 15.90 | 0.47, 6.76 |
| Change from baseline at week 12 | | |
| n | 94 | 88 |
| Mean (SD) | -1.36(3.30) | -1.74(2.16) |
| Median | -0.99 | -1.27 |
| Min, Max | -24.47, 12.69 | -9.52, 2.06 |
| P-value [1] | <0.0001 (w) | <0.0001 (w) |
| ANCOVA result | | |
| LS mean (SE) | -1.58(0.17) | -1.60(0.17) |
| LS mean difference | 0.02 | |
| 95% confidence interval for difference | [-0.43, 0.47] | |
| P-value [2] | 0.9442 | |
| Week 18 | | |
| n | 90 | 87 |
| Mean (SD) | 1.86(1.15) | 2.28(1.28) |
| Median | 1.51 | 2.07 |
| Min, Max | 0.39, 6.09 | 0.36, 6.47 |
| Change from baseline at week 18 | | |
| n | 90 | 87 |
| Mean (SD) | -1.58(3.10) | -1.61(2.09) |
| Median | -0.94 | -1.07 |
| Min, Max | -26.87, 1.34 | -8.17, 4.40 |
| P-value [1] | <0.0001 (w) | <0.0001 (w) |
| ANCOVA result | | |
| LS mean (SE) | -1.79(0.11) | -1.47(0.12) |
| LS mean difference | -0.32 | |
| 95% confidence interval for difference | [-0.63, -0.01] | |
| P-value [2] | 0.0420 | |
| Week 24 | | |
| n | 95 | 90 |
| Mean (SD) | 1.76(1.07) | 2.39(1.67) |
| Median | 1.48 | 1.82 |
| Min, Max | 0.48, 5.34 | 0.42, 9.95 |
| Change from baseline at week 24 | | |
| n | 95 | 90 |
| Mean (SD) | -1.69(2.98) | -1.47(2.30) |
| Median | -1.06 | -1.17 |
| Min, Max | -26.66, 0.77 | -8.89, 6.19 |
| P-value [1] | <0.0001 (w) | <0.0001 (w) |
| ANCOVA result | | |
| LS mean (SE) | -1.85(0.13) | -1.31(0.14) |
| LS mean difference | -0.53 | |
| 95% confidence interval for difference | [-0.90, -0.17] | |
| P-value [2] | 0.0041 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

**6) Changes in renal function-related indicators (UACR, UGCR) at each of weeks 6, 12, 18, and 24 after administration of clinical trial drug compared to Visit 2 (randomization)**

### ① UACR

The result of summarizing the UACR changes from baseline in PPS at weeks 6, 12, 18, and 24 after administration of the clinical trial drug is shown in Table 37.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for changes in UACR from the baseline at different time points after administration of the clinical trial drug were -12.75(10.48) g/kg in the test group (enavogliflozin, 0.3 mg) and 8.13(10.88) g/kg in the control (dapagliflozin, 10 mg) at week 6, -21.65(7.38) g/kg in the test group and -3.38(7.57) g/kg in the control at week 12, -23.05(5.06) g/kg in the test group and -12.95(5.11) g/kg in the control at week 18, and -24.29(3.38) g/kg in the test group and -17.37(3.48) g/kg in the control at week 24. The between-group differences (LS mean difference/test group-control (95% CI)) were -20.88(-49.57, 7.82) at week 6, -18.27(-38.36, 1.82) at week 12, -10.10(-23.81, 3.61) at week 18, and -6.93(-16.14, 2.29) at week 24, showing no statistically significant between-group differences at any time point (p=0.1528 at week 6, p=0.0744 at week 12, p=0.1479 at week 18, and p=0.1399 at week 24).

The UACR changes (SD) over time were -11.44(123.47) g/kg in the test group and 4.49(84.95) g/kg in the control at week 6, -18.55(130.70) g/kg in the test group and -8.38(40.82) g/kg in the control at week 12, -17.48(143.19) g/kg in the test group and -20.54(102.64) g/kg in the control at week 18, and -18.27(144.42) g/kg in the test group and -25.76(128.93) g/kg in the control at week 24, showing statistically significant between-group differences at all time points and in all administration groups except the test group and the control at week 6 and the test group at week 18 (p=0.7353 in the test group and p=0.7282 in the control at week 6, p=0.0070 in the test group and p=0.0090 in the control at week 12, p=0.0656 in the test group and p=0.0067 in the control at week 18, and p=0.0270 in the test group and p=0.0013 in the control at week 24).

**[Table 37]**

| **Change from baseline in renal function parameters at weeks 6, 12, 18 and 24 (per-protocol set) [UACR]** | | |
|---|---|---|
| | **Enavogliflozin 0.3 mg (N=95)** | **Dapagliflozin 10 mg (N=90)** |
| **Baseline** | | |
| n | 95 | 89 |
| Mean (SD) | 38.19(163.98) | 57.42(198.64) |
| Median | 10.10 | 15.50 |
| Min, Max | 2.80, 1600.00 | 2.10, 1474.70 |

| **Week 6** | | |
|---|---|---|
| **n** | 95 | 87 |
| Mean (SD) | 26.76(57.01) | 52.95(236.30) |
| Median | 11.40 | 13.90 |
| Min, Max | 3.70, 433.30 | 2.20, 1752.20 |

| **Change from baseline at week 6** | | |
|---|---|---|
| n | 95 | 87 |
| Mean (SD) | -11.44(123.47) | 4.49(84.95) |
| Median | J.00 | J.00 |
| Min, Max | -1166.70, 221.90 | -375.80, 639.50 |
| P-value [1] | 0.7353 (w) | 0.7282 (w) |

| **ANCOVA result** | | |
|---|---|---|
| LS mean (SE) | -12.75(10.48) | 8.13(10.88) |
| LS mean difference | -20.88 | |
| 95% confidence interval for difference | [-49.57, 7.82] | |
| P-value [2] | 0.1528 | |

| **Week 12** | | |
|---|---|---|
| n | 94 | 88 |
| Mean (SD) | 19.80(37.19) | 49.42(183.74) |
| Median | 11.05 | 11.95 |
| Min, Max | 1.90, 341.00 | 1.70, 1402.80 |

| **Change from baseline at week 12** | | |
|---|---|---|
| n | 94 | 87 |
| Mean (SD) | -18.55(130.70) | -8.38(40.82) |
| Median | -1.40 | -1.80 |
| Min, Max | -1259.00, 74.90 | -311.30, 113.00 |
| P-value [1] | 0.0070 (w) | 0.0090 (w) |

| **ANCOVA result** | | |
|---|---|---|
| LS mean (SE) | -21.65(7.38) | -3.38(7.57) |
| LS mean difference | -18.27 | |
| 95% confidence interval for difference | [-38.36, 1.82] | |
| P-value [2] | 0.0744 | |

| **Week 18** | | |
|---|---|---|
| n | 90 | 87 |
| Mean (SD) | 20.32(34.13) | 37.26(113.12) |
| Median | 9.80 | 11.60 |
| Min, Max | 3.30, 260.90 | 1.40, 983.60 |

| **Change from baseline at week 18** | | |
|---|---|---|
| n | 90 | 86 |
| Mean (SD) | -17.48(143.19) | -20.54(102.64) |
| Median | -0.70 | -1.75 |
| Min, Max | -1339.10, 129.10 | -775.20, 78.60 |
| P-value [1] | 0.0656 (w) | 0.0067 (w) |

| **ANCOVA result** | | |
|---|---|---|
| LS mean (SE) | -23.05(5.06) | -12.95(5.11) |
| LS mean difference | -10.10 | |
| 95% confidence interval for difference | [-23.81, 3.61] | |
| P-value [2] | 0.1479 | |

| **Week 24** | | |
|---|---|---|
| n | 95 | 90 |
| Mean (SD) | 19.93(29.10) | 31.37(76.71) |
| Median | 10.20 | 11.10 |
| Min, Max | 2.50, 207.40 | 3.20, 650.70 |

| **Change from baseline at week 24** | | |
|---|---|---|
| n | 95 | 89 |
| Mean (SD) | -18.27(144.42) | -25.76(128.93) |
| Median | -1.10 | -1.70 |
| Min, Max | -1392.60, 131.90 | -850.60, 48.60 |
| P-value [1] | 0.0270 (w) | 0.0013 (w) |

| **ANCOVA result** | | |
|---|---|---|
| LS mean (SE) | -24.29(3.38) | -17.37(3.48) |
| LS mean difference | -6.93 | |
| 95% confidence interval for difference | [-16.14, 2.29] | |
| P-value [2] | 0.1399 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

### ② UGCR

The result of summarizing the UGCR changes from baseline in PPS at weeks 6, 12, 18, and 24 after administration of the clinical trial drug is shown in Table 38.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the UGCR changes from the baseline at different time points after administration of the clinical trial drug were 65.72(2.70) mg/mg in the test group (enavogliflozin, 0.3 mg) and 50.10(2.80) mg/mg in the control (dapagliflozin, 10 mg) at week 6, 64.70(2.58) mg/mg in the test group and 45.14(2.66) mg/mg in the control at week 12, 60.97(2.13) mg/mg in the test group and 42.72(2.15) mg/mg in the test group at week 18, and 60.48(2.12) mg/mg in the test group and 44.94(2.18) mg/mg in the control at week 24. The between-group differences (LS mean difference/test group-control (95% CI)) were 15.62(8.25, 23.00) mg/mg at week 6, 19.57(12.55, 26.58) mg/mg at week 12, 18.25(12.50, 24.00) mg/mg at week 18, and 15.54(9.77, 21.31) mg/mg at week 24, showing statistically significant between-group differences at all time points (p<0.0001).

The UGCR changes (SD) over time were 63.13(24.93) mg/mg in the test group and 47.54(26.73) mg/mg in the control at week 6, 63.04(24.63) mg/mg in the test group and 43.59(23.33) mg/mg in the control at week 12, 59.41(19.10) mg/mg in the test group and 41.06(21.79) mg/mg in the control at week 18, and 58.90(20.59) mg/mg in the test group and 43.01(20.50) mg/mg in the control at week 24, showing statistically significant between-group differences at all time points (p<0.0001).

**[Table 38]**

| **Change from baseline in renal function parameters at weeks 6, 12, 18 and 24 (per-protocol set) [UGCR]** | | |
|---|---|---|
| | **Enavogliflozin 0.3 mg (N=95)** | **Dapagliflozin 10 mg (N=90)** |
| **Baseline** | | |
| n | 95 | 89 |
| Mean (SD) | 1.54(8.70) | 1.83(7.49) |
| Median | 0.14 | 0.15 |
| Min, Max | 0.02, 82.79 | 0.01, 66.03 |

| **Week 6** | | |
|---|---|---|
| n | 95 | 87 |
| Mean (SD) | 64.66(25.06) | 48.64(27.88) |
| Median | 58.45 | 43.08 |
| Min, Max | 23.06, 152.75 | 0.04, 177.89 |

| **Change from baseline at week 6** | | |
|---|---|---|
| n | 95 | 87 |
| Mean (SD) | 63.13(24.93) | 47.54(26.73) |
| Median | 57.91 | 42.30 |
| Min, Max | 10.38, 149.85 | -0.06, 164.09 |
| P-value [1] | <0.0001 (w) | <0.0001 (w) |

| **ANCOVA result** | | |
|---|---|---|
| LS mean (SE) | 65.72(2.70) | 50.10(2.80) |
| LS mean difference | 15.62 | |
| 95% confidence interval for difference | [8.25, 23.00] | |
| P-value [2] | <0.0001 | |

| **Week 12** | | |
|---|---|---|
| n | 94 | 88 |
| Mean (SD) | 64.53(24.11) | 45.57(27.08) |
| Median | 52.30 | 40.89 |
| Min, Max | 4.01, 131.80 | 0.03, 186.83 |

| **Change from baseline at week 12** | | |
|---|---|---|
| n | 94 | 87 |
| Mean (SD) | 63.04(24.63) | 43.59(23.33) |
| Median | 58.91 | 40.62 |
| Min, Max | 3.37, 131.66 | -1.61, 120.80 |
| P-value [1] | <0.0001 (t) | <0.0001 (t) |

| **ANCOVA result** | | |
|---|---|---|
| LS mean (SE) | 64.70(2.58) | 45.14(2.66) |
| LS mean difference | 19.57 | |
| 95% confidence interval for difference | [12.55, 26.58] | |
| P-value [2] | <0.0001 | |

| **Week 18** | | |
|---|---|---|
| n | 90 | 87 |
| Mean (SD) | 60.98(18.79) | 43.00(20.49) |
| Median | 58.42 | 40.86 |
| Min, Max | 20.09, 105.73 | 0.02, 101.64 |

| **Change from baseline at week 18** | | |
|---|---|---|
| n | 90 | 86 |
| Mean (SD) | 59.41(19.10) | 41.06(21.79) |
| Median | 57.54 | 40.72 |
| Min, Max | 13.05, 105.51 | -38.93, 95.28 |
| P-value [1] | <0.0001 (t) | <0.0001 (t) |

| **ANCOVA result** | | |
|---|---|---|
| LS mean (SE) | 60.97(2.13) | 42.72(2.15) |
| LS mean difference | 18.25 | |
| 95% confidence interval for difference | [12.50, 24.00] | |
| P-value [2] | <0.0001 | |

| **Week 24** | | |
|---|---|---|
| n | 95 | 90 |
| Mean (SD) | 50.43(20.76) | 45.27(20.91) |
| Median | 57.88 | 43.81 |
| Min, Max | 23.67, 118.49 | 0.05, 103.17 |

| **Change from baseline at week 24** | | |
|---|---|---|
| n | 95 | 89 |
| Mean (SD) | 58.90(20.59) | 43.01(20.50) |
| Median | 56.43 | 42.01 |
| Min, Max | 14.79, 118.34 | -0.05, 103.03 |
| P-value [1] | <0.0001 (t) | <0.0001 (t) |

| **ANCOVA result** | | |
|---|---|---|
| LS mean (SE) | 60.48(2.12) | 44.94(2.18) |
| LS mean difference | 15.54 | |
| 95% confidence interval for difference | [9.77, 21.31] | |
| P-value [2] | <0.0001 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

**7) Changes in obesity-related indicators (adiponectin, leptin) at each time point of weeks 6, 12, 18, and 24 after administration of clinical trial drug compared to Visit 2 (randomization)**

### ① Adiponectin

The result of summarizing adiponectin changes from baseline in PPS at weeks 6, 12, 18, and 24 after administration of the clinical trial drug is shown in Table 39.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the adiponectin changes from the baseline at different time points after administration of the clinical trial drug were 0.92(0.28) ng/mL in the test group (enavogliflozin, 0.3 mg) and 0.80(0.29) ng/mL in the control (dapagliflozin, 10 mg) at week 6, 0.84(0.27) ng/mL in the test group and 1.02(0.27) ng/mL in the control at week 12, 1.33(0.28) ng/mL in the test group and 1.29(0.29) ng/mL in the control at week 18, and 1.67(0.31) ng/mL in the test group and 1.69(0.32) ng/mL in the control at week 24. The between-group differences (LS mean difference/test group-control (95% CI)) were 0.13(-0.65, 0.90) ng/mL at week 6, -0.19(-0.91, 0.54) ng/mL at week 12, 0.04(-0.74, 0.81) ng/mL at week 18, and -0.02(-0.87, 0.84) ng/mL at week 24, showing no statistically significant between-group differences at any time point (p=0.7474 at week 6, p=0.6107 at week 12, p=0.9266 at week 18, and p=0.9688 at week 24).

The adiponectin changes (SD) over time were 0.84(3.17) ng/mL in the test group and 0.77(2.20) ng/mL in the control at week 6, 0.90(2.61) ng/mL in the test group and 1.08(2.33) ng/mL in the control at week 12, 1.26(2.49) ng/mL in the test group and 1.28(2.77) ng/mL in the control at week 18, and 1.72(3.03) ng/mL in the test group and 1.72(2.78) ng/mL in the control at week 24, showing statistically significant between-group differences at all time points and in all administration groups (p=0.0010 in the test group and p=0.0007 in the control at week 6, and p<0.0001 in the test group and the control at weeks 12, 18, and 24).

**[Table 39]**

| **Change from baseline in obesity parameters at weeks 6, 12, 18 and 24 (per-protocol set) [adiponectin]** | | |
|---|---|---|
| | **Enavogliflozin 0.3 mg (N=95)** | **Dapagliflozin 10 mg (N=90)** |
| **Baseline** | | |
| n | 95 | 89 |
| Mean (SD) | 5.96(4.84) | 5.60(4.23) |
| Median | 5.99 | 5.94 |
| Min, Max | 2.14, 42.19 | 1.06, 27.09 |

| **Week 6** | | |
|---|---|---|
| n | 95 | 89 |
| Mean (SD) | 7.80(4.79) | 7.40(4.41) |
| Median | 6.87 | 6.36 |
| Min, Max | 2.32, 38.80 | 1.43, 26.28 |

| **Change from baseline at week 6** | | |
|---|---|---|
| n | 95 | 88 |
| Mean (SD) | 0.84(3.17) | 0.77(2.20) |
| Median | 0.58 | 0.51 |
| Min, Max | -11.28, 12.35 | -4.43, 10.81 |
| P-value [1] | 0.0010 (w) | 0.0007 (w) |

| **ANCOVA result** | | |
|---|---|---|
| LS mean (SE) | 0.92(0.28) | 0.80(0.29) |
| LS mean difference | 0.13 | |
| 95% confidence interval for difference | [-0.65, 0.90] | |
| P-value [2] | 0.7474 | |

| **Week 12** | | |
|---|---|---|
| n | 94 | 88 |
| Mean (SD) | 7.87(5.03) | 7.76(5.01) |
| Median | 6.91 | 5.53 |
| Min, Max | 2.38, 45.25 | 1.45, 33.98 |

| **Change from baseline at week 12** | | |
|---|---|---|
| n | 94 | 87 |
| Mean (SD) | 0.90(2.61) | 1.08(2.33) |
| Median | 0.68 | 0.83 |
| Min, Max | -10.52, 8.35 | -3.53, 10.38 |
| P-value [1] | <0.0001 (w) | <0.0001 (w) |

| **ANCOVA result** | | |
|---|---|---|
| LS mean (SE) | 0.84(0.27) | 1.02(0.27) |
| LS mean difference | -0.19 | |
| 95% confidence interval for difference | [-0.91, 0.54] | |
| P-value [2] | 0.6107 | |

| **Week 18** | | |
|---|---|---|
| n | 90 | 87 |
| Mean (SD) | 8.30(4.95) | 7.98(4.70) |
| Median | 7.33 | 7.08 |
| Min, Max | 2.70, 40.68 | 1.33, 27.32 |

| **Change from baseline at week 18** | | |
|---|---|---|
| n | 90 | 86 |
| Mean (SD) | 1.26(2.49) | 1.28(2.77) |
| Median | 1.14 | 0.73 |
| Min, Max | -6.25, 8.37 | -4.73, 17.48 |
| P-value [1] | <0.0001 (w) | <0.0001 (w) |

| **ANCOVA result** | | |
|---|---|---|
| LS mean (SE) | 1.33(0.28) | 1.29(0.29) |
| LS mean difference | 0.04 | |
| 95% confidence interval for difference | [-0.74, 0.81] | |
| P-value [2] | 0.9266 | |

| **Week 24** | | |
|---|---|---|
| n | 95 | 90 |
| Mean (SD) | 8.68(5.52) | 8.35(5.52) |
| Median | 7.64 | 7.19 |
| Min, Max | 2.83, 45.18 | 1.76, 39.40 |

| **Change from baseline at week 24** | | |
|---|---|---|
| n | 95 | 89 |
| Mean (SD) | 1.72(3.03) | 1.72(2.78) |
| Median | 1.21 | 1.06 |
| Min, Max | -7.29, 11.46 | -5.08, 12.31 |
| P-value [1] | <0.0001 (w) | <0.0001 (w) |

| **ANCOVA result** | | |
|---|---|---|
| LS mean (SE) | 1.67(0.31) | 1.69(0.32) |
| LS mean difference | -0.02 | |
| 95% confidence interval for difference | [-0.87, 0.84] | |
| P-value [2] | 0.9688 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

### ② Leptin

The result of summarizing leptin changes from baseline in PPS at weeks 6, 12, 18, and 24 after administration of the clinical trial drug is shown in Table 40.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the leptin changes from the baseline at different time points after administration of the clinical trial drug were -2.43(0.46) ng/mL in the test group (enavogliflozin, 0.3 mg) and -1.62(0.47) ng/mL in the control (dapagliflozin, 10 mg) at week 6, -2.64(0.49) ng/mL in the test group and -2.70(0.51) ng/mL in the control at week 12, -2.49(0.52) ng/mL in the test group and -1.23(0.53) ng/mL in the control at week 18, and -2.40(0.51) ng/mL in the test group and -1.28(0.53) ng/mL in the control at week 24. The between-group differences (LS mean difference/test group-control (95% CI)) were -0.81(-2.05, 0.44) ng/mL at week 6, 0.07(-1.28, 1.41) ng/mL at week 12, -1.26(-2.68, 0.16) ng/mL at week 18, and -1.12(-2.51, 0.27) ng/mL at week 24, showing no statistically significant between-group differences at any time point (p=0.2035 at week 6, p=0.9233 at week 12, p=0.0825 at week 18, and p=0.1151 at week 24).

The leptin changes (SD) over time were -2.54(4.67) ng/mL in the test group and -1.61(4.75) ng/mL in the control at week 6, -2.73(4.67) ng/mL in the test group and -2.70(5.87) ng/mL in the control at week 12, -2.50(4.99) ng/mL in the test group and -1.20(5.47) ng/mL in the control at week 18, and -2.53(4.17) ng/mL in the test group and -1.31(6.08) ng/mL in the control at week 24, showing statistically significant between-group differences at all time points and in all administration groups (p<0.0001).

**[Table 40]**

| **Change from baseline in obesity parameters at weeks 6, 12, 18 and 24 (per-protocol set) [leptin]** | | |
|---|---|---|
| | Enavogliflozin 0.3 mg (N=95) | Dapagliflozin 10 mg (N=90) |
| Baseline | | |
| N | 95 | 89 |
| Mean (SD) | 12.21(11.08) | 11.76(10.51) |
| Median | 8.00 | 8.32 |
| Min, Max | 0.74, 46.07 | 0.32, 62.65 |

| Week 6 | | |
|---|---|---|
| N | 95 | 89 |
| Mean (SD) | 9.66(8.89) | 9.85(10.35) |
| Median | 5.99 | 5.51 |
| Min, Max | 0.41, 34.04 | 0.54, 55.17 |

| Change from baseline at week 6 | | |
|---|---|---|
| N | 95 | 88 |
| Mean (SD) | -2.54(4.67) | -1.61(4.75) |
| Median | -1.36 | -1.46 |
| Min, Max | -18.64, 16.55 | -20.22, 16.61 |
| P-value [1] | <0.0001 (w) | <0.0001 (w) |

| ANCOVA result | | |
|---|---|---|
| LS mean (SE) | -2.43(0.46) | -1.62(0.47) |
| LS mean difference | -0.81 | |
| 95% confidence interval for difference | [-2.05, 0.44] | |
| P-value [2] | 0.2035 | |

| Week 12 | | |
|---|---|---|
| N | 94 | 88 |
| Mean (SD) | 9.56(8.48) | 9.07(10.18) |
| Median | 5.60 | 5.05 |
| Min, Max | 0.53, 41.85 | 0.35, 65.39 |

| Change from baseline at week 12 | | |
|---|---|---|
| N | 94 | 87 |
| Mean (SD) | -2.73(4.67) | -2.70(5.87) |
| Median | -1.45 | -1.94 |
| Min, Max | -23.19, 6.88 | -27.30, 23.09 |
| P-value [1] | <0.0001 (w) | <0.0001 (w) |

| ANCOVA result | | |
|---|---|---|
| LS mean (SE) | -2.64(0.49) | -2.70(0.51) |
| LS mean difference | 0.07 | |
| 95% confidence interval for difference | [-1.28, 1.41] | |
| P-value [2] | 0.9233 | |

| Week 18 | | |
|---|---|---|
| N | 90 | 87 |
| Mean (SD) | 0.71(8.27) | 10.72(11.35) |
| Median | 7.22 | 6.16 |
| Min, Max | 0.48, 38.62 | 0.20, 65.25 |

| Change from baseline at week 18 | | |
|---|---|---|
| N | 90 | 86 |
| Mean (SD) | -2.50(4.99) | -1.20(5.47) |
| Median | -1.32 | -1.37 |
| Min, Max | -20.85, 7.76 | -18.32, 22.95 |
| P-value [1] | <0.0001 (w) | <0.0001 (w) |

| ANCOVA result | | |
|---|---|---|
| LS mean (SE) | -2.49(0.52) | -1.23(0.53) |
| LS mean difference | -1.26 | |
| 95% confidence interval for difference | [-2.68, 0.16] | |
| P-value [2] | 0.0825 | |

| Week 24 | | |
|---|---|---|
| N | 95 | 90 |
| Mean (SD) | 9.68(8.31) | 10.37(11.37) |
| Median | 7.45 | 6.69 |
| Min, Max | 0.70, 36.17 | 0.54, 75.67 |

| Change from baseline at week 24 | | |
|---|---|---|
| N | 95 | 89 |
| Mean (SD) | -2.53(4.17) | -1.31(6.08) |
| Median | -1.77 | -1.17 |
| Min, Max | -16.62, 7.69 | -17.60, 33.37 |
| P-value [1] | <0.0001 (w) | <0.0001 (w) |

| ANCOVA result | | |
|---|---|---|
| LS mean (SE) | -2.40(0.51) | -1.28(0.53) |
| LS mean difference | -1.12 | |
| 95% confidence interval for difference | [-2.51, 0.27] | |
| P-value [2] | 0.1151 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen [monotherapy or combination] and HbAlc level at visit 1 [screening][< 8% or ≥ 8%])) as covariates). Note: Baseline is Visit 2 (randomization). Data source: Listing 16.2.3:1, Listing 16.2.6:1. | | |

### 3. Conclusion

As a result of the efficacy evaluation, the upper limit of the 95% confidence interval for the between-group difference (LS mean difference/test group-control) in the change in HbAlc from baseline, which is the primary efficacy endpoint, at week 24 after administration of the clinical trial drug was 0.12%p, which was smaller than the non-inferiority margin of 0.35, proving that the test group was non-inferior to the control group. In addition, most secondary and exploratory endpoints, including FPG, showed similar results between the test group and the control.

In the safety evaluation results, there were no statistically significant differences in the incidence of adverse events or serious adverse events between the test group and the control, and there was a significant between-group difference in the incidence of serious adverse drug reactions, but the incidence was high in the control (1 case for the test group, and 7 cases for the control). In addition, the incidence of serious adverse events and adverse events requiring careful monitoring (hypoglycemia, urinary tract infection, genital infection, polyuria, and urinary urgency) tended to be lower in the test group than in the control, although there was no significant difference in incidence. All adverse drug reactions that occurred were previously reported adverse events in SGLT2 inhibitor-based drugs. However, in this clinical trial, one adverse event (PT name: prostate cancer) occurred in the control that led to discontinuation of the clinical trial drug, but it was confirmed that this was not related to the clinical trial drug. In addition, there were no serious adverse drug reactions, adverse drug reactions leading to discontinuation of clinical trial drugs, adverse events leading to death, or adverse drug reactions. There were no clinically significant findings in other safety evaluation items, except for one adverse event related to vital signs (PT name: hypertension).

Based on the above, the efficacy and safety of enavogliflozin were confirmed in combination therapy with metformin in patients with type 2 diabetes whose glycemic control was insufficient with metformin, demonstrating that enavogliflozin is an effective therapeutic agent for diabetes when co-administered with metformin.

### Example 3: Combination therapy with enavogliflozin, metformin, and gemigliptin in patients with type 2 diabetes

### 1. Research process

1) When metformin (≥1,000 mg/day) was administered at a constant dose for at least 8 weeks prior to Pre-visit (pre-screening)
   : Metformin (≥1,000 mg/day) and gemigliptin (50 mg/day) were co-administered during the 8-week stabilization period.
2) When metformin (<1,000 mg/day) and a hypoglycemic agent for oral administration were co-administered at constant doses for at least 8 weeks prior to Pre-visit (pre-screening)
   : Metformin was increased to at least 1,000 mg/day for a maximum 4-week dose increase period, followed by combination therapy with metformin (≥1,000 mg/day) and gemigliptin (50 mg/day) for an 8-week stabilization period, and other than these, the hypoglycemic agent for oral administration had a washout period.
3) When metformin (≥1,000 mg/day) and a hypoglycemic agent for oral administration (except gemigliptin) were co-administered at constant doses for at least 8 weeks prior to pre-visit (prescreening)
   : Metformin (≥1,000 mg/day) and gemigliptin (50 mg/day) were co-administered during the 8-week stabilization period, and another hypoglycemic agent for oral administration that was co-administered with metformin had a washout period.
4) When metformin (≥1,000 mg/day) and gemigliptin (50 mg/day) were co-administered at constant doses for at least 8 weeks prior to pre-visit (prescreening)
   : Without a separate stabilization period, Pre-visit (pre-screening) was Visit 1 (screening).

In each case, patients with type 2 diabetes who showed inadequate glycemic control at visit 1 (screening) after screening participated in this clinical trial.

### [Run-in period]

Subjects who met the inclusion/exclusion criteria continued to receive metformin and gemigliptin while receiving a placebo of the clinical trial drug in a single-blind (subject-blinded) state for a 2-week run-in period. Subjects who met the final inclusion/exclusion criteria and had a 70 to 130% compliance to each of placebo, metformin, and gemigliptin during the run-in period were randomized at Visit 2 (randomization).

### [Treatment period]

Subjects were centrally randomized to one of the test group (enavogliflozin, 0.3 mg) and the control (dapagliflozin, 10 mg) at a ratio of 1:1 at Visit 2. Here, the randomized subjects were stratified according to the administration therapy (co-administration with two or less agents (one or two agents) or three or more agents) of a conventional hypoglycemic agent (before washout of another hypoglycemic agent) within 24 weeks prior to obtaining written informed consent and by an HbAlc value (< 8% or ≥ 8%) at Visit 1 (screening) measured in the central laboratory.

The randomized subjects were administered two tablets of the clinical trial drug corresponding to each administration group once a day, and then visited the institution at weeks 6, 12, 18, and 24 during the 24-week administration period to conduct efficacy and safety assessments. In addition, during the clinical trial period, they were instructed to exercise regularly and control their diet without changing their daily life patterns.

### [Inclusion criteria]

### Inclusion criteria in Pre-visit (pre-screening) or Visit 1 (screening)

1. Patients with type 2 diabetes aged 19 to 80 as of the date of obtaining written consent
2. Those whose HbAlc that has been confirmed at Pre-visit (pre-screening) is as follows:
   1) When metformin (≥1,000 mg/day) is administered at a constant dose for at least 8 weeks prior to Pre-visit (pre-screening): 7.5% ≤ HbAlc ≤ 11%
   2) When metformin (<1,000 mg/day) and a hypoglycemic agent for oral administration are co-administered at constant doses for at least 8 weeks prior to Pre-visit (pre-screening): 7.5% ≤ HbAlc ≤ 11%
   3) When metformin (≥1,000 mg/day) and hypoglycemic agent for oral administration (except gemigliptin) are co-administered at constant doses for at least 8 weeks prior to Pre-visit (pre-screening): 7% ≤ HbAlc ≤ 11%
   4) When metformin (≥1,000 mg/day) and gemigliptin (50 mg/day) are co-administered at constant doses for at least 8 weeks prior to Pre-visit (pre-screening): 7% ≤ HbAlc ≤ 11%
3. HbAlc is 7% ≤ HbA1c* ≤ 11% at Visit 1 (screening)
   (*Results from the test institution or central laboratory are available. The final decision was made based on the results from the central laboratory, but when the results from the test institution were appropriate before the results from the central laboratory were confirmed for the run-in, the run-in was allowed to proceed.)
4. Those with FPG < 270 mg/dL at Pre-visit (pre-screening) and Visit 1 (screening)
5. Those with BMI of 20 to 45 kg/m² at Pre-visit (pre-screening) and Visit 1 (screening)
6. Those who voluntarily decided to participate and gave written consent after hearing an explanation of the purpose, method, and effects of this clinical trial

### Inclusion criteria at Visit 2 (randomization)

1. Those with 7% ≤ HbAlc ≤ 10.5% as tested at the central laboratory at Visit 1 (screening)
2. Those with placebo and metformin compliances of 70 to 130% during the run-in period confirmed at Visit 2 (randomization)

### [Exclusion criteria]

### Exclusion criteria in Pre-visit (pre-screening) or Visit 1 (screening)

1. Those who have a medical history of or hypersensitivity to drugs and components including the clinical trial drug in this test or any drug in the same class (e.g., a history of hypersensitivity to biguanide, DPP-4 inhibitor and SGLT2 inhibitor-based drugs)
2. Those with the following medical history or surgical/therapeutic history
   History of medically significant kidney disease: renal vascular occlusive disease, nephrectomy, kidney transplant, etc.
   History of major gastrointestinal surgery: total gastrectomy, total colectomy, small bowel resection, gastroenterostomy, gastrointestinal bypass, etc.
   History of acute pancreatitis or pancreatic surgery
   History of bariatric surgery within the past 2 years
   Diabetic ketoacidosis, diabetic coma or precoma within the past year
   Recurrent urinary tract infections or genital infections requiring treatment within the past year
   Alcohol or drug addiction within the past year
   Occurrence of acute coronary syndrome, unstable angina, myocardial infarction requiring hospitalization, stroke, transient ischemic attack, or arrhythmia within the past 24 weeks
   (However, those who had the disease more than 24 weeks ago and were currently cured or in a stable condition were allowed to enroll)
   Those who have had a history of major surgery resulting in electrolyte imbalance within the past 12 weeks, or who are scheduled for major surgery within 12 weeks after the end of the clinical trial
   Those with lactic acidosis or a history of lactic acidosis
   Those with history of Stevens-Johnson syndrome
3. Those with the following diseases or symptoms
   Types of diabetes other than type 2 diabetes (type 1 diabetes, secondary diabetes, or congenital renal diabetes)
   Symptoms of urinary incontinence, anuria, oliguria, and urinary retention that are not controlled by medication due to stress incontinence, neurogenic bladder, and benign prostatic hyperplasia
   Severe diabetic complications (proliferative diabetic retinopathy, kidney disease (nephropathy) of stage 4 or higher, or severe diabetic neuropathy)
   Chronic disease requiring continued use (oral, injected, or inhaled) of diuretics, systemic steroids, or immunosuppressants
   Pituitary insufficiency or adrenal insufficiency
   Severe infection (e.g., severe infection requiring continued use of antibiotics or immunotherapy), clinically significant major trauma
   Unstable mental illness whose symptoms are not controlled by medication
   Severe gastrointestinal disease: active ulcers, gastrointestinal/rectal bleeding, active inflammatory bowel syndrome, patients with biliary obstruction, active gastritis not controlled by medication, etc.
   Uncontrolled high blood pressure (SBP > 180 mmHg or DBP > 110 mmHg)
   Patients with moderate to severe nephropathy (eGFR < 60 mL/min/1.73 m²)
   Patients with severe liver impairment who fall under one of the following categories:
      - AST or ALT > three times the upper limit of the normal level
      - Total bilirubin > two times the upper limit of the normal level
      - Hepatitis or liver failure
   Patients with severe hypertriglyceridemia (triglycerides > 500 mg/dL)
   Acquired immunodeficiency syndrome
   Severe heart failure (NYHA class II~IV)
   Patients with pulmonary infarction, severe pulmonary dysfunction, and other conditions prone to hypoxemia
   Those who require treatment for dehydration due to persistent diarrhea or vomiting, or are at risk of fluid volume depletion
   Those with genetic problems such as galactose intolerance, Lapp lactase deficiency, or glucose-galactose malabsorption
4. Patients who have used other clinical trial drugs/medical devices within the past 4 weeks (however, enrollment was permitted in the case of studies that, in the opinion of the investigator, were unlikely to affect the validity and safety of the study, such as observational or retrospective studies)
5. Patients with a history of malignant tumor within the past 5 years
   - However, patients with appropriately controlled basal cell carcinoma, squamous cell carcinoma, or cervical intraepithelial carcinoma were allowed participate.
   - However, participation was possible when there had been no recurrence for more than 5 years after a complete cure (the complete removal of the tumor through surgery or completion of chemotherapy, etc.)
6. Pregnant or lactating women or those who do not agree to use appropriate contraception during the clinical trial period
   Appropriate contraception for a subject or his/her partner: should agree to one of the following:
   ① one of sterilization surgery (vasectomy, etc.), intrauterine device (copper loop, hormone-containing intrauterine system),
   ② combination of non-oral hormonal contraceptives or spermicides with a barrier method, or
   ③ combination of a cervical cap or diaphragm together with a male condom
7. Those who are judged by the investigator to be ineligible for this clinical trial

### Exclusion criteria at Visit 1 (screening)

1. Those who have a history of taking the following drugs or are expected to require continuous administration during the clinical trial period
   Systemic steroids within the past 2 weeks (more than 30 mg of prednisolone per day)
   Those who have had a dose change of any drug for thyroid dysfunction within the past 6 weeks (combination therapy was allowed when dosing had been stable prior to enrollment in the clinical trial; dose reductions were permitted when stable)
   Therapeutic agents for diabetes except metformin within the past 8 weeks
   Weight loss drugs taken within the past 12 weeks
   Those expected to require continuous administration of hypoglycemic agents for oral administration, excluding clinical trial drugs, concomitant drugs (metformin and gemigliptin), and rescue drugs during the clinical trial period

### Primary efficacy endpoint

1) Change in HbAlc at week 24 after administration of clinical trial drug compared to Visit 2 (randomization)

### Secondary efficacy endpoints

1) Change in HbAlc at each time point of weeks 6, 12, and 18 after administration of clinical trial drug compared to Visit 2 (randomization)
2) Change in FPG at each time point of weeks 6, 12, 18, and 24 after administration of clinical trial drug compared to Visit 2 (randomization)
3) Proportion of subjects achieving HbAlc < 7% at each time point of weeks 6, 12, 18, and 24 after administration of clinical trial drug
4) Proportion of subjects achieving HbAlc < 6.5% at each time point of weeks 6, 12, 18, and 24 after administration of clinical trial drug
5) Proportion of subjects achieving therapeutic response (HbAlc change (HbAlc at Visit 2 - HbAlc at each evaluation time point) > 0.5% or HbAlc < 7% at each evaluation time point compared to Visit 2 (randomization)) at each time point of weeks 6, 12, 18, and 24 after administration of clinical trial drug
6) Proportion of subjects achieving therapeutic response (HbAlc change (HbAlc at Visit 2 - HbAlc at each evaluation time point) > 0.7% or HbAlc < 7% at each evaluation time point compared to Visit 2 (randomization)) at each time point of weeks 6, 12, 18, and 24 after administration of clinical trial drug
7) Proportion of subjects achieving therapeutic response (HbAlc change (HbAlc at Visit 2 - HbAlc at each evaluation time point) > 1% or HbAlc < 7% at each evaluation time point compared to Visit 2 (randomization)) at each time point of weeks 6, 12, 18, and 24 after administration of clinical trial drug

### Exploratory endpoints

1) Change in fasting c-peptide at each time point of weeks 6, 12, 18, and 24 after administration of clinical trial drug compared to Visit 2 (randomization)
2) Change in body weight at each time point of weeks 6, 12, 18, and 24 after administration of clinical trial drug compared to Visit 2 (randomization)
3) Change in fasting lipid concentration (total cholesterol, LDL-C, HDL-C, and triglycerides) at each time point of weeks 6, 12, 18, and 24 after administration of clinical trial drug compared to Visit 2 (randomization)
4) Changes in systolic or diastolic blood pressure at each time point of weeks 6, 12, 18, and 24 after administration of clinical trial drug compared to Visit 2 (randomization)
5) Changes in β-cell function and insulin resistance measured by HOMA-β and HOMA-IR at each time point of weeks 6, 12, 18, and 24 after administration of clinical trial drug compared to Visit 2 (randomization)
6) Changes in renal function-related indicators (UACR, UGCR) measured by UACR at each time point of weeks 6, 12, 18, and 24 after administration of clinical trial drug compared to Visit 2 (randomization)
7) Changes in obesity-related indicators (adiponectin, leptin) at each time point of weeks 6, 12, 18, and 24 after administration of clinical trial drug compared to Visit 2 (randomization)
8) Proportion of subjects who received rescue drug at each time point of weeks 6, 12,18, and 24 after administration of the clinical trial drug

### 2. Experimental results

### Primary efficacy endpoint

### 1) Change in HbAlc at week 24 after administration of clinical trial drug compared to Visit 2 (randomization)

The result of summarizing the change in HbAlc in PPS at week 24 after administration of the clinical trial drug, which is the primary efficacy endpoint in this clinical trial, is shown in Table 41.

As a result of analysis of covariance (ANCOVA) on PPS with the baseline value and stratification factors (the conventional regimen (co-administration with two or more agents (one or two agents) or three or more agents) for administering a hypoglycemic agent (before the washout period of another hypoglycemic agent) within 24 weeks prior to obtaining written consent, Visit 1 (screening) HbAlc level (< 8% or ≥ 8%)) as covariates, the least square means (LS means (SE)) for the HbAlc change from the baseline at week 24 after administration of the clinical trial drug were -0.92(0.05)%p in the test group (enavogliflozin, 0.3 mg) and -0.86(0.05)%p in the control (dapagliflozin, 10 mg), and the between-group difference (LS mean difference (95% CI)) was -0.06(-0.19, 0.06)%p. The upper limit of the 95% confidence interval for [test group-control] was 0.12%p, which was less than the non-inferiority margin of 0.35, demonstrating that the test group was not inferior to the control.

The HbAlc changes (SD) between administration groups were -0.93(0.70)%p in the test group and -0.89(0.65)%p in the control, and there were statistically significant differences in both groups (p<0.0001).

**[Table 41]**

| **Change from baseline in HbA1c at week 24 (per-protocol set)** | | |
|---|---|---|
| | **Enavogliflozin 0.3 mg (N=119)** | **Dapagliflozin 10 mg (N=123)** |
| **Baseline** | | |
| n | 119 | 123 |
| Mean (SD) | 7.79(0.77) | 7.83(0.81) |
| Median | 7.60 | 7.64 |
| Min, Max | 6.81, 10.54 | 6.73, 10.30 |

| **Week 24** | | |
|---|---|---|
| n | 119 | 123 |
| Mean (SD) | 6.86(0.54) | 6.94(0.64) |
| Median | 6.76 | 6.81 |
| Min, Max | 5.44, 8.62 | 5.78, 9.87 |

| **Change from baseline at week 24** | | |
|---|---|---|
| n | 119 | 123 |
| Mean (SD) | -0.93(0.70) | -0.89(0.65) |
| Median | -0.83 | -0.86 |
| Min, Max | -3.64, 0.95 | -3.05, 1.59 |
| P-value [1] | <0.0001 (w) | <0.0001 (w) |

| **ANCOVA result [2]** | | |
|---|---|---|
| LS mean (SE) | -0.92(0.05) | -0.86(0.05) |
| LS mean difference | -0.06 | |
| 95% confidence interval for difference | [-0.19, 0.06] | |
| Non-inferiority (UCI < 0.35%) | Yes | |

| | | |
|---|---|---|
| SD = standard deviation, Min = minimum, Max = maximum, ANCOVA = analysis of covariance, LS Mean = least squares mean, SE = standard error, UCI = upper limit confidence interval. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbAlc level at visit 1 [screening][< 8% or ≥ 8%]) as covariates). Note: Baseline is Visit 2 (randomization). Data source: Listing 16.2.3:1, Listing 16.2.6:1. | | |

### Secondary efficacy endpoints

### 1) Change in HbA1c at each of weeks 6, 12, and 18 after administration of clinical trial drug compared to Visit 2 (randomization)

The results of the summarizing HbAlc changes from base line in PPS at weeks 6, 12, and 18 after administration of the clinical trial are presented in Table 42.

As a result of analysis of covariance (ANCOVA) on PPS with the baseline value and stratification factors (the conventional regimen (co-administration with two or more agents (one or two agents) or three or more agents) for administering a hypoglycemic agent (before the washout period of another hypoglycemic agent) within 24 weeks prior to obtaining written consent, Visit 1 (screening) HbAlc level (< 8% or ≥ 8%)) as covariates, the least square means (LS means (SE)) for the HbAlc changes from the baseline at different time points after administration of the clinical trial drug were -0.73(0.03)%p in the test group (enavogliflozin, 0.3 mg) and -0.70(0.03)%p in the control (dapagliflozin, 10 mg) at week 6, -0.87(0.04)%p in the test group and -0.83(0.04)%p in the control at week 12, and -0.83(0.05)%p in the test group and -0.75(0.04)%p in the control at week 18. The between-group differences (LS mean differences (95% CI)) were -0.03(-0.11, 0.06)%p at week 6, -0.04(-0.15, 0.06)%p at week 12, and -0.07(-0.19, 0.04)%p at week 18, showing no statistically significant between-group differences at any time point (p=0.5606 at week 6, p=0.4085 at week 12, and p=0.1951 at week 18).

The HbAlc changes (SD) over time were -0.74(0.46)%p in the test group and -0.72(0.41)%p in the control at week 6, -0.89(0.62)%p in the test group and -0.87(0.57)%p in the control at week 12, and -0.84(0.69)%p in the test group and -0.79(0.61)%p in the control at week 18, showing statistically significant between-group differences at all time points and in all administration groups.

**[Table 42]**

| **Change from baseline in HbA1c at weeks 6, 12 and 18 (per-protocol set)** | | |
|---|---|---|
| | **Enavogliflozin 0.3 mg (N=119)** | **Dapagliflozin 10 mg (N=123)** |
| **Baseline** | | |
| n | 119 | 123 |
| Mean (SD) | 7.79(0.77) | 7.83(0.81) |
| Median | 7.60 | 7.64 |
| Min, Max | 6.81, 10.54 | 6.73, 10.30 |

| **Week 6** | | |
|---|---|---|
| n | 116 | 122 |
| Mean (SD) | 7.06(0.58) | 7.10(0.67) |
| Median | 6.94 | 6.96 |
| Min, Max | 6.22,9.38 | 5.90, 9.48 |

| **Change from baseline at week 6** | | |
|---|---|---|
| n | 116 | 122 |
| Mean (SD) | -0.74(0.46) | -0.72(0.41) |
| Median | -0.65 | -0.65 |
| Min, Max | -3.06, -0.06 | -2.15,0.19 |
| P-value [1] | <0.0001 (w) | <0.0001 (w) |

| **ANCOVA result** | | |
|---|---|---|
| LS mean (SE) | -0.73(0.03) | -0.70(0.03) |
| LS mean difference | -0.03 | |
| 95% confidence interval for difference | [-0.11, 0.06] | |
| P-value [2] | 0.5606 | |

| **Week 12** | | |
|---|---|---|
| n | 117 | 123 |
| Mean (SD) | 6.90(0.55) | 6.96(0.60) |
| Median | 6.83 | 6.84 |
| Min, Max | 5.71, 8.86 | 5.93, 9.32 |

| **Change from baseline at week 12** | | |
|---|---|---|
| n | 117 | 123 |
| Mean (SD) | -0.89(0.62) | -0.87(0.57) |
| Median | -0.75 | -0.85 |
| Min, Max | -3.66, 0.25 | -2.88, 0.30 |
| P-value [1] | <0.0001 (w) | <0.0001 (w) |

| **ANCOVA result** | | |
|---|---|---|
| LS mean (SE) | -0.87(0.04) | -0.83(0.04) |
| LS mean difference | -0.04 | |
| 95% confidence interval for difference | [-0.15, 0.06] | |
| P-value [2] | 0.4085 | |

| **Week 18** | | |
|---|---|---|
| n | 117 | 123 |
| Mean (SD) | 6.95(0.53) | 7.04(0.56) |
| Median | 6.87 | 6.97 |
| Min, Max | 5.75, 8.49 | 5.03, 9.51 |

| **Change from baseline at week 18** | | |
|---|---|---|
| n | 117 | 123 |
| Mean (SD) | -0.84(0.69) | -0.79(0.61) |
| Median | -0.72 | -0.72 |
| Min, Max | -3.61,0.79 | -2.97,0.99 |
| P-value [1] | <0.0001 (w) | <0.0001 (w) |

| **ANCOVA result** | | |
|---|---|---|
| LS mean (SE) | -0.83(0.05) | -0.75(0.04) |
| LS mean difference | -0.07 | |
| 95% confidence interval for difference | [-0.19, 0.04] | |
| P-value [2] | 0.1951 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbAlc level at visit 1 [screening][< 8% or ≥ 8%]) as covariates). Note: Baseline is Visit 2 (randomization). Data source: Listing 16.2.3:1, Listing 16.2.6:1. | | |

### 2) FPG change at each time point of weeks 6, 12, 18, and 24 after administration of clinical trial drug compared to Visit 2 (randomization)

The result of summarizing FPG changes from baseline in PPS at weeks 6, 12, 18, and 24 after administration of the clinical trial drug is shown in Table 43.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbAlc level (< 8% or ≥ 8%) at Visit 1 (screening)) as covariates, the least square means (LS means (SE)) for the FPG changes from the baseline at different time points after administration of the clinical trial drug were -25.96(1.42) mg/dL in the test group (enavogliflozin, 0.3 mg) and -22.71(1.39) mg/dL in the control (dapagliflozin, 10 mg) at week 6, -27.17(1.54) mg/dL in the test group and -24.42(1.50) mg/dL in the control at week 12, -27.77(1.63) mg/dL in the test group and -23.55(1.59) mg/dL in the control at week 18, and -27.21(1.81) mg/dL in the test group and -23.72(1.78) mg/dL in the control at week 24. The between-group differences (LS mean difference/test group-control (95% CI)) were -3.24(-6.84, 0.36) mg/dL at week 6, -2.75(-6.64, 1.14) mg/dL at week 12, -4.22(-8.33, -0.11) mg/dL at week 18, and -3.49(-8.08, 1.10) mg/dL at week 24, showing a statistically significant between-group difference at week 18 (p=0.0773 at week 6, p=0.1651 at week 12, p=0.0444 at week 18, and p=0.1354 at week 24).

The FPG changes (SD) over time were -25.72(21.79) mg/dL in the test group and -22.87(23.96) in the control at week 6, -27.47(22.64) mg/dL in the test group and -25.28(27.86) mg/dL in the control at week 12, -28.00(26.03) mg/dL in the test group and -24.80(27.29) mg/dL in the control at week 18, and -26.31(25.46) mg/dL in the test group and -23.70(29.86) mg/dL in the control at week 24, showing statistically significant between-group differences at all time points and in all administration groups.

**[Table 43]**

| **Change from baseline in FPG at weeks 6, 12, 18 and 24 (per-protocol set)** | | |
|---|---|---|
| | **Enavogliflozin 0.3 mg (N=119)** | **Dapagliflozin 10 mg (N=123)** |
| **Baseline** | | |
| n | 119 | 123 |
| Mean (SD) | 139.34(27.95) | 140.52(29.73) |
| Median | 135.00 | 136.00 |
| Min, Max | 87.00, 240.00 | 85.00, 265.00 |

| **Week 6** | | |
|---|---|---|
| n | 116 | 122 |
| Mean (SD) | 114.21(17.63) | 117.64(17.75) |
| Median | 113.00 | 116.50 |
| Min, Max | 65.00, 157.00 | 58.00, 169.00 |

| **Change from baseline at week 6** | | |
|---|---|---|
| n | 116 | 122 |
| Mean (SD) | -25.72(21.79) | -22.87(23.96) |
| Median | -24.50 | -18.50 |
| Min, Max | -87.00, 13.00 | -123.00, 41.00 |
| P-value [1] | <0.0001 (w) | <0.0001 (w) |

| **ANCOVA result** | | |
|---|---|---|
| LS mean (SE) | -25.96(1.42) | -22.71(1.39) |
| LS mean difference | -3.24 | |
| 95% confidence interval for difference | [-6.84, 0.36] | |
| P-value [2] | 0.0773 | |

| **Week 12** | | |
|---|---|---|
| n | 117 | 123 |
| Mean (SD) | 112.29(17.27) | 115.24(17.60) |
| Median | 110.00 | 112.00 |
| Min, Max | 72.00, 154.00 | 70.00, 166.00 |

| **Change from baseline at week 12** | | |
|---|---|---|
| n | 117 | 123 |
| Mean (SD) | -27.47(22.64) | -25.28(27.86) |
| Median | -25.00 | -20.00 |
| Min, Max | -94.00, 15.00 | -181.00, 21.00 |
| P-value [1] | <0.0001 (w) | <0.0001 (w) |

| **ANCOVA result** | | |
|---|---|---|
| LS mean (SE) | -27.17(1.54) | -24.42(1.50) |
| LS mean difference | -2.75 | |
| 95% confidence interval for difference | [-6.64, 1.14] | |
| P-value [2] | 0.1651 | |

| **Week 18** | | |
|---|---|---|
| n | 117 | 123 |
| Mean (SD) | 111.10(15.80) | 115.72(19.61) |
| Median | 109.00 | 114.00 |
| Min, Max | 81.00, 168.00 | 48.00, 175.00 |

| **Change from baseline at week 18** | | |
|---|---|---|
| n | 117 | 123 |
| Mean (SD) | -28.00(26.03) | -24.80(27.29) |
| Median | -24.00 | -19.00 |
| Min, Max | -119.00, 25.00 | -156.00, 23.00 |
| P-value [1] | <0.0001 (w) | <0.0001 (w) |

| **ANCOVA result** | | |
|---|---|---|
| LS mean (SE) | -27.77(1.63) | -23.55(1.59) |
| LS mean difference | -4.22 | |
| 95% confidence interval for difference | [-8.33, -0.11] | |
| P-value [2] | 0.0444 | |

| **Week 24** | | |
|---|---|---|
| n | 119 | 123 |
| Mean (SD) | 113.03(17.63) | 116.82(21.31) |
| Median | 110.00 | 114.00 |
| Min, Max | 60.00, 180.00 | 66.00, 226.00 |

| **Change from baseline at week 24** | | |
|---|---|---|
| n | 119 | 123 |
| Mean (SD) | -26.31(25.46) | -23.70(29.86) |
| Median | -20.00 | -20.00 |
| Min, Max | -102.00, 28.00 | -151.00, 79.00 |
| P-value [1] | <0.0001 (w) | <0.0001 (w) |

| **ANCOVA result** | | |
|---|---|---|
| LS mean (SE) | -27.21(1.81) | -23.72(1.78) |
| LS mean difference | -3.49 | |
| 95% confidence interval for difference | [-8.08, 1.10] | |
| P-value [2] | 0.1354 | |

| | | |
|---|---|---|
| FPG = fasting plasma glucose, SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbAlc level at visit 1 [screening][< 8% or ≥ 8%]) as covariates). Note: Baseline is Visit 2 (randomization). Data source: Listing 16.2.3:1, Listing 16.2.6:1. | | |

### 3) Proportion of subjects achieving HbAlc < 7% at each time point of weeks 6, 12, 18, and 24 after administration of clinical trial drug

The result of summarizing the proportion of subjects achieving HbAlc **<** 7% in PPS at weeks 6, 12, 18, and 24 after administration of the clinical trial drug is shown in Table 44.

As a result of logistic regression on PPS, corrected with stratification factors (the conventional regimen for administering a hypoglycemic agent (mono or combination) within 24 weeks prior to obtaining written consent (before the washout period of another hypoglycemic agent), HbAlc level (< 8% or ≥ 8%) at Visit 1 (screening)), compared to the control (dapagliflozin, 10 mg), the odds ratios (95% CI) for achieving HbAlc **<** 7% in the test group (enavogliflozin, 0.3 mg) at different time points were 1.22(0.66, 2.25) at week 6, 1.20(0.66, 2.19) at week 12, 1.36(0.78, 2.38) at week 18, and 1.17(0.66, 2.09) at week 24, showing no statistically significant between-group differences at any time point (p=0.5326 at week 6, p=0.5560 at week 12, p=0.2743 at week 18, and p=0.5862 week 24).

The proportion of subjects achieving HbAlc **<** 7% over time was 56.03%(65/116) in the test group and 51.64%(63/122) in the control at week 6, 67.52%(79/117) in the test group and 63.41%(78/123) in the control at week 12, 63.25%(74/117) in the test group and 56.10%(69/123) in the control at week 18, and 66.39%(79/119) in the test group and 62.60%(77/123) in the control at week 24.

**[Table 44]**

| **Proportion of subjects achieving HbA1c target of < 7% at weeks 6, 12, 18 and 24 (per-protocol set)** | | |
|---|---|---|
| | **Enavogliflozin 0.3 mg (N=119)** | **Dapagliflozin 10 mg (N=123)** |
| **Proportion of subjects achieving HbAlc target of < 7% at week 6, N** | 116 | 122 |
| Subjects achieving HbAlc target of < 7%, n(%) | 65(56.03) | 63(51.64) |

| **Logistic regression model** | | |
|---|---|---|
| Odds ratio | 1.22 | |
| 95% confidence interval | [0.66, 2.25] | |
| P-value [1] | 0.5326 | |
| **Proportion of subjects achieving HbAlc target of < 7% at week 12, N** | 117 | 123 |
| Subjects achieving HbAlc target of < 7%, n(%) | 79(67.52) | 78(63.41) |

| **Logistic regression model** | | |
|---|---|---|
| Odds ratio | 1.20 | |
| 95% confidence interval | [0.66, 2.19] | |
| P-value [1] | 0.5560 | |
| **Proportion of subjects achieving HbAlc target of < 7% at week 18, N** | 117 | 123 |
| Subjects achieving HbAlc target of < 7%, n(%) | 74(63.25) | 69(56.10) |

| **Logistic regression model** | | |
|---|---|---|
| Odds ratio | 1.36 | |
| 95% confidence interval | [0.78, 2.38] | |
| P-value [1] | 0.2743 | |
| **Proportion of subjects achieving HbAlc target of < 7% at week 24, N** | 119 | 123 |
| Subjects achieving HbAlc target of < 7%, n(%) | 79(66.39) | 77(62.60) |

| **Logistic regression model** | | |
|---|---|---|
| Odds ratio | 1.17 | |
| 95% confidence interval | [0.66, 2.09] | |
| P-value [1] | 0.5862 | |

| | | |
|---|---|---|
| Proportion = The number of subjects with HbAlc less than 7% at week n / the number of subjects with available data at week n in the efficacy analysis set * 100, n = 6, 12, 18, 24. [1] Testing for difference between DWP16001 and dapagliflozin (logistic regression model with treatment group as a factor and stratification factors (regimen ['Doublet and/or less combination' or 'Triplet and/or more combination'] and HbAlc level at visit 1 [screening][< 8% or ≥ 8%]) as covariates). | | |

Data source: Listing 16.2.3:1, Listing 16.2.6:1.

### 4) Proportion of subjects achieving HbAlc < 6.5% at each time point of weeks 6, 12, 18, and 24 after administration of clinical trial drug

The result of summarizing the proportion of subjects achieving HbAlc < 6.5% in PPS at weeks 6, 12, 18, and 24 after administration of the clinical trial drug is shown in Table 45.

As a result of logistic regression on PPS, corrected with stratification factors (the conventional regimen for administering a hypoglycemic agent (mono or combination) within 24 weeks prior to obtaining written consent (before the washout period of another hypoglycemic agent), HbAlc level (< 8% or ≥ 8%) at Visit 1 (screening)), compared to the control (dapagliflozin, 10 mg), the odds ratios (95% CI) for achieving HbAlc < 6.5% in the test group (enavogliflozin, 0.3 mg) at different time points were 0.65(0.30, 1.41) at week 6, 1.44(0.74, 2.80) at week 12, 1.20(0.56, 2.58) at week 18, and 1.27(0.66, 2.46) at week 24, showing no statistically significant between-group differences at any time point (p = 0.2729 at week 6, p = 0.2784 at week 12, p = 0.6372 at week 18, and p = 0.4729 at week 24).

The proportion of subjects achieving HbAlc < 6.5% was 11.21%(13/116) in the test group and 15.57%(19/122) in the control at week 6, 23.08%(27/117) in the test group and 17.07%(21/123) in the control at week 12, 14.53%(17/117) in the test group and 12.20%(15/123) in the control at week 18, and 21.01%(25/119) in the test group and 17.07%(21/123) in the control at week 24.

**[Table 45]**

| **Proportion of subjects achieving HbA1c target of < 6.5% at weeks 6, 12, 18 and 24 (per-protocol set)** | | |
|---|---|---|
| | **Enavogliflozin 0.3 mg(N=119)** | **Dapagliflozin 10 mg(N=123)** |
| **Proportion of subjects achieving HbAlc target of < 6.5%** at **week 6, N** | 116 | 122 |
| Subjects achieving HbAlc target of < 6.5%, n(%) | 13(11.21) | 19(15.57) |

| **Logistic regression model** | | |
|---|---|---|
| Odds ratio | 0.65 | |
| 95% confidence interval | [0.30, 1.41] | |
| P-value [1] | 0.2729 | |
| **Proportion of subjects achieving HbAlc target of < 6.5% at week 12, N** | 117 | 123 |
| Subjects achieving HbAlc target of < 6.5%, n(%) | 27(23.08) | 21(17.07) |

| **Logistic regression model** | | |
|---|---|---|
| Odds ratio | 1.44 | |
| 95% confidence interval | [0.74, 2.80] | |
| P-value [1] | 0.2784 | |
| **Proportion of subjects achieving HbA1c target of < 6.5% at week 18, N** | 117 | 123 |
| Subjects achieving HbAlc target of < 6.5%, n(%) | 17(14.53) | 15(12.20) |

| **Logistic regression model** | | |
|---|---|---|
| Odds ratio | 1.20 | |
| 95% confidence interval | [0.56, 2.58] | |
| P-value [1] | 0.6372 | |
| **Proportion of subjects achieving HbAlc target of < 6.5% at week 24, N** | 119 | 123 |
| Subjects achieving HbAlc target of < 6.5%, n(%) | 25(21.01) | 21(17.07) |

| **Logistic regression model** | | |
|---|---|---|
| Odds ratio | 1.27 | |
| 95% confidence interval | [0.66, 2.46] | |
| P-value [1] | 0.4729 | |

| | | |
|---|---|---|
| Proportion = The number of subjects with HbAlc less than 6.5% at week n / the number of subjects with available data at week n in the efficacy analysis set * 100, n = 6, 12, 18, 24. [1] Testing for difference between DWP16001 and dapagliflozin (logistic regression model with treatment group as a factor and stratification factors (regimen ['Doublet and/or less combination' or 'Triplet and/or more combination'] and HbAlc level at visit 1 [screening][< 8% or ≥8%]) as covariates). | | |

Data source: Listing 16.2.3:1, Listing 16.2.6:1.

### 5) Proportion of subjects achieving therapeutic response (HbAlc change (HbAlc at Visit 2 - HbA1c at each evaluation time point) > 0.5% or HbAlc < 7% at each evaluation time point compared to Visit 2 (randomization)) at each time point of weeks 6, 12, 18 and 24 after administration of clinical trial drug

The result of summarizing the proportion of subjects achieving therapeutic response (HbAlc change (HbAlc at Visit 2 - HbAlc at each evaluation time point) > 0.5% or HbAlc < 7% at each evaluation time point compared to Visit 2 (randomization)) at each time point of weeks 6, 12, 18, and 24 after administration of clinical trial drug is shown in Table 46.

As a result of logistic regression on PPS, corrected with stratification factors (the conventional regimen for administering a hypoglycemic agent (mono or combination) within 24 weeks prior to obtaining written consent (before the washout period of another hypoglycemic agent), HbAlc level (< 8% or ≥ 8%) at Visit 1 (screening)), compared to the control (dapagliflozin, 10 mg), the odds ratios (95% CI) achieving HbAlc < 7% in the test group (enavogliflozin, 0.3 mg) at different time points were 1.52(0.74, 3.13) at week 6, 1.09(0.47, 2.55) at week 12, 1.02(0.52, 2.02) at week 18, and 1.15(0.55, 2.40) at week 24, showing no statistically significant between-group differences at any time point (p=0.2571 at week 6, p=0.8399 at week 12, p=0.9545 at week 18, p=0.7187 at week 24).

The proportion of subjects achieving therapeutic response over time was 87.07(101/116) in the test group and 81.97%(100/122) in the control at week 6, 89.74%(105/117) in the test group and 89.43%(110/123) in the control at week 12, 82.91%(97/117) in the test group and 82.93%(102/123) in the control at week 18, and 86.55%(103/119) in the test group and 85.37%(105/123) in the control at week 24.

**[Table 46]**

| **Proportion of subjects achieving therapeutic glycemic response 1 at weeks 6, 12, 18 and 24 (per-protocol set)** | | |
|---|---|---|
| | **Enavogliflozin 0.3 mg (N=119)** | **Dapagliflozin 10 mg (N=123)** |
| **Proportion of subjects achieving therapeutic glycemic response 1 at week 6, N** | 116 | 122 |
| Subjects achieving therapeutic glycemic response 1, n(%) | 101(87.07) | 100(81.97) |

| **Logistic regression model** | | |
|---|---|---|
| Odds ratio | 1.52 | |
| 95% confidence interval | [0.74, 3.13] | |
| P-value [1] | 0.2571 | |
| **Proportion of subjects achieving therapeutic glycemic response 1 at week 12, N** | 117 | 123 |
| Subjects achieving therapeutic glycemic response 1, n(%) | 105(89.74) | 110(89.43) |

| **Logistic regression model** | | |
|---|---|---|
| Odds ratio | 1.09 | |
| 95% confidence interval | [0.47, 2.55] | |
| P-value [1] | 0.8399 | |
| **Proportion of subjects achieving therapeutic glycemic response 1 at week 18, N** | 117 | 123 |
| Subjects achieving therapeutic glycemic response 1, n(%) | 97(82.91) | 102(82.93) |

| **Logistic regression model** | | |
|---|---|---|
| Odds ratio | 1.02 | |
| 95% confidence interval | [0.52, 2.02] | |
| P-value [1] | 0.9545 | |
| **Proportion of subjects achieving therapeutic glycemic response 1 at week 24, N** | 119 | 123 |
| Subjects achieving therapeutic glycemic response 1, n(%) | 103(86.55) | 105(85.37) |
| Odds ratio | 1.15 | |
| 95% confidence interval | [0.55, 2.40] | |
| P-value [1] | 0.7187 | |

| | | |
|---|---|---|
| Proportion = The number of subjects achieving a therapeutic glycemic response 1 at week n / the number of subjects with available data at week n in the efficacy analysis set * 100, n = 6, 12, 18, 24. [1] Testing for difference between DWP16001 and dapagliflozin (logistic regression model with treatment group as a factor and stratification factors (regimen ['Doublet and/or less combination' or 'Triplet and/or more combination'] and HbAlc level at visit 1 [screening][< 8% or ≥ 8%]) as covariates). Note: Therapeutic glycemic response 1 = Change from Visit 2 [randomization] in HbAlc [HbAlc at Visit 2 - HbAlc at each assessment time point] > 0.5% at each assessment time point or HbAlc < 7%. | | |

Data source: Listing 16.2.3:1, Listing 16.2.6:1.

### 6) Proportion of subjects achieving therapeutic response (HbAlc change (HbAlc at Visit 2 - HbA1c at each evaluation time point) > 0.7% or HbAlc < 7% at each evaluation time point compared to Visit 2 (randomization)) at each time point of weeks 6, 12, 18 and 24 after administration of clinical trial drug

The result of summarizing the proportion of subjects achieving therapeutic response (HbAlc change (HbAlc at Visit 2 - HbAlc at each evaluation time point) > 0.5% or HbAlc < 7% at each evaluation time point compared to Visit 2 (randomization)) at each time point of weeks 6, 12, 18, and 24 after administration of clinical trial drug is shown in Table 47.

As a result of logistic regression on PPS, corrected with stratification factors (the conventional regimen for administering a hypoglycemic agent (mono or combination) within 24 weeks prior to obtaining written consent (before the washout period of another hypoglycemic agent), HbAlc level (< 8% or ≥ 8%) at Visit 1 (screening)), compared to the control (dapagliflozin, 10 mg), the odds ratios (95% CI) achieving therapeutic response at each time point in the test group (enavogliflozin, 0.3 mg) compared to the control (dapagliflozin, 10 mg) were 1.16(0.62, 2.15) at week 6, 1.30(0.65, 2.60) at week 12, 1.09(0.60, 2.00) at week 18, and 0.98(0.51, 1.91) at week 24, showing no statistically significant between-group differences at any time point (p=0.6458 at week 6, p=0.4581 at week 12, p=0.7760 at week 18, and p=0.9633 at week 24).

The proportion of subjects achieving therapeutic response over time was 78.45%(91/116) in the test group and 76.23%(93/122) in the control at week 6, 85.47%(100/117) in the test group and 82.11%(101/123) in the control at week 12, 77.78%(91/117) in the test group and 76.42%(94/123) in control at week 18, and 81.51%(97/119) in the test group and 82.11%(101/123) in the control at week 24.

**[Table 47]**

| **Proportion of subjects achieving therapeutic glycemic response 2 at weeks 6, 12, 18** and **24 (per-protocol set)** | | |
|---|---|---|
| | **Enavogliflozin 0.3 mg (N=119)** | **Dapagliflozin 10 mg (N=123)** |
| **Proportion of subjects achieving therapeutic glycemic response 2 at week 6, N** | 116 | 122 |
| Subjects achieving therapeutic glycemic response 2, n(%) | 91(78.45) | 93(76.23) |

| **Logistic regression model** | | |
|---|---|---|
| Odds ratio | 1.16 | |
| 95% confidence interval | [0.62, 2.15] | |
| P-value [1] | 0.6458 | |
| **Proportion of subjects achieving therapeutic glycemic response 2 at week 12, N** | 117 | 123 |
| Subjects achieving therapeutic glycemic response 2, n(%) | 100(85.47) | 101(82.11) |
| Odds ratio | 1.30 | |
| 95% confidence interval | [0.65, 2.60] | |
| P-value [1] | 0.4581 | |
| **Proportion of subjects achieving therapeutic glycemic response 2 at week 18, N** | 117 | 123 |
| Subjects achieving therapeutic glycemic response 2, n(%) | 91(77.78) | 94(76.42) |

| **Logistic regression model** | | |
|---|---|---|
| Odds ratio | 1.09 | |
| 95% confidence interval | [0.60, 2.00] | |
| P-value [1] | 0.7760 | |
| **Proportion of subjects achieving therapeutic glycemic response 2 at week 24, N** | 119 | 123 |
| Subjects achieving therapeutic glycemic response 2, n(%) | 97(81.51) | 101(82.11) |

| **Logistic regression model** | | |
|---|---|---|
| Odds ratio | 0.98 | |
| 95% confidence interval | [0.51, 1.91] | |
| P-value [1] | 0.9633 | |

| | | |
|---|---|---|
| Proportion = The number of subjects achieving a therapeutic glycemic response 2 at week n / the number of subjects with available data at week n in the efficacy analysis set * 100, n = 6, 12, 18, 24. [1] Testing for difference between DWP16001 and dapagliflozin (logistic regression model with treatment group as a factor and stratification factors (regimen ['Doublet and/or less combination' or 'Triplet and/or more combination'] and HbAlc level at visit 1 [screening][< 8% or ≥ 8%]) as covariates). Note: Therapeutic glycemic response 2 = Change from Visit 2 [randomization] in HbAlc [HbAlc at Visit 2 - HbAlc at each assessment time point] > 0.7% at each assessment time point or HbAlc < 7%. | | |

Data source: Listing 16.2.3:1, Listing 16.2.6:1.

### 7) Proportion of subjects achieving therapeutic response (HbAlc change (HbAlc at Visit 2 - HbA1c at each evaluation time point) > 1% or HbAlc < 7% at each evaluation time point compared to Visit 2 (randomization)) at each time point of weeks 6, 12, 18 and 24 after administration of clinical trial drug

The proportion of subjects achieving therapeutic response (HbAlc change (HbAlc at Visit 2 - HbAlc at each evaluation time point) > 1% or HbAlc **<** 7% at each evaluation time point compared to Visit 2 (randomization)) at weeks 6,12, 18, and 24 after administration of the clinical trial drug is shown in Table 48.

As a result of logistic regression on PPS, corrected with stratification factors (the conventional regimen for administering a hypoglycemic agent (mono or combination) within 24 weeks prior to obtaining written consent (before the washout period of another hypoglycemic agent), HbAlc level (< 8% or ≥ 8%) at Visit 1 (screening)), compared to the control (dapagliflozin, 10 mg), the odds ratios (95% CI) achieving therapeutic response at each time point in the test group (enavogliflozin, 0.3 mg) were 1.38(0.78, 2.43) at week 6, 1.31(0.71, 2.42) at week 12, 1.22(0.68, 2.17) at week 18, and 1.22(0.67, 2.25) at week 24, showing no statistically significant between-group differences at any time point (p=0.2640 at week 6, p=0.3950 at week 12, p=0.5063 at week 18, p=0.5130 at week 24).

The proportion of subjects achieving therapeutic response over time was 70.69%(82/116) in the test group and 63.93%(78/122) in the control at week 6, 78.63%(92/117) in the test group and 73.98%(91/123) in the control at week 12, 74.36%(87/117) in the test group and 70.73%(87/123) in the control at week 18, and 78.15%(93/119) in the test group and 74.80%(92/123) in the control at week 24.

**[Table 48]**

| **Proportion of subjects achieving therapeutic glycemic response 3 at weeks 6, 12, 18 and 24 (per-protocol set)** | | |
|---|---|---|
| | **Enavogliflozin 0.3 mg (N=119)** | **Dapagliflozin 10 mg (N=123)** |
| **Proportion of subjects achieving therapeutic glycemic response 3 at week 6, N** | 116 | 122 |
| Subjects achieving therapeutic glycemic response 3, n(%) | 82(70.69) | 78(63.93) |

| **Logistic regression model** | | |
|---|---|---|
| Odds ratio | 1.38 | |
| 95% confidence interval | [0.78, 2.43] | |
| P-value [1] | 0.2640 | |
| **Proportion of subjects achieving therapeutic glycemic response 3 at week 12, N** | 117 | 123 |
| Subjects achieving therapeutic glycemic response 3, n(%) | 92(78.63) | 91(73.98) |

| **Logistic regression model** | | |
|---|---|---|
| Odds ratio | 1.31 | |
| 95% confidence interval | [0.71, 2.42] | |
| P-value [1] | 0.3950 | |
| **Proportion of subjects achieving therapeutic glycemic response 3 at week 18, N** | 117 | 123 |
| Subjects achieving therapeutic glycemic response 3, n(%) | 87(74.36) | 87(70.73) |

| **Logistic regression model** | | |
|---|---|---|
| Odds ratio | 1.22 | |
| 95% confidence interval | [0.68, 2.17] | |
| P-value [1] | 0.5063 | |
| **Proportion of subjects achieving therapeutic glycemic response 3 at week 24, N** | 119 | 123 |
| Subjects achieving therapeutic glycemic response 3, n(%) | 93(78.15) | 92(74.80) |

| **Logistic regression model** | | |
|---|---|---|
| Odds ratio | 1.22 | |
| 95% confidence interval | [0.67, 2.25] | |
| P-value [1] | 0.5130 | |

| | | |
|---|---|---|
| Proportion = The number of subjects achieving a therapeutic glycemic response 3 at week n / the number of subjects with available data at week n in the efficacy analysis set * 100, n = 6, 12, 18, 24. [1] Testing for difference between DWP16001 and dapagliflozin (logistic regression model with treatment group as a factor and stratification factors (regimen ['Doublet and/or less combination' or 'Triplet and/or more combination'] and HbAlc level at visit 1 [screening][< 8% or ≥ 8%]) as covariates). Note: Therapeutic glycemic response 3 = Change from Visit 2 [randomization] in HbAlc [HbAlc at Visit 2 - HbAlc at each assessment time point] > 1% at each assessment time point or HbAlc < 7%. | | |

Data source: Listing 16.2.3:1, Listing 16.2.6:1.

### Exploratory endpoints

### 1) Change in fasting C-peptide at each time point of weeks 6, 12, 18, and 24 after administration of the clinical trial drug compared to Visit 2 (randomization)

The result of summarizing the change in fasting C-peptide from baseline in PPS at weeks 6, 12, 18, and 24 after administration of the clinical trial drug is shown in Table 49.

As a result of analysis of covariance (ANCOVA) on PPS with the baseline value and stratification factors (the conventional regimen (co-administration with two or more agents (one or two agents) or three or more agents) for administering a hypoglycemic agent (before the washout period of another hypoglycemic agent) within 24 weeks prior to obtaining written consent, Visit 1 (screening) HbAlc level (< 8% or ≥ 8%)) as covariates, the least square means (LS means (SE)) for the fasting C-peptide change from baseline at different time points after administration of the clinical trial drug were -0.05(0.02) ng/mL in the test group (enavogliflozin, 0.3 mg) and -0.04(0.02) ng/mL in the control (dapagliflozin, 10 mg) at week 6, -0.09(0.02) ng/mL in the test group and -0.07(0.02) ng/mL in the control at week 12, -0.08(0.02) ng/mL in the test group and -0.06(0.02) ng/mL in the control at week 18, and -0.09(0.02) ng/mL in the test group and -0.08(0.02) ng/mL in the control at week 24. The between-group differences (LS mean differences (95% CI)) were -0.01(-0.06, 0.04) ng/mL at week 6, -0.02(-0.06, 0.03) ng/mL at week 12, -0.02(-0.07, 0.03) ng/mL at week 18, and -0.01(-0.05, 0.03) ng/mL at week 24, showing no statistically significant between-group differences at any time point.

The fasting C-peptide changes (SD) over time were -0.06(0.21) ng/mL in the test group and -0.04(0.15) ng/mL in the control at week 6, -0.09(0.20) ng/mL in the test group and -0.06(0.19) ng/mL in the control at week 12, -0.09(0.22) ng/mL in the test group and -0.06(0.18) ng/mL in the control at week 18, and -0.09(0.18) ng/mL in the test group and -0.07(0.19) ng/mL in the control at week 24, showing statistically significant between-group differences at all time points and in all administration groups.

**[Table 49]**

| **Change from baseline in fasting C-peptide at weeks 6, 12, 18 and 24 (per-protocol set)** | | |
|---|---|---|
| | **Enavogliflozin 0.3 mg (N=119)** | **Dapagliflozin 10 mg (N=123)** |
| **Baseline** | | |
| n | 119 | 123 |
| Mean (SD) | 0.73(0.25) | 0.69(0.21) |
| Median | 0.70 | 0.70 |
| Min, Max | 0.30, 1.46 | 0.20, 1.42 |

| **Week 6** | | |
|---|---|---|
| n | 116 | 122 |
| Mean (SD) | 0.67(0.29) | 0.65(0.21) |
| Median | 0.63 | 0.63 |
| Min, Max | 0.23, 1.92 | 0.17, 1.36 |

| **Change from baseline at week 6** | | |
|---|---|---|
| n | 116 | 122 |
| Mean (SD) | -0.06(0.21) | -0.04(0.15) |
| Median | -0.07 | -0.03 |
| Min, Max | -0.66, 0.79 | -0.56, 0.33 |
| P-value [1] | <0.0001 (w) | 0.0045 (w) |

| **ANCOVA result** | | |
|---|---|---|
| LS mean (SE) | -0.05(0.02) | -0.04(0.02) |
| LS mean difference | -0.01 | |
| 95% confidence interval for difference | [-0.06, 0.04] | |
| P-value [2] | 0.6602 | |

| **Week 12** | | |
|---|---|---|
| n | 117 | 123 |
| Mean (SD) | 0.64(0.23) | 0.63(0.23) |
| Median | 0.63 | 0.60 |
| Min, Max | 0.20, 1.22 | 0.20, 1.56 |

| **Change from baseline at week 12** | | |
|---|---|---|
| n | 117 | 123 |
| Mean (SD) | -0.09(0.20) | -0.06(0.19) |
| Median | -0.10 | -0.03 |
| Min, Max | -0.83, 0.60 | -0.66, 0.70 |
| P-value [1] | <0.0001 (w) | <0.0001 (w) |

| **ANCOVA result** | | |
|---|---|---|
| LS mean (SE) | -0.09(0.02) | -0.07(0.02) |
| LS mean difference | -0.02 | |
| 95% confidence interval for difference | [-0.06, 0.03] | |
| P-value [2] | 0.4519 | |

| **Week 18** | | |
|---|---|---|
| n | 117 | 123 |
| Mean (SD) | 0.64(0.26) | 0.63(0.22) |
| Median | 0.60 | 0.60 |
| Min, Max | 0.26, 1.92 | 0.13, 1.46 |

| **Change from baseline at week 18** | | |
|---|---|---|
| n | 117 | 123 |
| Mean (SD) | -0.09(0.22) | -0.06(0.18) |
| Median | -0.10 | -0.07 |
| Min, Max | -0.89, 1.29 | -0.56, 0.60 |
| P-value [1] | <0.0001 (w) | <0.0001 (w) |

| **ANCOVA result** | | |
|---|---|---|
| LS mean (SE) | -0.08(0.02) | -0.06(0.02) |
| LS mean difference | -0.02 | |
| 95% confidence interval for difference | [-0.07, 0.03] | |
| P-value [2] | 0.4398 | |

| **Week 24** | | |
|---|---|---|
| n | 119 | 123 |
| Mean (SD) | 0.64(0.20) | 0.62(0.24) |
| Median | 0.63 | 0.56 |
| Min, Max | 0.26, 1.26 | 0.13, 1.42 |

| **Change from baseline at week 24** | | |
|---|---|---|
| n | 119 | 123 |
| Mean (SD) | -0.09(0.18) | -0.07(0.19) |
| Median | -0.07 | -0.10 |
| Min, Max | -0.73, 0.56 | -0.60, 0.40 |
| P-value [1] | <0.0001 (w) | <0.0001 (t) |

| **ANCOVA result** | | |
|---|---|---|
| LS mean (SE) | -0.09(0.02) | -0.08(0.02) |
| LS mean difference | -0.01 | |
| 95% confidence interval for difference | [-0.05, 0.03] | |
| P-value [2] | 0.6760 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbAlc level at visit 1 [screening][< 8% or ≥ 8%]) as covariates). Note: Baseline is Visit 2 (randomization). Data source: Listing 16.2.3:1, Listing 16.2.6:1. | | |

### 2) Change in body weight at each time point of weeks 6, 12, 18, and 24 after administration of the clinical trial drug compared to Visit 2 (randomization)

The result of the summarizing the change in body weight from baseline in PPS at weeks 6, 12, 18, and 24 after administration of the clinical trial drug is shown in Table 50.

As a result of analysis of covariance (ANCOVA) on PPS with the baseline value and stratification factors (the conventional regimen (co-administration with two or more agents (one or two agents) or three or more agents) for administering a hypoglycemic agent (before the washout period of another hypoglycemic agent) within 24 weeks prior to obtaining written consent, Visit 1 (screening) HbAlc level (< 8% or ≥ 8%)) as covariates, the least square means (LS means (SE)) for the change in body weight from the baseline at different time points after administration of the clinical trial drug were -1.92(0.17) kg in the test group (enavogliflozin, 0.3 mg) and -1.80(0.16) kg in the control (dapagliflozin, 10 mg) at week 6, -2.72(0.20) kg in the test group and -2.71(0.19) kg in the control at week 12, -3.16(0.24) kg, in the test group and -3.01(0.23) kg in the control at week 18, and -3.15(0.23) kg in the test group and -3.01(0.22) kg in the control at week 24. The between-group differences (LS mean differences (95% CI)) were -0.13(-0.55, 0.30) kg at week 6, -0.01(-0.51, 0.49) kg at week 12, -0.15(-0.76, 0.46) kg at week 18, and -0.15(-0.73, 0.44) kg at week 24, showing no statistically significant between-group differences at any time point.

The changes in body weight (SD) over time were -1.87(1.98) kg in the test group and -1.68(1.41) kg in the control at week 6, -2.65(2.23) kg in the test group and -2.58(1.79) kg in the control at week 12, -3.10(2.94) kg in the test group and -2.84(1.94) kg in the control at week 18, and -3.09(2.72) kg in the test group and -2.83(2.02) kg in the control at week 24, showing statistically significant between-group differences at all time points and in all administration groups.

**[Table 50]**

| **Change from baseline in body weight at weeks 6, 12, 18 and 24 (per-protocol set)** | | |
|---|---|---|
| | **Enavogliflozin 0.3 mg (N=119)** | **Dapagliflozin 10 mg (N=123)** |
| **Baseline** | | |
| n | 119 | 123 |
| Mean (SD) | 69.28(12.11) | 67.22(11.40) |
| Median | 67.90 | 64.80 |
| Min, Max | 49.60, 107.30 | 47.50, 109.20 |

| **Week 6** | | |
|---|---|---|
| n | 116 | 122 |
| Mean (SD) | 67.13(11.90) | 55.20(10.46) |
| Median | 66.80 | 53.90 |
| Min, Max | 46.40, 104.50 | 46.00, 97.70 |

| **Change from baseline at week 6** | | |
|---|---|---|
| n | 116 | 122 |
| Mean (SD) | -1.87(1.98) | -1.68(1.41) |
| Median | -1.90 | -1.55 |
| Min, Max | -8.10, 9.40 | -5.20, 1.20 |
| P-value [1] | <0.0001 (w) | <0.0001 (t) |

| **ANCOVA result** | | |
|---|---|---|
| LS mean (SE) | -1.92(0.17) | -1.80(0.16) |
| LS mean difference | -0.13 | |
| 95% confidence interval for difference | [-0.55, 0.30] | |
| P-value [2] | 0.5627 | |

| **Week 12** | | |
|---|---|---|
| n | 117 | 123 |
| Mean (SD) | 66.54(11.79) | 64.64(11.16) |
| Median | 65.80 | 53.20 |
| Min, Max | 44.00, 102.10 | 45.00, 106.20 |

| **Change from baseline at week 12** | | |
|---|---|---|
| n | 117 | 123 |
| Mean (SD) | -2.65(2.23) | -2.58(1.79) |
| Median | -2.90 | -2.50 |
| Min, Max | -11.10, 7.40 | -10.70, 1.00 |
| P-value [1] | <0.0001 (w) | <0.0001 (w) |

| **ANCOVA result** | | |
|---|---|---|
| LS mean (SE) | -2.72(0.20) | -2.71(0.19) |
| LS mean difference | -0.01 | |
| 95% confidence interval for difference | [-0.51, 0.49] | |
| P-value [2] | 0.9748 | |

| **Week 18** | | |
|---|---|---|
| n | 117 | 123 |
| Mean (SD) | 66.18(11.64) | 64.37(11.11) |
| Median | 65.40 | 62.30 |
| Min, Max | 44.50, 101.80 | 45.00, 104.50 |

| **Change from baseline at week 18** | | |
|---|---|---|
| n | 117 | 123 |
| Mean (SD) | -3.10(2.94) | -2.84(1.94) |
| Median | -2.90 | -2.70 |
| Min, Max | -14.90, 7.60 | -8.90, 5.30 |
| P-value [1] | <0.0001 (w) | <0.0001 (w) |

| **ANCOVA result** | | |
|---|---|---|
| LS mean (SE) | -3.16(0.24) | -3.01(0.23) |
| LS mean difference | -0.15 | |
| 95% confidence interval for difference | [-0.76, 0.46] | |
| P-value [2] | 0.6226 | |

| **Week 24** | | |
|---|---|---|
| n | 119 | 123 |
| Mean (SD) | 66.19(11.58) | 54.38(11.03) |
| Median | 65.10 | 52.90 |
| Min, Max | 46.50, 101.40 | 44.00, 105.70 |

| **Change from baseline at week 24** | | |
|---|---|---|
| n | 119 | 123 |
| Mean (SD) | -3.09(2.72) | -2.83(2.02) |
| Median | -3.00 | -2.60 |
| Min, Max | -16.00, 7.50 | -8.50, 1.40 |
| P-value [1] | <0.0001 (w) | <0.0001 (t) |

| **ANCOVA result** | | |
|---|---|---|
| LS mean (SE) | -3.15(0.23) | -3.01(0.22) |
| LS mean difference | -0.15 | |
| 95% confidence interval for difference | [-0.73, 0.44] | |
| P-value [2] | 0.6226 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbAlc level at visit 1 [screening][< 8% or ≥ 8%]) as covariates). Note: Baseline is Visit 2 (randomization). Data source: Listing 16.2.3:1, Listing 16.2.6:1. | | |

### 3) Changes in fasting lipid concentrations (total cholesterol, LDL-C, HDL-C, and triglyceride) at each time point of weeks 6, 12, 18, and 24 after administration of the clinical trial drug compared to Visit 2 (randomization)

### ① Total cholesterol

The result of summarizing the change in total cholesterol from baseline in modified per-protocol set 1 (mPPS1) at weeks 6, 12, 18, and 24 after administration of the clinical trial drug is shown in Table 51.

As a result of analysis of covariance (ANCOVA) on mPPS1 with the baseline value and stratification factors (the conventional regimen (co-administration with two or more agents (one or two agents) or three or more agents) for administering a hypoglycemic agent (before the washout period of another hypoglycemic agent) within 24 weeks prior to obtaining written consent, Visit 1 (screening) HbAlc level (< 8% or ≥ 8%)) as covariates, the least square means (LS means (SE)) for the change in total cholesterol from the baseline at different time points after administration of the clinical trial drug were 5.07(1.97) mg/dL in the test group (enavogliflozin, 0.3 mg) and 2.63(1.93) mg/dL in the control (dapagliflozin, 10 mg) at week 6, 6.80(2.04) mg/dL in the test group and 5.40(1.98) mg/dL in the control at week 12, 4.27(1.94) mg/dL in the test group and 3.66(1.88) mg/dL in the control at week 18, and 4.48(2.07) mg/dL in the test group and 5.57(2.03) mg/dL in the control at week 24. The between-group differences (LS mean differences (95% CI)) were 2.45(-2.63, 7.53) mg/dL at week 6, 1.40(-3.85, 6.64) mg/dL at week 12, 0.61(-4.36, 5.58) mg/dL at week 18, and -1.09(-6.43, 4.25) mg/dL at week 24, showing no statistically significant between-group differences at any time point.

The changes in total cholesterol (SD) over time were 4.65(19.51) mg/dL in the test group and 1.55(19.56) mg/dL in the control at week 6, 7.79(20.63) mg/dL in the test group and 4.61(20.98) mg/dL in the control at week 12, 5.06(19.64) mg/dL in the test group and 2.51(20.37) mg/dL in the control at week 18, and 5.59(17.04) mg/dL in the test group and 4.69(25.08) mg/dL in the control at week 24, showing statistically significant between-group differences at all time points and in all administration groups except the controls at weeks 6 and 18.

**[Table 51]**

| **Change from baseline in fasting lipid parameters at weeks 6, 12, 18 and 24 (modified per-protocol set 1) [total cholesterol]** | | |
|---|---|---|
| | **Enavogliflozin 0.3 mg (N=118)** | **Dapagliflozin 10 mg (N=120)** |
| **Baseline** | | |
| n | 118 | 120 |
| Mean (SD) | 134.47(24.91) | 143.80(35.51) |
| Median | 132.00 | 138.00 |
| Min, Max | 90.00, 199.00 | 77.00, 265.00 |

| **Week 6** | | |
|---|---|---|
| n | 115 | 119 |
| Mean (SD) | 138.43(30.14) | 145.04(38.88) |
| Median | 134.00 | 136.00 |
| Min, Max | 76.00, 230.00 | 71.00, 279.00 |

| **Change from baseline at week 6** | | |
|---|---|---|
| n | 115 | 119 |
| Mean (SD) | 4.65(19.51) | 1.55(19.56) |
| Median | 3.00 | 2.00 |
| Min, Max | -37.00, 111.00 | -94.00, 41.00 |
| P-value [1] | 0.0174 (w) | 0.2378 (w) |

| **ANCOVA result** | | |
|---|---|---|
| LS mean (SE) | 5.07(1.97) | 2.63(1.93) |
| LS mean difference | 2.45 | |
| 95% confidence interval for difference | [-2.63, 7.53] | |
| P-value [2] | 0.3438 | |

| **Week 12** | | |
|---|---|---|
| n | 116 | 120 |
| Mean (SD) | 142.09(27.82) | 148.41(36.32) |
| Median | 136.00 | 140.50 |
| Min, Max | 79.00, 228.00 | 77.00, 278.00 |

| **Change from baseline at week 12** | | |
|---|---|---|
| n | 116 | 120 |
| Mean (SD) | 7.79(20.63) | 4.61(20.98) |
| Median | 7.00 | 6.00 |
| Min, Max | -35.00, 82.00 | -78.00, 66.00 |
| P-value [1] | 0.0001 (w) | 0.0177 (t) |

| **ANCOVA result** | | |
|---|---|---|
| LS mean (SE) | 6.80(2.04) | 5.40(1.98) |
| LS mean difference | 1.40 | |
| 95% confidence interval for difference | [-3.85, 6.64] | |
| P-value [2] | 0.5998 | |

| **Week 18** | | |
|---|---|---|
| n | 116 | 120 |
| Mean (SD) | 139.38(29.27) | 146.31(32.64) |
| Median | 134.00 | 141.00 |
| Min, Max | 88.00, 237.00 | 81.00, 247.00 |

| **Change from baseline at week 18** | | |
|---|---|---|
| n | 116 | 120 |
| Mean (SD) | 5.06(19.64) | 2.51(20.37) |
| Median | 3.00 | 1.00 |
| Min, Max | -46.00, 119.00 | -67.00, 51.00 |
| P-value [1] | 0.0086 (w) | 0.1377 (w) |

| **ANCOVA result** | | |
|---|---|---|
| LS mean (SE) | 4.27(1.94) | 3.66(1.88) |
| LS mean difference | 0.61 | |
| 95% confidence interval for difference | [-4.36, 5.58] | |
| P-value [2] | 0.8088 | |

| **Week 24** | | |
|---|---|---|
| n | 118 | 120 |
| Mean (SD) | 140.06(26.82) | 148.49(36.41) |
| Median | 134.00 | 145.00 |
| Min, Max | 86.00, 217.00 | 73.00, 265.00 |

| **Change from baseline at week 24** | | |
|---|---|---|
| n | 118 | 120 |
| Mean (SD) | 5.59(17.04) | 4.69(25.08) |
| Median | 5.50 | 3.00 |
| Min, Max | -42.00, 73.00 | -103.00, 113.00 |
| P-value [1] | 0.0002 (w) | 0.0440 (w) |

| **ANCOVA result** | | |
|---|---|---|
| LS mean (SE) | 4.48(2.07) | 5.57(2.03) |
| LS mean difference | -1.09 | |
| 95% confidence interval for difference | [-6.43, 4.25] | |
| P-value [2] | 0.6885 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbAlc level at visit 1 [screening][< 8% or ≥ 8%]) as covariates). Note: Baseline is Visit 2 (randomization). Data source: Listing 16.2.3:1, Listing 16.2.6:1. | | |

### ② LDL-C

The result of summarizing LDL-C changes from baseline in mPPS1 at weeks 6, 12, 18, and 24 after administration of the clinical trial drug is shown in Table 52.

As a result of analysis of covariance (ANCOVA) on mPPS1 with the baseline value and stratification factors (the conventional regimen for administering a hypoglycemic agent (in combination with two or less agent ((one or two agents) or three or more agents) within 24 weeks prior to obtaining written consent (before the washout period of another hypoglycemic agent), HbAlc level (< 8% or ≥ 8%) at Visit 1 (screening)) as covariates, the least square means (LS means (SE)) for the changes in LDL-C from the baseline at different time points after administration of the clinical trial drug were 2.49(1.81) mg/dL in the test group (enavogliflozin, 0.3 mg) and 1.91(1.78) mg/dL in the control (dapagliflozin, 10 mg) at week 6, 2.95(1.79) mg/dL in the test group and 2.71(1.75) mg/dL in the control at week 12, 0.29(1.63) mg/dL in the test group and 1.38(1.59) mg/dL in the control at week 18, and 0.85(1.89) mg/dL in the test group and 3.12(1.85) mg/dL in the control at week 24. The between-group differences (LS mean difference/test group-control (95% CI)) were 0.57(-4.10, 5.24) mg/dL at week 6, 0.24(-4.37, 4.85) mg/dL at week 12, -1.10(-5.28, 3.08) mg/dL at week 18, and -2.27(-7.13, 2.59) mg/dL at week 24, showing no statistically significant between-group differences at any time point.

The changes in LDL-C (SD) over time were 2.42(16.77) mg/dL in the test group and 1.40(19.16) mg/dL in the control at week 6, 3.76(15.91) mg/dL in the test group and 2.45(20.28) mg/dL in the control at week 12, 0.94(14.64) mg/dL in the test group and 0.93(18.49) mg/dL in the control at week 18, and 1.75(14.83) mg/dL in the test group and 2.72(23.25) mg/dL in the control at week 24, showing a statistically significant between-group difference only in the test group at week 12.

**[Table 52]**

| **Change from baseline in fasting lipid parameters at weeks 6, 12, 18 and 24 (modified per-protocol set 1) [LDL-C]** | | |
|---|---|---|
| | **Enavogliflozin 0.3 mg (N=118)** | **Dapagliflozin 10 mg (N=120)** |
| **Baseline** | | |
| n | 118 | 120 |
| Mean (SD) | 71.41(22.84) | 77.41(29.44) |
| Median | 68.00 | 71.00 |
| Min, Max | 29.00, 129.00 | 24.00, 177.00 |

| **Week 6** | | |
|---|---|---|
| n | 115 | 119 |
| Mean (SD) | 73.35(27.86) | 78.50(33.38) |
| Median | 68.00 | 70.00 |
| Min, Max | 23.00, 171.00 | 29.00, 193.00 |

| **Change from baseline at week 6** | | |
|---|---|---|
| n | 115 | 119 |
| Mean (SD) | 2.42(16.77) | 1.40(19.16) |
| Median | -1.00 | 1.00 |
| Min, Max | -28.00, 103.00 | -107.00, 80.00 |
| P-value [1] | 0.5573 (w) | 0.2588 (w) |

| **ANCOVA result** | | |
|---|---|---|
| LS mean (SE) | 2.49(1.81) | 1.91(1.78) |
| LS mean difference | 0.57 | |
| 95% confidence interval for difference | [-4.10, 5.24] | |
| P-value [2] | 0.8092 | |

| **Week 12** | | |
|---|---|---|
| n | 116 | 120 |
| Mean (SD) | 75.11(25.25) | 79.86(31.47) |
| Median | 72.00 | 72.50 |
| Min, Max | 25.00, 160.00 | 25.00, 191.00 |

| **Change from baseline at week 12** | | |
|---|---|---|
| n | 116 | 120 |
| Mean (SD) | 3.76(15.91) | 2.45(20.28) |
| Median | 3.00 | 4.00 |
| Min, Max | -24.00, 67.00 | -86.00, 78.00 |
| P-value [1] | 0.0353 (w) | 0.0542 (w) |

| **ANCOVA result** | | |
|---|---|---|
| LS mean (SE) | 2.95(1.79) | 2.71(1.75) |
| LS mean difference | 0.24 | |
| 95% confidence interval for difference | [-4.37, 4.85] | |
| P-value [2] | 0.9179 | |

| **Week 18** | | |
|---|---|---|
| n | 116 | 120 |
| Mean (SD) | 72.10(26.14) | 78.33(28.02) |
| Median | 68.00 | 73.00 |
| Min, Max | 16.00, 165.00 | 36.00, 167.00 |

| **Change from baseline at week 18** | | |
|---|---|---|
| n | 116 | 120 |
| Mean (SD) | 0.94(14.64) | 0.93(18.49) |
| Median | 0.00 | 1.50 |
| Min, Max | -29.00, 74.00 | -82.00, 44.00 |
| P-value [1] | 0.9464 (w) | 0.2481 (w) |

| **ANCOVA result** | | |
|---|---|---|
| LS mean (SE) | 0.29(1.63) | 1.38(1.59) |
| LS mean difference | -1.10 | |
| 95% confidence interval for difference | [-5.28, 3.08] | |
| P-value [2] | 0.6056 | |

| **Week 24** | | |
|---|---|---|
| n | 118 | 120 |
| Mean (SD) | 73.15(25.01) | 80.13(31.18) |
| Median | 70.50 | 71.50 |
| Min, Max | 25.00, 145.00 | 28.00, 182.00 |

| **Change from baseline at week 24** | | |
|---|---|---|
| n | 118 | 120 |
| Mean (SD) | 1.75(14.83) | 2.72(23.25) |
| Median | 0.50 | 1.50 |
| Min, Max | -31.00, 71.00 | -114.00, 113.00 |
| P-value [1] | 0.5077 (w) | 0.1251 (w) |

| **ANCOVA result** | | |
|---|---|---|
| LS mean (SE) | 0.85(1.89) | 3.12(1.85) |
| LS mean difference | -2.27 | |
| 95% confidence interval for difference | [-7.13, 2.59] | |
| P-value [2] | 0.3580 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbAlc level at visit 1 [screening][< 8% or ≥ 8%]) as covariates). Note: Baseline is Visit 2 (randomization). Data source: Listing 16.2.3:1, Listing 16.2.6:1. | | |

### ③ HDL-C

The result of summarizing the change in HDL-C from baseline in mPPS1 at weeks 6, 12, 18, and 24 after administration of the clinical trial drug is shown in Table 53.

As a result of analysis of covariance (ANCOVA) on mPPS1 with the baseline value and stratification factors (the conventional regimen (co-administration with two or more agents (one or two agents) or three or more agents) for administering a hypoglycemic agent (before the washout period of another hypoglycemic agent) within 24 weeks prior to obtaining written consent, Visit 1 (screening) HbAlc level (< 8% or ≥ 8%)) as covariates, the least square means (LS means (SE)) for the changes in HDL-C from the baseline at different time points after administration of the clinical trial drug were 1.96(0.57) mg/dL in the test group (enavogliflozin, 0.3 mg) and 1.13(0.56) mg/dL in the control (dapagliflozin, 10 mg) at week 6, 3.18(0.62) mg/dL in the test group and 2.84(0.60) mg/dL in the control at week 12, 3.82(0.64) mg/dL in the test group and 3.42(0.62) mg/dL in the control at week 18, and 4.32(0.71) mg/dL in the test group and 4.40(0.69) mg/dL in the control at week 24. The between-group differences (LS mean difference/test group-control (95% CI)) were 0.83(-0.63, 2.29) mg/dL at week 6, 0.34(-1.25, 1.92) mg/dL at week 12, 0.40(-1.23, 2.02) mg/dL at week 18, and -0.08(-1.90, 1.73) mg/dL at week 24, showing no statistically significant between-group differences at any time point.

The changes in HDL-C over time were 1.90(5.34) mg/dL in the test group and 0.94(6.05) mg/dL in the control at week 6, 3.17(6.08) mg/dL in the test group and 2.65(6.41) mg/dL in the control at week 12, 4.09(6.15) mg/dL in the test group and 3.57(6.53) mg/dL in the control at week 18, and 4.53(6.90) mg/dL in the test group and 4.44(7.34) mg/dL in the control at week 24, showing statistically significant between-group differences at all time points and in all administration groups except the control at week 6.

**[Table 53]**

| **Change from baseline in fasting lipid parameters at weeks 6, 12, 18 and 24 (modified per-protocol set 1) [HDL-C]** | | |
|---|---|---|
| | **Enavogliflozin 0.3 mg (N=118)** | **Dapagliflozin 10 mg (N=120)** |
| **Baseline** | | |
| n | 118 | 120 |
| Mean (SD) | 46.06(9.88) | 47.43(10.83) |
| Median | 43.00 | 47.00 |
| Min, Max | 30.00, 69.00 | 27.00, 86.00 |
| **Week 6** | | |
| n | 115 | 119 |
| Mean (SD) | 47.99(11.09) | 48.39(11.63) |
| Median | 46.00 | 48.00 |
| Min, Max | 26.00, 75.00 | 28.00, 101.00 |

| **Change from baseline at week 6** | | |
|---|---|---|
| n | 115 | 119 |
| Mean (SD) | 1.90(5.34) | 0.94(6.05) |
| Median | 2.00 | 1.00 |
| Min, Max | -12.00, 16.00 | -15.00, 23.00 |
| P-value [1] | 0.0002 (t) | 0.0895 (w) |

| **ANCOVA result** | | |
|---|---|---|
| LS mean (SE) | 1.96(0.57) | 1.13(0.56) |
| LS mean difference | 0.83 | |
| 95% confidence interval for difference | [-0.63, 2.29] | |
| P-value [2] | 0.2652 | |

| **Week 12** | | |
|---|---|---|
| n | 116 | 120 |
| Mean (SD) | 49.21(11.35) | 50.08(11.22) |
| Median | 47.50 | 49.00 |
| Min, Max | 31.00, 82.00 | 30.00, 99.00 |

| **Change from baseline at week 12** | | |
|---|---|---|
| n | 116 | 120 |
| Mean (SD) | 3.17(6.08) | 2.65(6.41) |
| Median | 3.00 | 2.00 |
| Min, Max | -12.00, 21.00 | -10.00, 24.00 |
| P-value [1] | <0.0001 (t) | <0.0001 (w) |

| **ANCOVA result** | | |
|---|---|---|
| LS mean (SE) | 3.18(0.62) | 2.84(0.60) |
| LS mean difference | 0.34 | |
| 95% confidence interval for difference | [-1.25, 1.92] | |
| P-value [2] | 0.6765 | |

| **Week 18** | | |
|---|---|---|
| n | 116 | 120 |
| Mean (SD) | 50.17(11.21) | 50.99(11.88) |
| Median | 49.50 | 51.00 |
| Min, Max | 29.00, 78.00 | 28.00, 102.00 |

| **Change from baseline at week 18** | | |
|---|---|---|
| n | 116 | 120 |
| Mean (SD) | 4.09(6.15) | 3.57(6.53) |
| Median | 3.00 | 3.00 |
| Min, Max | -11.00, 22.00 | -12.00, 20.00 |
| P-value [1] | <0.0001 (t) | <0.0001 (t) |

| **ANCOVA result** | | |
|---|---|---|
| LS mean (SE) | 3.82(0.64) | 3.42(0.62) |
| LS mean difference | 0.40 | |
| 95% confidence interval for difference | [-1.23, 2.02] | |
| P-value [2] | 0.6317 | |

| **Week 24** | | |
|---|---|---|
| n | 118 | 120 |
| Mean (SD) | 50.58(11.93) | 51.87(11.84) |
| Median | 48.50 | 50.50 |
| Min, Max | 28.00, 92.00 | 29.00, 106.00 |

| **Change from baseline at week 24** | | |
|---|---|---|
| n | 118 | 120 |
| Mean (SD) | 4.53(6.90) | 4.44(7.34) |
| Median | 4.00 | 4.00 |
| Min, Max | -12.00, 29.00 | -15.00, 34.00 |
| P-value [1] | <0.0001 (t) | <0.0001 (w) |

| **ANCOVA result** | | |
|---|---|---|
| LS mean (SE) | 4.32(0.71) | 4.40(0.69) |
| LS mean difference | -0.08 | |
| 95% confidence interval for difference | [-1.90, 1.73] | |
| P-value [2] | 0.9279 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbAlc level at visit 1 [screening][< 8% or ≥ 8%]) as covariates). Note: Baseline is Visit 2 (randomization). Data source: Listing 16.2.3:1, Listing 16.2.6:1. | | |

### ④ Triglycerides

The result of summarizing the change in triglycerides from baseline in mPPS1 at weeks 6, 12, 18, and 24 after administration of the clinical trial drug is shown in Table 54.

As a result of analysis of covariance (ANCOVA) on mPPS1 with the baseline value and stratification factors (the conventional regimen (co-administration with two or more agents (one or two agents) or three or more agents) for administering a hypoglycemic agent (before the washout period of another hypoglycemic agent) within 24 weeks prior to obtaining written consent, Visit 1 (screening) HbAlc level (< 8% or ≥ 8%)) as covariates, the least square means (LS means (SE)) for the triglyceride changes from the baseline at different time points after administration of the clinical trial drug were -6.85(4.47) mg/dL in the test group (enavogliflozin, 0.3 mg) and -5.39(4.38) mg/dL in the control (dapagliflozin, 10 mg) at week 6, -3.23(5.25) mg/dL in the test group and -12.02(5.12) mg/dL in the control at week 12, -11.64(4.46) mg/dL in the test group and -12.39(4.34) mg/dL in the control at week 18, and -8.22(4.01) mg/dL in the test group and -8.78(3.94) mg/dL in the control at week 24. The between-group differences (LS mean difference/test group-control (95% CI)) were -1.46(-12.95, 10.03) mg/dL at week 6, 8.79(-4.68, 22.27) mg/dL at week 12, 0.75(-10.66, 12.16) mg/dL at week 18, and 0.56(-9.75, 10.87) mg/dL at week 24, showing no statistically significant between-group differences at any time point.

The changes in triglycerides (SD) over time were -4.37(40.21) mg/dL in the test group and -10.00(72.87) mg/dL in the control at week 6, 0.59(51.73) mg/dL in the test group and -16.03(81.60) mg/dL in the control at week 12, -9.60(49.20) mg/dL in the test group and -17.31(70.25) mg/dL in the control at week 18, and -6.05(42.91) mg/dL in the test group and -13.81(71.91) mg/dL in the control at week 24, showing statistically significant between-group differences at all time points and in all administration groups except the test group and the control at week 18.

**[Table 54]**

| **Change from baseline in fasting parameters at weeks 6, 12, 18 and 24 lipid (modified per-protocol set 1) [triglycerides]** | | |
|---|---|---|
| | **Enavogliflozin 0.3 mg (N=118)** | **Dapagliflozin 10 mg (N=120)** |
| **Baseline** | | |
| n | 118 | 120 |
| Mean (SD) | 119.06(58.44) | 132.49(94.92) |
| Median | 106.00 | 103.00 |
| Min, Max | 47.00, 385.00 | 42.00, 754.00 |

| **Week 6** | | |
|---|---|---|
| n | 115 | 119 |
| Mean (SD) | 112.71(51.53) | 121.81(65.42) |
| Median | 101.00 | 98.00 |
| Min, Max | 44.00, 312.00 | 46.00, 435.00 |

| **Change from baseline at week 6** | | |
|---|---|---|
| n | 115 | 119 |
| Mean (SD) | -4.37(40.21) | -10.00(72.87) |
| Median | -1.00 | 1.00 |
| Min, Max | -112.00, 115.00 | -559.00, 147.00 |
| P-value [1] | 0.2178 (w) | 0.3894 (w) |

| **ANCOVA result** | | |
|---|---|---|
| LS mean (SE) | -6.85(4.47) | -5.39(4.38) |
| LS mean difference | -1.46 | |
| 95% confidence interval for difference | [-12.95, 10.03] | |
| P-value [2] | 0.8024 | |

| **Week 12** | | |
|---|---|---|
| n | 116 | 120 |
| Mean (SD) | 118.98(69.11) | 116.46(54.96) |
| Median | 98.50 | 104.50 |
| Min, Max | 45.00, 420.00 | 46.00, 347.00 |

| **Change from baseline at week 12** | | |
|---|---|---|
| n | 116 | 120 |
| Mean (SD) | 0.59(51.73) | -16.03(81.60) |
| Median | -3.00 | -6.00 |
| Min, Max | -104.00, 233.00 | -574.00, 121.00 |
| P-value [1] | 0.6211 (w) | 0.1008 (w) |

| **ANCOVA result** | | |
|---|---|---|
| LS mean (SE) | -3.23(5.25) | -12.02(5.12) |
| LS mean difference | 8.79 | |
| 95% confidence interval for difference | [-4.68, 22.27] | |
| P-value [2] | 0.1997 | |

| **Week 18** | | |
|---|---|---|
| n | 116 | 120 |
| Mean (SD) | 109.52(62.14) | 115.18(53.02) |
| Median | 95.50 | 103.00 |
| Min, Max | 42.00, 443.00 | 37.00, 322.00 |

| **Change from baseline at week 18** | | |
|---|---|---|
| n | 116 | 120 |
| Mean (SD) | -9.60(49.20) | -17.31(70.25) |
| Median | -2.00 | -8.50 |
| Min, Max | -152.00, 216.00 | -432.00, 122.00 |
| P-value [1] | 0.0318 (w) | 0.0238 (w) |

| **ANCOVA result** | | |
|---|---|---|
| LS mean (SE) | -11.64(4.46) | -12.39(4.34) |
| LS mean difference | 0.75 | |
| 95% confidence interval for difference | [-10.66, 12.16] | |
| P-value [2] | 0.8965 | |

| **Week 24** | | |
|---|---|---|
| n | 118 | 120 |
| Mean (SD) | 113.01(50.84) | 118.68(55.61) |
| Median | 101.00 | 104.00 |
| Min, Max | 51.00, 322.00 | 46.00, 352.00 |

| **Change from baseline at week 24** | | |
|---|---|---|
| n | 118 | 120 |
| Mean (SD) | -6.05(42.91) | -13.81(71.91) |
| Median | -1.00 | -6.00 |
| Min, Max | -131.00, 112.00 | -402.00, 145.00 |
| P-value [1] | 0.1283 (t) | 0.1593 (w) |

| **ANCOVA result** | | |
|---|---|---|
| LS mean (SE) | -8.22(4.01) | -8.78(3.94) |
| LS mean difference | 0.56 | |
| 95% confidence interval for difference | [-9.75, 10.87] | |
| P-value [2] | 0.9148 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbAlc level at visit 1 [screening][< 8% or ≥ 8%]) as covariates). Note: Baseline is Visit 2 (randomization). Data source: Listing 16.2.3:1, Listing 16.2.6:1. | | |

### 4) Changes in systolic or diastolic blood pressure at each of weeks 6, 12, 18, and 24 after administration of clinical trial drug compared to Visit 2 (randomization)

### ① Systolic blood pressure (SBP)

The result of summarizing the changes in systolic blood pressure from baseline in modified per-protocol set 2 (mPPS2) at weeks 6, 12, 18, and 24 after administration of the clinical trial drug is shown in Table 55.

As a result of analysis of covariance (ANCOVA) on mPPS2 with the baseline value and stratification factors (the conventional regimen for administering a hypoglycemic agent (mono or combination) within 24 weeks prior to obtaining written consent (before the washout period of another hypoglycemic agent), HbAlc level (< 8% or ≥ 8%) at Visit 1 (screening)) as covariates, the least square means (LS means (SE)) for the changes in systolic blood pressure from the baseline at different time points after administration of the clinical trial drug were -2.76(0.90) mmHg in the test group (enavogliflozin, 0.3 mg) and -3.49(0.87) mmHg in the control (dapagliflozin, 10 mg) at week 6, -6.43(0.88) mmHg in the test group and -5.06(0.85) mmHg in the control at week 12, -6.82(0.92) mmHg in the test group and -5.16(0.88) mmHg in the control at week 18, and -4.49(0.96) mmHg in the test group and -4.27(0.93) mmHg in the control at week 24. The between-group differences (LS mean difference/test group-control (95% CI)) were 0.73(-1.56, 3.02) mmHg at week 6, -1.37(-3.62, 0.88) mmHg at week 12, -1.66(-4.00, 0.68) mmHg at week 18, and -0.22(-2.67, 2.23) mmHg at week 24, showing no statistically significant between-group differences at any time point.

The changes in systolic blood pressure (SD) over time were -3.67(9.93) mmHg in the test group and -3.23(8.88) mmHg in the control at week 6, -7.10(9.09) mmHg in the test group and -4.43(9.82) mmHg in the control at week 12, -7.37(9.35) mmHg in the test group and -4.63(10.16) mmHg in the control at week 18, and -5.20(9.81) mmHg in the test group and -3.74(10.73) mmHg in the control at week 24, showing statistically significant between-group differences at all time points and in all administration groups.

**[Table 55]**

| **Change from baseline in blood pressures at weeks 6, 12, 18 and 24 (modified per-protocol set 2) [systolic blood pressure]** | | |
|---|---|---|
| | **Enavogliflozin 0.3 mg (N=114)** | **Dapagliflozin 10 mg (N=121)** |
| **Baseline** | | |
| n | 114 | 121 |
| Mean (SD) | 127.49(12.74) | 123.94(11.74) |
| Median | 127.50 | 123.00 |
| Min, Max | 91.00, 166.00 | 97.00, 153.00 |

| **Week 6** | | |
|---|---|---|
| n | 111 | 120 |
| Mean (SD) | 124.04(13.24) | 120.64(11.35) |
| Median | 123.00 | 119.00 |
| Min, Max | 93.00, 152.00 | 96.00, 153.00 |

| **Change from baseline at week 6** | | |
|---|---|---|
| n | 111 | 120 |
| Mean (SD) | -3.67(9.93) | -3.23(8.88) |
| Median | -3.00 | -3.50 |
| Min, Max | -35.00, 20.00 | -30.00, 20.00 |
| P-value [1] | 0.0002 (t) | 0.0001 (t) |

| **ANCOVA result** | | |
|---|---|---|
| LS mean (SE) | -2.76(0.90) | -3.49(0.87) |
| LS mean difference | 0.73 | |
| 95% confidence interval for difference | [-1.56, 3.02] | |
| P-value [2] | 0.5318 | |

| **Week 12** | | |
|---|---|---|
| n | 112 | 121 |
| Mean (SD) | 120.73(12.13) | 119.51(11.32) |
| Median | 121.00 | 120.00 |
| Min, Max | 92.00, 148.00 | 91.00, 147.00 |

| **Change from baseline at week 12** | | |
|---|---|---|
| n | 112 | 121 |
| Mean (SD) | -7.10(9.09) | -4.43(9.82) |
| Median | -8.00 | -4.00 |
| Min, Max | -31.00, 19.00 | -33.00, 22.00 |
| P-value [1] | <0.0001 (t) | <0.0001 (t) |

| **ANCOVA result** | | |
|---|---|---|
| LS mean (SE) | -6.43(0.88) | -5.06(0.85) |
| LS mean difference | -1.37 | |
| 95% confidence interval for difference | [-3.62, 0.88] | |
| P-value [2] | 0.2306 | |

| **Week 18** | | |
|---|---|---|
| n | 112 | 121 |
| Mean (SD) | 119.95(12.98) | 119.31(10.95) |
| Median | 119.00 | 118.00 |
| Min, Max | 82.00, 153.00 | 91.00, 149.00 |

| **Change from baseline at week 18** | | |
|---|---|---|
| n | 112 | 121 |
| Mean (SD) | -7.37(9.35) | -4.63(10.16) |
| Median | -7.00 | -4.00 |
| Min, Max | -33.00, 19.00 | -34.00, 22.00 |
| P-value [1] | <0.0001 (t) | <0.0001 (t) |

| **ANCOVA result** | | |
|---|---|---|
| LS mean (SE) | -6.82(0.92) | -5.16(0.88) |
| LS mean difference | -1.66 | |
| 95% confidence interval for difference | [-4.00, 0.68] | |
| P-value [2] | 0.1634 | |

| **Week 24** | | |
|---|---|---|
| n | 114 | 121 |
| Mean (SD) | 122.29(12.63) | 120.20(11.96) |
| Median | 121.00 | 119.00 |
| Min, Max | 89.00, 152.00 | 93.00, 157.00 |

| **Change from baseline at week 24** | | |
|---|---|---|
| n | 114 | 121 |
| Mean (SD) | -5.20(9.81) | -3.74(10.73) |
| Median | -5.00 | -4.00 |
| Min, Max | -34.00, 18.00 | -25.00, 53.00 |
| P-value [1] | <0.0001 (t) | <0.0001 (w) |

| **ANCOVA result** | | |
|---|---|---|
| LS mean (SE) | -4.49(0.96) | -4.27(0.93) |
| LS mean difference | -0.22 | |
| 95% confidence interval for difference | [-2.67, 2.23] | |
| P-value [2] | 0.8568 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbAlc level at visit 1 [screening][< 8% or ≥ 8%]) as covariates). Note: Baseline is Visit 2 (randomization). Data source: Listing 16.2.3:1, Listing 16.2.6:1. | | |

### ② Diastolic blood pressure (DBP)

The result of summarizing the changes in diastolic blood pressure from baseline in mPPS2 at weeks 6, 12, 18, and 24 after administration of the clinical trial drug is shown in Table 56.

As a result of analysis of covariance (ANCOVA) on mPPS2 with the baseline value and stratification factors (the conventional regimen for administering a hypoglycemic agent (mono or combination) within 24 weeks prior to obtaining written consent (before the washout period of another hypoglycemic agent), HbAlc level (< 8% or ≥ 8%) at Visit 1 (screening)) as covariates, the least square means (LS means (SE)) for the change in diastolic blood pressure from the baseline at different time points after administration of the clinical trial drug were -2.04(0.70) mmHg in the test group (enavogliflozin, 0.3 mg) and -1.88(0.67) mmHg in the control (dapagliflozin, 10 mg) at week 6, -4.30(0.69) mmHg in the test group and -2.26(0.66) mmHg in the control at week 12, -3.41(0.71) mmHg in the test group and -2.42(0.68) mmHg in the control at week 18, and -2.55(0.72) mmHg in the test group and -1.89(0.70) mmHg in the control at week 24. The between-group differences (LS mean difference/test group-control (95% CI)) were -0.16(-1.94, 1.62) mmHg at week 6, -2.04(-3.81, -0.28) mmHg at week 12, -0.99(-2.80, 0.82) mmHg at week 18, and -0.66(-2.50, 1.19) mmHg at week 24, showing a statistically significant between-group difference only at week 12.

The changes in diastolic blood pressure (SD) over time were -2.76(8.36) mmHg in the test group and -1.57(6.64) mmHg in the control at week 6, -5.07(7.87) mmHg in the test group and -1.88(7.29) mmHg in the control at week 12, -4.03(7.86) mmHg in the test group and -2.12(7.17) mmHg in the control at week 18, and -3.19(7.31) mmHg in the test group and -1.60(7.95) mmHg in the control at week 24, showing statistically significant between-group differences at all time points and in all administration groups.

**[Table 56]**

| **Change from baseline in blood pressures at weeks 6, 12, 18 and 24 (modified per-protocol set 2) [diastolic blood pressure]** | | |
|---|---|---|
| | **Enavogliflozin 0.3 mg(N=114)** | **Dapagliflozin 10 mg(N=121)** |
| **Baseline** | | |
| n | 114 | 121 |
| Mean (SD) | 76.60(9.25) | 73.87(8.58) |
| Median | 76.00 | 74.00 |
| Min, Max | 53.00, 103.00 | 51.00, 99.00 |

| **Week 6** | | |
|---|---|---|
| n | 111 | 120 |
| Mean (SD) | 73.72(9.48) | 72.20(8.45) |
| Median | 74.00 | 72.00 |
| Min, Max | 49.00, 93.00 | 51.00, 93.00 |

| **Change from baseline at week 6** | | |
|---|---|---|
| n | 111 | 120 |
| Mean (SD) | -2.76(8.36) | -1.57(6.64) |
| Median | -3.00 | -2.00 |
| Min, Max | -32.00, 23.00 | -21.00, 16.00 |
| P-value [1] | 0.0007 (t) | 0.0109 (t) |

| **ANCOVA result** | | |
|---|---|---|
| LS mean (SE) | -2.04(0.70) | -1.88(0.67) |
| LS mean difference | -0.16 | |
| 95% confidence interval for difference | [-1.94, 1.62] | |
| P-value [2] | 0.8618 | |

| **Week 12** | | |
|---|---|---|
| n | 112 | 121 |
| Mean (SD) | 71.61(8.54) | 71.99(8.70) |
| Median | 72.00 | 72.00 |
| Min, Max | 49.00, 91.00 | 52.00, 92.00 |

| **Change from baseline at week 12** | | |
|---|---|---|
| n | 112 | 121 |
| Mean (SD) | -5.07(7.87) | -1.88(7.29) |
| Median | -5.00 | -1.00 |
| Min, Max | -36.00, 24.00 | -26.00, 22.00 |
| P-value [1] | <0.0001 (w) | 0.0055 (t) |

| **ANCOVA result** | | |
|---|---|---|
| LS mean (SE) | -4.30(0.69) | -2.26(0.66) |
| LS mean difference | -2.04 | |
| 95% confidence interval for difference | [-3.81, -0.28] | |
| P-value [2] | 0.0234 | |

| **Week 18** | | |
|---|---|---|
| n | 112 | 121 |
| Mean (SD) | 72.53(9.81) | 71.75(8.24) |
| Median | 72.00 | 71.00 |
| Min, Max | 50.00, 98.00 | 52.00, 92.00 |

| **Change from baseline at week 18** | | |
|---|---|---|
| n | 112 | 121 |
| Mean (SD) | -4.03(7.86) | -2.12(7.17) |
| Median | -5.00 | -2.00 |
| Min, Max | -29.00, 20.00 | -21.00, 19.00 |
| P-value [1] | <0.0001 (t) | 0.0015 (t) |

| **ANCOVA result** | | |
|---|---|---|
| LS mean (SE) | -3.41(0.71) | -2.42(0.68) |
| LS mean difference | -0.99 | |
| 95% confidence interval for difference | [-2.80, 0.82] | |
| P-value [2] | 0.2825 | |

| **Week 24** | | |
|---|---|---|
| n | 114 | 121 |
| Mean (SD) | 73.40(9.39) | 72.27(9.11) |
| Median | 73.50 | 73.00 |
| Min, Max | 52.00, 97.00 | 53.00, 107.00 |

| **Change from baseline at week 24** | | |
|---|---|---|
| n | 114 | 121 |
| Mean (SD) | -3.19(7.31) | -1.60(7.95) |
| Median | -3.00 | -2.00 |
| Min, Max | -25.00, 14.00 | -21.00, 28.00 |
| P-value [1] | <0.0001 (t) | 0.0293 (t) |

| **ANCOVA result** | | |
|---|---|---|
| LS mean (SE) | -2.55(0.72) | -1.89(0.70) |
| LS mean difference | -0.66 | |
| 95% confidence interval for difference | [-2.50, 1.19] | |
| P-value [2] | 0.4842 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbA1c level at visit 1 [screening][< 8% or ≥ 8%]) as covariates). Note: Baseline is Visit 2 (randomization). Data source: Listing 16.2.3:1, Listing 16.2.6:1. | | |

### 5) Changes in β-cell function and insulin resistance measured by HOMA-β and HOMA-IR at each time point of weeks 6, 12, 18, and 24 after administration of clinical trial drug compared to Visit 2 (randomization)

### ① β-cell function measured by HOMA-β

The result of summarizing the changes in HOMA-β from baseline in PPS at weeks 6, 12, 18, and 24 after administration of the clinical trial drug is shown in Table 57.

As a result of analysis of covariance (ANCOVA) on PPS with the baseline value and stratification factors (the conventional regimen (co-administration with two or more agents (one or two agents) or three or more agents) for administering a hypoglycemic agent (before the washout period of another hypoglycemic agent) within 24 weeks prior to obtaining written consent, Visit 1 (screening) HbA1c level (< 8% or ≥ 8%)) as covariates, the least square means (LS means (SE)) for the changes in HOMA-β from the baseline at different time points after administration of the clinical trial drug were 8.13(3.08) in the test group (enavogliflozin, 0.3 mg) and 3.20(3.04) in the control (dapagliflozin, 10 mg) at week 6, 7.78(1.94) in the test group and 5.23(1.90) in the control at week 12, 5.97(2.27) in the test group and 4.49(2.22) in the control at week 18, and 1.09(4.79) in the test group and 5.78(4.72) in the control at week 24. The between-group differences (LS mean differences (95% CI)) were 4.93(-2.95, 12.82) at week 6, 2.55(-2.42, 7.51) at week 12, 1.48(-4.31, 7.26) at week 18, and -4.69(-16.95, 7.56) at week 24, showing no statistically significant between-group differences at any time point.

The HOMA-β changes (SD) over time were 8.74(39.40) in the test group and 4.48(21.23) in the control at week 6, 6.71(20.59) in the test group and 4.70(20.22) in the control at week 12, 6.09(25.33) in the test group and 5.32(21.88) in the control at week 18, and 2.57(63.77) in the test group and 7.95(26.82) in the control at week 24, showing statistically significant between-group differences at all time points and in all administration groups.

**[Table 57]**

| **Change from baseline in beta-cell function at weeks 6, 12, 18 and 24 (per-protocol set) [HOMA-β]** | | |
|---|---|---|
| | **Enavogliflozin 0.3 mg (N=119)** | **Dapagliflozin 10 mg (N=123)** |
| **Baseline** | | |
| n | 119 | 123 |
| Mean (SD) | 42.91(27.09) | 40.47(27.30) |
| Median | 37.50 | 33.30 |
| Min, Max | 6.10, 141.80 | 6.90, 204.90 |

| **Week 6** | | |
|---|---|---|
| n | 116 | 122 |
| Mean (SD) | 51.08(43.59) | 44.56(29.07) |
| Median | 38.70 | 36.75 |
| Min, Max | 7.80, 360.00 | -72.00, 164.00 |

| **Change from baseline at week 6** | | |
|---|---|---|
| n | 116 | 122 |
| Mean (SD) | 8.74(39.40) | 4.48(21.23) |
| Median | 4.35 | 5.30 |
| Min, Max | -87.50, 321.40 | -99.80, 75.60 |
| P-value [1] | 0.0097 (w) | 0.0024 (w) |

| **ANCOVA result** | | |
|---|---|---|
| LS mean (SE) | 8.13(3.08) | 3.20(3.04) |
| LS mean difference | 4.93 | |
| 95% confidence interval for difference | [-2.95, 12.82] | |
| P-value [2] | 0.2191 | |

| **Week 12** | | |
|---|---|---|
| n | 117 | 123 |
| Mean (SD) | 49.46(30.06) | 45.17(27.17) |
| Median | 41.50 | 38.20 |
| Min, Max | 5.70, 180.00 | 9.90, 166.80 |

| **Change from baseline at week 12** | | |
|---|---|---|
| n | 117 | 123 |
| Mean (SD) | 6.71(20.59) | 4.70(20.22) |
| Median | 4.80 | 3.50 |
| Min, Max | -43.30, 82.40 | -80.40, 62.00 |
| P-value [1] | 0.0009 (w) | 0.0057 (w) |

| **ANCOVA result** | | |
|---|---|---|
| LS mean (SE) | 7.78(1.94) | 5.23(1.90) |
| LS mean difference | 2.55 | |
| 95% confidence interval for difference | [-2.42,7.51] | |
| P-value [2] | 0.3136 | |

| **Week 18** | | |
|---|---|---|
| n | 117 | 123 |
| Mean (SD) | 49.26(29.93) | 45.79(30.81) |
| Median | 40.00 | 36.80 |
| Min, Max | 9.70, 174.60 | -12.00, 226.70 |

| **Change from baseline at week 18** | | |
|---|---|---|
| n | 117 | 123 |
| Mean (SD) | 6.09(25.33) | 5.32(21.88) |
| Median | 4.10 | 2.20 |
| Min, Max | -88.40, 97.40 | -113.00, 65.80 |
| P-value [1] | 0.0040 (w) | 0.0067 (w) |

| **ANCOVA result** | | |
|---|---|---|
| LS mean (SE) | 5.97(2.27) | 4.49(2.22) |
| LS mean difference | 1.48 | |
| 95% confidence interval for difference | [-4.31, 7.26] | |
| P-value [2] | 0.6155 | |

| **Week 24** | | |
|---|---|---|
| n | 119 | 123 |
| Mean (SD) | 45.48(67.01) | 48.41(31.90) |
| Median | 41.40 | 36.70 |
| Min, Max | -576.00, 295.20 | 7.50, 159.20 |

| **Change from baseline at week 24** | | |
|---|---|---|
| n | 119 | 123 |
| Mean (SD) | 2.57(63.77) | 7.95(26.82) |
| Median | 5.70 | 7.20 |
| Min, Max | -607.10, 252.70 | -113.10, 93.40 |
| P-value [1] | 0.0002 (w) | 0.0003 (w) |

| **ANCOVA result** | | |
|---|---|---|
| LS mean (SE) | 1.09(4.79) | 5.78(4.72) |
| LS mean difference | -4.69 | |
| 95% confidence interval for difference | [-16.95, 7.56] | |
| P-value [2] | 0.4513 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbA1c level at visit 1 [screening][< 8% or ≥ 8%]) as covariates). Note: Baseline is Visit 2 (randomization). Data source: Listing 16.2.3:1, Listing 16.2.6:1. | | |

### ② Insulin resistance detected by HOMA-IR

The result of summarizing the change in HOMA-IR from baseline in PPS at weeks 6, 12, 18, and 24 after administration of the clinical trial drug is presented in Table 58.

As a result of analysis of covariance (ANCOVA) on PPS with the baseline value and stratification factors (the conventional regimen (co-administration with two or more agents (one or two agents) or three or more agents) for administering a hypoglycemic agent (before the washout period of another hypoglycemic agent) within 24 weeks prior to obtaining written consent, Visit 1 (screening) HbA1c level (< 8% or ≥ 8%)) as covariates, the least square means (LS means (SE)) for the changes in HOMA-IR from the baseline at different time points after administration of the clinical trial drug were -0.94(0.15) in the test group (enavogliflozin, 0.3 mg) and -0.99(0.14) in the control (dapagliflozin, 10 mg) at week 6, -1.11(0.10) in the test group and -1.05(0.10) in the test group at week 12, -1.14(0.11) in the test group and -1.01(0.11) in the control at week 18, and -1.09(0.11) in the test group and -0.93(0.11) in the control at week 24. The between-group differences (LS mean differences (95% CI)) were 0.05(-0.32, 0.42) at week 6, -0.06(-0.33, 0.20) at week 12, -0.13(-0.41, 0.15) at week 18, and -0.16(-0.44, 0.13) at week 24, showing no statistically significant between-group differences at any time point.

The HOMA-IR changes (SD) over time were -0.90(1.94) in the test group and -0.92(1.49) in the control at week 6, -1.16(1.54) in the test group and -1.06(1.70) in the control at week 12, -1.16(1.71) in the test group and -1.00(1.58) in the control at week 18, and -1.07(1.52) in the test group and -0.88(1.72) in the control at week 24, showing statistically significant between-group differences at all time points and in all administration groups.

**[Table 58]**

| **Change from baseline in insulin resistance at weeks 6, 12, 18 and 24 (per-protocol set) [HOMA-IR]** | | |
|---|---|---|
| | **Enavogliflozin 0.3 mg (N=119)** | **Dapagliflozin 10 mg (N=123)** |
| **Baseline** | | |
| n | 119 | 123 |
| Mean (SD) | 2.90(1.91) | 2.86(1.84) |
| Median | 2.42 | 2.38 |
| Min, Max | 0.45, 9.40 | 0.21, 10.08 |

| **Week 6** | | |
|---|---|---|
| n | 116 | 122 |
| Mean (SD) | 2.00(2.26) | 1.90(1.05) |
| Median | 1.54 | 1.66 |
| Min, Max | 0.27,21.81 | 0.14, 5.47 |

| **Change from baseline at week 6** | | |
|---|---|---|
| n | 116 | 122 |
| Mean (SD) | -0.90(1.94) | -0.92(1.49) |
| Median | -0.68 | -0.72 |
| Min, Max | -5.66, 13.12 | -7.71, 3.03 |
| P-value [1] | <0.0001 (w) | <0.0001 (w) |

| **ANCOVA result** | | |
|---|---|---|
| LS mean (SE) | -0.94(0.15) | -0.99(0.14) |
| LS mean difference | 0.05 | |
| 95% confidence interval for difference | [-0.32, 0.42] | |
| P-value [2] | 0.7912 | |

| **Week 12** | | |
|---|---|---|
| n | 117 | 123 |
| Mean (SD) | 1.76(1.09) | 1.80(1.32) |
| Median | 1.46 | 1.55 |
| Min, Max | 0.16,5.17 | 0.22,9.87 |

| **Change from baseline at week 12** | | |
|---|---|---|
| n | 117 | 123 |
| Mean (SD) | -1.16(1.54) | -1.06(1.70) |
| Median | -0.79 | -0.76 |
| Min, Max | -6.94, 1.54 | -7.81, 4.83 |
| P-value [1] | <0.0001 (w) | <0.0001 (w) |

| **ANCOVA result** | | |
|---|---|---|
| LS mean (SE) | -1.11(0.10) | -1.05(0.10) |
| LS mean difference | -0.06 | |
| 95% confidence interval for difference | [-0.33, 0.20] | |
| P-value [2] | 0.6426 | |

| **Week 18** | | |
|---|---|---|
| n | 117 | 123 |
| Mean (SD) | 1.74(1.23) | 1.85(1.34) |
| Median | 1.42 | 1.53 |
| Min, Max | 0.36, 8.84 | 0.06, 7.92 |

| **Change from baseline at week 18** | | |
|---|---|---|
| n | 117 | 123 |
| Mean (SD) | -1.16(1.71) | -1.00(1.58) |
| Median | -0.82 | -0.75 |
| Min, Max | -7.20, 6.25 | -7.26, 2.88 |
| P-value [1] | <0.0001 (w) | <0.0001 (w) |

| **ANCOVA result** | | |
|---|---|---|
| LS mean (SE) | -1.14(0.11) | -1.01(0.11) |
| LS mean difference | -0.13 | |
| 95% confidence interval for difference | [-0.41, 0.15] | |
| P-value [2] | 0.3553 | |

| **Week 24** | | |
|---|---|---|
| n | 119 | 123 |
| Mean (SD) | 1.84(1.10) | 1.97(1.49) |
| Median | 1.56 | 1.55 |
| Min, Max | 0.36,5.35 | 0.11, 8.65 |

| **Change from baseline at week 24** | | |
|---|---|---|
| n | 119 | 123 |
| Mean (SD) | -1.07(1.52) | -0.88(1.72) |
| Median | -0.80 | -0.70 |
| Min, Max | -7.03, 2.76 | -6.59, 3.94 |
| P-value [1] | <0.0001 (w) | <0.0001 (w) |

| **ANCOVA result** | | |
|---|---|---|
| LS mean (SE) | -1.09(0.11) | -0.93(0.11) |
| LS mean difference | -0.16 | |
| 95% confidence interval for difference | [-0.44, 0.13] | |
| P-value [2] | 0.2767 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbA1c level at visit 1 [screening][< 8% or ≥8%]) as covariates). Note: Baseline is Visit 2 (randomization). Data source: Listing 16.2.3:1, Listing 16.2.6:1. | | |

### 6) Changes in renal function-related indicators (UACR, UGCR) at each time point of weeks 6, 12, 18, and 24 after administration of clinical trial drug compared to Visit 2 (randomization)

### ① UACR

The result of summarizing UACR changes from baseline in PPS at weeks 6, 12, 18, and 24 after administration of the clinical trial drug is shown in Table 59.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbA1c level (< 8% or ≥ 8%) at Visit 1 (screening)) as covariates, the least square means (LS means (SE)) for the UACR changes from the baseline at different time points after administration of the clinical trial drug were -8.76(3.43) g/kg in the test group (enavogliflozin, 0.3 mg) and -14.85(3.36) g/kg in the control (dapagliflozin, 10 mg) at week 6, -15.12(4.60) g/kg in the test group and -11.76(4.47) g/kg in the control at week 12, -11.08(3.89) g/kg in the test group and -8.90(3.79) g/kg in the control at week 18, and -15.34(3.93) g/kg in the test group and -16.13(3.84) g/kg in the control at week 24. The between-group differences (LS mean difference/test group-control (95% CI)) were 6.09(-2.68, 14.87) g/kg at week 6, -3.35(-15.10, 8.39) g/kg at week 12, -2.18(-12.10, 7.73) g/kg at week 18, and 0.79(-9.26, 10.84) g/kg at week 24, showing no statistically significant between-group differences at any time point.

The UACR changes (SD) over time were -11.43(89.49) g/kg in the test group and -7.25(52.04) g/kg in the control at week 6, -18.62(103.10) g/kg in the test group and -7.42(53.04) g/kg in the control at week 12, -12.07(46.16) g/kg in the test group and -8.84(63.74) g/kg in the control at week 18, and -19.52(108.55) g/kg in the test group and -11.12(70.82) g/kg in the control at week 24, showing statistically significant between-group differences in the test groups at weeks 12, 18, and 24.

**[Table 59]**

| **Change from baseline in renal function parameters at weeks 6, 12, 18 and 24 (per-protocol set) [UACR]** | | |
|---|---|---|
| | **Enavogliflozin 0.3 mg (N=119)** | **Dapagliflozin 10 mg (N=123)** |
| **Baseline** | | |
| n | 119 | 123 |
| Mean (SD) | 47.15(129.16) | 34.70(97.22) |
| Median | 12.30 | 10.10 |
| Min, Max | 2.50, 1262.90 | 1.50, 816.40 |

| **Week 6** | | |
|---|---|---|
| n | 116 | 122 |
| Mean (SD) | 36.67(71.37) | 23.20(39.05) |
| Median | 14.80 | 11.20 |
| Min, Max | 1.80, 450.90 | 2.00, 328.70 |

| **Change from baseline at week 6** | | |
|---|---|---|
| n | 116 | 122 |
| Mean (SD) | -11.43(89.49) | -7.25(52.04) |
| Median | -0.50 | -0.40 |
| Min, Max | -812.00, 309.10 | -487.70, 78.50 |
| P-value [1] | 0.3557 (w) | 0.6266 (w) |

| **ANCOVA result** | | |
|---|---|---|
| LS mean (SE) | -8.76(3.43) | -14.85(3.36) |
| LS mean difference | 6.09 | |
| 95% confidence interval for difference | [-2.68, 14.87] | |
| P-value [2] | 0.1726 | |

| **Week 12** | | |
|---|---|---|
| n | 116 | 123 |
| Mean (SD) | 29.37(67.19) | 27.28(64.60) |
| Median | 11.20 | 11.80 |
| Min, Max | 1.20, 501.70 | 2.60, 567.10 |

| **Change from baseline at week 12** | | |
|---|---|---|
| n | 116 | 123 |
| Mean (SD) | -18.62(103.10) | -7.42(53.04) |
| Median | -1.05 | 0.20 |
| Min, Max | -978.90, 360.80 | -536.90, 87.80 |
| P-value [1] | 0.0017 (w) | 0.7070 (w) |

| **ANCOVA result** | | |
|---|---|---|
| LS mean (SE) | -15.12(4.60) | -11.76(4.47) |
| LS mean difference | -3.35 | |
| 95% confidence interval for difference | [-15.10, 8.39] | |
| P-value [2] | 0.5740 | |

| **Week 18** | | |
|---|---|---|
| n | 117 | 123 |
| Mean (SD) | 25.02(50.17) | 25.86(63.91) |
| Median | 11.30 | 10.20 |
| Min, Max | 2.10, 460.70 | 2.70, 640.00 |

| **Change from baseline at week 18** | | |
|---|---|---|
| n | 117 | 123 |
| Mean (SD) | -12.07(46.16) | -8.84(63.74) |
| Median | -1.50 | 0.20 |
| Min, Max | -248.10, 118.00 | -658.10, 87.50 |
| P-value [1] | 0.0062 (w) | 0.3524 (w) |

| **ANCOVA result** | | |
|---|---|---|
| LS mean (SE) | -11.08(3.89) | -8.90(3.79) |
| LS mean difference | -2.18 | |
| 95% confidence interval for difference | [-12.10, 7.73] | |
| P-value [2] | 0.6651 | |

| **Week 24** | | |
|---|---|---|
| n | 118 | 123 |
| Mean (SD) | 27.90(53.69) | 23.58(47.43) |
| Median | 11.10 | 10.20 |
| Min, Max | 2.00, 432.40 | 2.20, 416.80 |

| **Change from baseline at week 24** | | |
|---|---|---|
| n | 118 | 123 |
| Mean (SD) | -19.52(108.55) | -11.12(70.82) |
| Median | -1.55 | -0.70 |
| Min, Max | -1116.40, 132.30 | -695.50, 168.30 |
| P-value [1] | 0.0008 (w) | 0.0538 (w) |

| **ANCOVA result** | | |
|---|---|---|
| LS mean (SE) | -15.34(3.93) | -16.13(3.84) |
| LS mean difference | J.79 | |
| 95% confidence interval for difference | [-9.26, 10.84] | |
| P-value [2] | 0.8774 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbA1c level at visit 1 [screening][< 8% or ≥ 8%]) as covariates). Note: Baseline is Visit 2 (randomization). Data source: Listing 16.2.3:1, Listing 16.2.6:1. | | |

### ② UGCR

The result of summarizing UGCR changes from baseline in PPS at weeks 6, 12, 18, and 24 after administration of the clinical trial drug is shown in Table 60 .

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbA1c level (< 8% or ≥ 8%) at Visit 1 (screening)) as covariates, the least square means (LS means (SE)) for the UGCR changes from the baseline at different time points after administration of the clinical trial drug were 58.10(2.18) mg/mg in the test group (enavogliflozin, 0.3 mg) and 45.75(2.14) mg/mg in the control (dapagliflozin, 10 mg) at week 6, 58.21(2.07) mg/mg in the test group and 44.84(2.02) mg/mg in the control at week 12, 58.74(2.33) mg/mg in the test group and 45.40(2.27) mg/mg in the control at week 18, and 60.22(2.31) mg/mg in the test group and 43.51(2.26) mg/mg in the control at week 24. The between-group differences (LS mean difference/test group-control (95% CI)) were 12.35(6.76, 17.94) mg/mg at week 6, 13.37(8.06, 18.67) mg/mg at week 12, 13.34(7.40, 19.27) mg/mg at week 18, and 16.71(10.79, 22.63) mg/mg at week 24, showing statistically significant between-group differences at all time points.

The UGCR changes (SD) over time were 56.38(24.08) mg/mg in the test group and 43.58(21.16) mg/mg in the control at week 6, 57.00(22.19) mg/mg in the test group and 43.19(20.18) mg/mg in the control at week 12, 57.23(24.63) mg/mg in the test group and 43.29(23.42) mg/mg in the control at week 18, and 59.39(24.52) mg/mg in the test group and 42.31(22.51) mg/mg in the control at week 24, showing statistically significant between-group differences at all time points and in all administration groups.

**[Table 60]**

| **Change from baseline in renal function parameters at weeks 6, 12, 18 and 24 (per-protocol set) [UGCR]** | | |
|---|---|---|
| | **Enavogliflozin 0.3 mg (N=119)** | **Dapagliflozin 10 mg (N=123)** |
| **Baseline** | | |
| n | 119 | 123 |
| Mean (SD) | 0.72(3.49) | 1.53(7.42) |
| Median | 0.12 | 0.12 |
| Min, Max | 0.00, 37.51 | 0.01, 75.38 |

| **Week 6** | | |
|---|---|---|
| n | 116 | 122 |
| Mean (SD) | 57.09(23.82) | 45.12(20.73) |
| Median | 54.57 | 43.37 |
| Min, Max | 0.06, 122.83 | 0.62, 137.45 |

| **Change from baseline at week 6** | | |
|---|---|---|
| n | 116 | 122 |
| Mean (SD) | 56.38(24.08) | 43.58(21.16) |
| Median | 54.10 | 42.62 |
| Min, Max | -0.14, 122.64 | -20.53, 135.53 |
| P-value [1] | <0.0001 (t) | <0.0001 (w) |

| **ANCOVA result** | | |
|---|---|---|
| LS mean (SE) | 58.10(2.18) | 45.75(2.14) |
| LS mean difference | 12.35 | |
| 95% confidence interval for difference | [6.76, 17.94] | |
| P-value [2] | <0.0001 | |

| **Week 12** | | |
|---|---|---|
| n | 116 | 123 |
| Mean (SD) | 57.71(21.94) | 44.72(19.93) |
| Median | 57.43 | 41.80 |
| Min, Max | 6.96, 123.90 | 0.07, 129.39 |

| **Change from baseline at week 12** | | |
|---|---|---|
| n | 116 | 123 |
| Mean (SD) | 57.00(22.19) | 43.19(20.18) |
| Median | 57.05 | 41.69 |
| Min, Max | 6.84, 122.30 | -12.39, 124.69 |
| P-value [1] | <0.0001 (t) | <0.0001 (w) |

| **ANCOVA result** | | |
|---|---|---|
| LS mean (SE) | 58.21(2.07) | 44.84(2.02) |
| LS mean difference | 13.37 | |
| 95% confidence interval for difference | [8.06, 18.67] | |
| P-value [2] | <0.0001 | |

| **Week 18** | | |
|---|---|---|
| n | 117 | 123 |
| Mean (SD) | 57.96(24.33) | 44.82(22.53) |
| Median | 54.71 | 42.32 |

| Min, Max | 0.05, 120.36 | 0.11, 120.52 |
|---|---|---|
| **Change from baseline at week 18** | | |
| n | 117 | 123 |
| Mean (SD) | 57.23(24.63) | 43.29(23.42) |
| Median | 54.56 | 41.28 |
| Min, Max | -0.08, 120.26 | -24.44, 120.27 |
| P-value [1] | <0.0001 (t) | <0.0001 (t) |

| **ANCOVA result** | | |
|---|---|---|
| LS mean (SE) | 58.74(2.33) | 45.40(2.27) |
| LS mean difference | 13.34 | |
| 95% confidence interval for difference | [7.40, 19.27] | |
| P-value [2] | <0.0001 | |

| **Week 24** | | |
|---|---|---|
| n | 118 | 123 |
| Mean (SD) | 60.12(24.59) | 43.84(22.15) |
| Median | 57.96 | 42.85 |
| Min, Max | 11.30, 124.74 | 0.07, 134.82 |

| **Change from baseline at week 24** | | |
|---|---|---|
| n | 118 | 123 |
| Mean (SD) | 59.39(24.52) | 42.31(22.51) |
| Median | 55.98 | 42.35 |
| Min, Max | 11.23, 124.33 | -13.64, 130.12 |
| P-value [1] | <0.0001 (w) | <0.0001 (t) |

| **ANCOVA result** | | |
|---|---|---|
| LS mean (SE) | 60.22(2.31) | 43.51(2.26) |
| LS mean difference | 16.71 | |
| 95% confidence interval for difference | [10.79, 22.63] | |
| P-value [2] | <0.0001 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbA1c level at visit 1 [screening][< 8% or ≥ 8%]) as covariates). Note: Baseline is Visit 2 (randomization). Data source: Listing 16.2.3:1, Listing 16.2.6:1. | | |

### 7) Changes in obesity-related indicators (adiponectin, leptin) at each time point of weeks 6, 12, 18, and 24 after administration of clinical trial drug compared to Visit 2 (randomization)

### ① Adiponectin

The result of summarizing adiponectin changes from baseline in PPS at weeks 6, 12, 18, and 24 after administration of the clinical trial drug is shown in Table 61.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbA1c level (< 8% or ≥ 8%) at Visit 1 (screening)) as covariates, the least square means (LS means (SE)) for the adiponectin changes from the baseline at different time points after administration of the clinical trial drug were -0.10(0.34) µg/mL in the test group (enavogliflozin, 0.3 mg) and 0.37(0.33) µg/mL in the control (dapagliflozin, 10 mg) at week 6, 0.89(0.34) µg/mL in the test group and 0.70(0.33) µg/mL in the control at week 12, 0.78(0.33) µg/mL in the test group and 0.36(0.32) µg/mL in the control at week 18, and 1.42(0.35) µg/mL in the test group and 0.92(0.35) µg/mL in the control at week 24, and the between-group differences (LS mean difference (95% CI)) were -0.47(-1.33, 0.39) µg/mL at week 6, 0.19(-0.68, 1.06) µg/mL at week 12, 0.42(-0.41, 1.25) µg/mL at week 18, and 0.49(-0.42, 1.40) µg/mL at week 24, showing no statistically significant between-group differences at any time point.

The adiponectin changes (SD) over time were -0.08(4.03) µg/mL in the test group and 0.31(4.15) µg/mL in the control at week 6, 0.88(4.15) µg/mL in the test group and 0.63(3.45) µg/mL in the control at week 12, 0.80(4.50) µg/mL in the test group and 0.30(3.21) µg/mL in the control at week 18, and 1.39(4.63) µg/mL in the test group and 0.84(3.23) µg/mL in the control at week 24, showing statistically significant between-group differences in all administration time at all time points except week 6.

**[Table 61]**

| **Change from baseline in obesity parameters at weeks 6, 12, 18 and 24 (per-protocol set) [adiponectin]** | | |
|---|---|---|
| | **Enavogliflozin 0.3 mg (N=119)** | **Dapagliflozin 10 mg (N=123)** |
| **Baseline** | | |
| n | 119 | 123 |
| Mean (SD) | 6.22(4.07) | 6.34(3.42) |
| Median | 5.01 | 5.61 |
| Min, Max | 1.00, 24.78 | 1.35, 23.79 |

| **Week 6** | | |
|---|---|---|
| n | 116 | 122 |
| Mean (SD) | 6.20(3.39) | 6.69(3.86) |
| Median | 5.40 | 5.80 |
| Min, Max | 1.00, 17.27 | 1.58, 29.02 |

| **Change from baseline at week 6** | | |
|---|---|---|
| n | 116 | 122 |
| Mean (SD) | -0.08(4.03) | 0.31(4.15) |
| Median | 0.50 | 0.34 |
| Min, Max | -21.27, 8.20 | -16.13, 23.71 |
| P-value [1] | 0.0976 (w) | 0.0862 (w) |

| **ANCOVA result** | | |
|---|---|---|
| LS mean (SE) | -0.10(0.34) | 0.37(0.33) |
| LS mean difference | -0.47 | |
| 95% confidence interval for difference | [-1.33, 0.39] | |
| P-value [2] | 0.2844 | |

| **Week 12** | | |
|---|---|---|
| n | 117 | 123 |
| Mean (SD) | 7.13(4.38) | 6.97(3.60) |
| Median | 6.24 | 6.27 |
| Min, Max | 1.00, 23.70 | 1.22, 23.79 |

| **Change from baseline at week 12** | | |
|---|---|---|
| n | 117 | 123 |
| Mean (SD) | 0.88(4.15) | 0.63(3.45) |
| Median | 1.00 | 0.43 |
| Min, Max | -21.16, 15.47 | -18.49, 9.53 |
| P-value [1] | 0.0008 (w) | 0.0062 (w) |

| **ANCOVA result** | | |
|---|---|---|
| LS mean (SE) | 0.89(0.34) | 0.70(0.33) |
| LS mean difference | 0.19 | |
| 95% confidence interval for difference | [-0.68, 1.06] | |
| P-value [2] | 0.6708 | |

| **Week 18** | | |
|---|---|---|
| n | 117 | 122 |
| Mean (SD) | 7.03(4.24) | 6.65(2.95) |
| Median | 6.26 | 6.21 |
| Min, Max | 1.00, 24.75 | 1.22, 14.77 |

| **Change from baseline at week 18** | | |
|---|---|---|
| n | 117 | 122 |
| Mean (SD) | 0.80(4.50) | 0.30(3.21) |
| Median | 0.60 | 0.36 |
| Min, Max | -21.28, 19.86 | -18.82, 7.34 |
| P-value [1] | 0.0019 (w) | 0.0147 (w) |

| **ANCOVA result** | | |
|---|---|---|
| LS mean (SE) | 0.78(0.33) | 0.36(0.32) |
| LS mean difference | 0.42 | |
| 95% confidence interval for difference | [-0.41, 1.25] | |
| P-value [2] | 0.3223 | |

| **Week 24** | | |
|---|---|---|
| n | 119 | 123 |
| Mean (SD) | 7.62(4.55) | 7.18(3.53) |
| Median | 6.49 | 6.39 |
| Min, Max | 1.00, 27.76 | 1.34, 17.81 |

| **Change from baseline at week 24** | | |
|---|---|---|
| n | 119 | 123 |
| Mean (SD) | 1.39(4.63) | 0.84(3.23) |
| Median | 1.11 | 0.78 |
| Min, Max | -20.88, 23.22 | -18.39, 9.90 |
| P-value [1] | <0.0001 (w) | <0.0001 (w) |

| **ANCOVA result** | | |
|---|---|---|
| LS mean (SE) | 1.42(0.35) | 0.92(0.35) |
| LS mean difference | 0.49 | |
| 95% confidence interval for difference | [-0.42, 1.40] | |
| P-value [2] | 0.2868 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbA1c level at visit 1 [screening][< 8% or ≥ 8%]) as covariates). Note: Baseline is Visit 2 (randomization). Data source: Listing 16.2.3:1, Listing 16.2.6:1. | | |

### ② Leptin

The result of summarizing leptin changes from baseline in PPS at weeks 6, 12, 18, and 24 after administration of the clinical trial drug is shown in Table 62.

As a result of analysis of covariance (ANCOVA) on PPS with the baseline value and stratification factors (the conventional regimen for administering a hypoglycemic agent (mono or combination) within 24 weeks prior to obtaining written consent (before the washout period of any hypoglycemic agent other than metformin), HbA1c level (< 8% or ≥ 8%) at Visit 1 (screening)) as covariates, the least square means (LS means (SE)) for the leptin changes from the baseline at different time points after administration of the clinical trial drug were -1.83(0.45) ng/mL in the test group (enavogliflozin, 0.3 mg) and -2.03(0.45) ng/mL in the control (dapagliflozin, 10 mg)at week 6, -1.97(0.48) ng/mL in the test group and -1.69(0.47) ng/mL in the control at week 12, -1.46(0.52) ng/mL in the test group and -1.06(0.51) ng/mL in the control at week 18, and -1.46(0.52) ng/mL in the test group and -0.57(0.51) ng/mL in the control at week 24. The between-group differences (LS mean difference (95% CI)) were 0.20(-0.96, 1.36) ng/mL at week 6, -0.28(-1.51, 0.94) ng/mL at week 12, -0.40(-1.74, 0.94) ng/mL at week 18, and -0.89(-2.21, 0.43) ng/mL at week 24, showing no statistically significant between-group differences at any time point.

The leptin changes (SD) over time were -1.75(5.38) ng/mL in the test group and -2.04(6.20) ng/mL in the control at week 6, -1.96(5.27) ng/mL in the test group and -1.82(6.56) ng/mL in the control at week 12, -1.63(5.66) ng/mL in the test group and -1.36(6.68) ng/mL in the control at week 18, and -1.52(4.96) ng/mL in the test group and -0.74(6.26) ng/mL in the control at week 24, showing statistically significant between-group differences at all time points and in all administration groups except the control at week 24.

**[Table 62]**

| **Change from baseline in obesity parameters at weeks 6, 12, 18 and 24 (per-protocol set) [leptin]** | | |
|---|---|---|
| | **Enavogliflozin 0.3 mg (N=119)** | **Dapagliflozin 10 mg (N=123)** |
| **Baseline** | | |
| n | 119 | 123 |
| Mean (SD) | 10.48(10.49) | 10.85(9.19) |
| Median | 6.58 | 8.52 |
| Min, Max | 0.68, 55.00 | 0.46, 55.99 |

| **Week 6** | | |
|---|---|---|
| n | 116 | 122 |
| Mean (SD) | 8.88(8.22) | 8.78(7.23) |
| Median | 6.26 | 7.03 |
| Min, Max | 0.29, 36.83 | 0.25, 51.59 |

| **Change from baseline at week 6** | | |
|---|---|---|
| n | 116 | 122 |
| Mean (SD) | -1.75(5.38) | -2.04(6.20) |
| Median | -0.94 | -1.26 |
| Min, Max | -29.17, 18.32 | -36.30, 23.71 |
| P-value [1] | <0.0001 (w) | <0.0001 (w) |

| **ANCOVA result** | | |
|---|---|---|
| LS mean (SE) | -1.83(0.45) | -2.03(0.45) |
| LS mean difference | 0.20 | |
| 95% confidence interval for difference | [-0.96, 1.36] | |
| P-value [2] | 0.7371 | |

| **Week 12** | | |
|---|---|---|
| n | 117 | 123 |
| Mean (SD) | 8.56(8.44) | 9.03(7.42) |
| Median | 5.74 | 7.69 |
| Min, Max | 0.20, 42.32 | 0.42, 56.24 |

| **Change from baseline at week 12** | | |
|---|---|---|
| n | 117 | 123 |
| Mean (SD) | -1.96(5.27) | -1.82(6.56) |
| Median | -1.28 | -0.96 |
| Min, Max | -33.77, 10.57 | -40.67, 21.07 |
| P-value [1] | <0.0001 (w) | <0.0001 (w) |

| **ANCOVA result** | | |
|---|---|---|
| LS mean (SE) | -1.97(0.48) | -1.69(0.47) |
| LS mean difference | -0.28 | |
| 95% confidence interval for difference | [-1.51, 0.94] | |
| P-value [2] | 0.6525 | |

| **Week 18** | | |
|---|---|---|
| n | 117 | 122 |
| Mean (SD) | 8.87(8.98) | 9.52(7.77) |
| Median | 5.86 | 7.80 |
| Min, Max | 0.20, 46.50 | 0.20, 63.92 |

| **Change from baseline at week 18** | | |
|---|---|---|
| n | 117 | 122 |
| Mean (SD) | -1.63(5.66) | -1.36(6.68) |
| Median | -0.89 | -0.75 |
| Min, Max | -35.90, 14.75 | -40.40, 19.16 |
| P-value [1] | <0.0001 (w) | 0.0066 (w) |

| **ANCOVA result** | | |
|---|---|---|
| LS mean (SE) | -1.46(0.52) | -1.06(0.51) |
| LS mean difference | -0.40 | |
| 95% confidence interval for difference | [-1.74, 0.94] | |
| P-value [2] | 0.5560 | |

| **Week 24** | | |
|---|---|---|
| n | 119 | 123 |
| Mean (SD) | 8.95(9.07) | 10.10(9.33) |
| Median | 6.03 | 8.14 |
| Min, Max | 0.39, 52.43 | 0.20, 78.57 |

| **Change from baseline at week 24** | | |
|---|---|---|
| n | 119 | 123 |
| Mean (SD) | -1.52(4.96) | -0.74(6.26) |
| Median | -0.91 | -0.46 |
| Min, Max | -28.49, 20.68 | -40.76, 22.58 |
| P-value [1] | <0.0001 (w) | 0.0621 (w) |

| **ANCOVA result** | | |
|---|---|---|
| LS mean (SE) | -1.46(0.52) | -0.57(0.51) |
| LS mean difference | -0.89 | |
| 95% confidence interval for difference | [-2.21, 0.43] | |
| P-value [2] | 0.1845 | |

| | | |
|---|---|---|
| SD = standard deviation, Min = minimum, Max = maximum, ANCOVA = analysis of covariance, LS Mean = least squares mean, SE = standard error. [1] Testing for within-treatment group (paired t-test (t) or Wilcoxon signed rank test (w)). [2] Testing for difference between DWP16001 and dapagliflozin (ANCOVA with treatment group as a factor, baseline value and stratification factors (regimen ['Doublet and/or less combination' or 'Triplet and/or more combination'] and HbA1c level at visit 1 [screening][< 8% or ≥ 8%]) as covariates). Note: Baseline is Visit 2 (randomization). Data source: Listing 16.2.3:1, Listing 16.2.6:1. | | |

### 8) Proportion of subjects who received rescue drug at each time point of weeks 6, 12, 18, and 24 after administration of the clinical trial drug

In the cases where a rescue drug was administered, an HbA1c value measured after the start of the administration of the rescue drug was processed by data imputation according to Article 12.2 of the statistical analysis plan (SAP) of this study. Since the HbA1c value at week 24 was excluded from the PPS analysis group by data imputation, no subjects were administered the rescue drug at any time point in the PPS result.

In the FAS results, the proportion of subjects who received a rescue drug after administration of the clinical trial drug was 0.74% (1/135) in the control (dapagliflozin, 10 mg) at week 24. The subjects who received the rescue drug (27S036) started administration of the rescue drug after the 18-week visit and continued to take the drug until the end of the clinical trial. At other time points, there were no such cases. As a result of logistic regression corrected with stratification factors (the conventional regimen for administering a hypoglycemic agent (mono or combination) within 24 weeks prior to obtaining written consent (before the washout period of another hypoglycemic agent), HbA1c level (< 8% or ≥ 8%) at Visit 1 (screening)), compared to the control, in the test group (enavogliflozin, 0.3 mg), the odds ratio (95% CI) for administering the rescue drug at week 24* was 0.36(0.02, 5.14), showing no statistically significant between-group difference (p=0.4497)
(*When the rescue drug was administered from week 18 to the day before week 24, it is presented at week 24.)

### 3. Conclusion

This clinical trial was conducted to demonstrate the non-inferiority of the therapeutic effect of enavogliflozin compared to dapagliflozin in patients with type 2 diabetes whose glycemic control was inadequate with metformin and gemigliptin, and to evaluate safety.

As a result of efficacy evaluation, the between-group difference (LS mean difference (95% CI)) in the change in HbA1c from the baseline, which is the primary efficacy endpoint, at week 24 after administration of the clinical trial drug was -0.06(-0.19, 0.06)%p, the upper limit of the 95% confidence interval for [test group-control] was 0.06%p, which is smaller than the non-inferiority margin of 0.35, proving that the test group was non-inferior to the control. In addition, most secondary and exploratory endpoints, including FPG, showed similar results between the test group and control.

In the result of safety evaluation, there were no statistically significant differences in the incidence of adverse events or adverse drug reactions, or between adverse events and adverse drug reactions, which require careful monitoring, and serious adverse events, and there were no serious adverse drug reactions, adverse events or adverse drug reactions leading to discontinuation of the clinical trial drug, or adverse events and adverse drug reactions leading to death. All adverse drug reactions (PT) that occurred in the clinical trial except for 'Lethargy,' and 'Ocular discomfort' were adverse events previously reported for the test drug enavogliflozin and similar drugs. Among them, 'Ocular discomfort' was an adverse event that occurred in the control, not the test group. In other safety evaluation items, all adverse events reported due to abnormalities in the laboratory test, vital signs, and electrocardiogram test results were confirmed to have no causal relationship with the clinical trial drug. In addition, some statistically significant changes were observed in the laboratory test, vital signs, and physical examination, but none was clinically significant. There were no other clinically significant fundings. When summarizing the safety evaluation results, no special safety-related issues were found in the test group compared to the control.

Based on the above, the efficacy and safety of enavogliflozin were confirmed in patients with type 2 diabetes whose glycemic control was insufficient with metformin and gemigliptin, and enavogliflozin was judged to be an effective therapeutic agent for diabetes.

### Example 4: Analysis of nephropathy patient subgroups in Examples 1 to 3

Among the patient groups that received the test drugs and the control drug of Examples 1 to 3, a group of patients with proteinuria (baseline UACR of 30 to 3500 g/kg) was additionally analyzed.

Amng the patients in Example 1, a subgroup with baseline UACR of 30 to 3500 g/kg was classified as a group of patients with proteinuria.

**[Table 63]**

| **Proportion of subjects UACR at baseline (full analysis set)** | | | |
|---|---|---|---|
| | **Enavogliflozin 0.3 mg** | **Placebo** | **Total** |
| | **(N**=**82)** | **(N=79)** | **(N=161**) |
| **UACR baseline characteristics, n(%)** | 82 | 79 | 161 |
| Normoalbuminuria (<30) | 58 (82.93) | 53 (79.75) | 131 (81.37) |
| Microalbuminuria (30<=, <=300) | 12 (14.63) | 14 (17.72) | 26 (16.15) |
| Macroalbuminuria (300<, <=3500) | 2 (2.44) | 2 (2.53) | 4 (2.48) |
| P-value [1] | | | J.8747 (f) |

| | | | |
|---|---|---|---|
| Note: The denominator of the percentages is the number of subjects in each group. [1] Testing for difference among treatment groups (chi-square test (c) or Fisher's exact test (f)). | | | |

In Example 1, the change in UACR at week 24 according to administration of the test drug and the control drug (placebo) in the patient group with proteinuria (baseline UACR of 30 to 3500 g/kg) is as follows.

**[Table 64]**

| **Percent change from baseline in UACR at week 24 (full analysis set) [UACR 30-3500 g/kg at baseline]** | | |
|---|---|---|
| | **Enavogliflozin 0.3 mg** | **Placebo** |
| | **(N=14)** | **(N=16)** |
| **Baseline** | | |
| n | 14 | 16 |
| Mean (SD) | 118.76 (136.45) | 222.13 (387.39) |
| Median | 55.05 | 54.15 |
| Min, Max | 32.90, 472.80 | 32.20, 1274.70 |

| **Week 24** | | |
|---|---|---|
| n | 14 | 16 |
| Mean (SD) | 70.29 (101.88) | 257.80 (594.37) |
| Median | 26.05 | 31.00 |
| Min, Max | 2.10, 384.80 | 8.80, 2288.80 |

| **Percent change from baseline at week 24** | | |
|---|---|---|
| n | 14 | 16 |
| Mean (SD) | -46.06 (38.60) | 4.83 (130.49) |
| Median | -64.48 | -23.35 |
| Min, Max | -94.55, 48.13 | -96.36, 466.77 |
| P-value [1] | J.0017 (w) | 0.1439 (w) |
| Difference (enavogliflozin 0.3 mg - Placebo) | -50.89 | |
| P-value [2] | J.1095 (w) | |

| | | |
|---|---|---|
| SD = standard deviation, Min = minimum, Max = maximum. [1] Testing or change within-treatment groups (paired t-test (t) or Wilcoxon signed rank test (w)) [2] Testing for percent change between DWP16001 and Placebo (two sample t-test (t) or Wilcoxon rank sum test (w)). | | |

Among the patients in Example 2, a subgroup with the baseline UACR of 30 to 3500 g/kg was classified as a group of patients with proteinuria.

**[Table 65]**

| **Proportion of subjects UACR at baseline (per-protocol set)** | | | |
|---|---|---|---|
| | **Enavogliflozin 0.3 mg** | **Dapagliflozin 10 mg** | **Total** |
| | **(N=95)** | **(N=90)** | **(N=185)** |
| **UACR baseline characteristics, n(%)** | 95 | 89 | 184 |
| Normoalbuminuria (<30) | 77 (81.05) | 57 (75.28) | 144 (78.26) |
| Microalbuminuria (30<=, <=300) | 17 (17.89) | 19 (21.35) | 36 (19.57) |
| Macroalbuminuria (300<, <=3500) | 1 (1.05) | 3 (3.37) | 4 (2.17) |
| P-value [1] | | | 0.4778 (f) |
| Note: The denominator of the percentages is the number of subjects in each group. | | | |
| [1] Testing for difference among treatment groups (chi-square test (c) or Fisher's exact test (f)). | | | |

In Example 2, the change in UACR at week 24 according to administration of the test drug and the control drug (dapagliflozin) in the patient group with proteinuria (baseline UACR of 30 to 3500 g/kg) is as follows.

**[Table 66]**

| **Percent change from baseline in UACR at week 24 (per-protocol set) [UACR 30-3500 g/kg at baseline]** | | |
|---|---|---|
| | **Enavogliflozin 0.3 mg** | **Dapagliflozin 10 mg** |
| | **(N=18)** | **(N=22)** |
| **Baseline** | | |
| n | 18 | 22 |
| Mean (SD) | 153.59 (362.03) | 193.97 (373.15) |
| Median | 66.00 | 55.20 |
| Min, Max | 30.60, 1600.00 | 30.90, 1474.70 |

| **Week 24** | | |
|---|---|---|
| n | 18 | 22 |
| Mean (SD) | 45.64 (46.71) | 93.37 (139.39) |
| Median | 27.65 | 45.70 |
| Min, Max | 4.70, 207.40 | 8.30, 650.70 |

| **Percent change from baseline at week 24** | | |
|---|---|---|
| n | 18 | 22 |
| Mean (SD) | -47.35 (33.42) | -26.01 (41.52) |
| Median | -48.74 | -39.48 |
| Min, Max | -92.46, 36.98 | -76.44, 68.07 |
| P-value [1] | <.0001 (t) | 0.0074 (w) |
| Difference (enavogliflozin 0.3 mg - dapagliflozin 10 mg) | -21.33 | |
| P-value [2] | 0.1261 (w) | |
| SD = standard deviation, Min = minimum, Max = maximum, NC = not calculated. | | |
| [1] Testing for change within-treatment groups (paired t-test (t) or Wilcoxon signed rank test (w)). | | |
| [2] Testing for percent change between DWP16001 and dapagliflozin (two sample t-test (t) or Wilcoxon rank sum test (w)). | | |

Similarly, among the patients in Example 3, a subgroup with the baseline UACR of 30 to 3500 g/kg was classified as a group of patients with proteinuria.

**[Table 67]**

| **Proportion of subjects UACR at baseline (per-protocol set)** | | | |
|---|---|---|---|
| | **Enavogliflozin 0.3 mg** | **Dapagliflozin 10 mg** | **Total** |
| | **(N=119)** | **(N=123)** | **(N=242)** |
| **UACR Baseline Characteristics, n(%)** | 119 | 123 | 242 |
| Normoalbuminuria (<30) | 89(74.79) | 101(82.11) | 190(78.51) |
| Microalbuminuria (30<=, <=300) | 27(22.69) | 19(15.45) | 46(19.01) |
| Macroalbuminuria (300<, <=3500) | 3(2.52) | 3(2.44) | 6(2.48) |
| P-value [1] | | | 0.3767 (f) |
| Note: The denominator of the percentages is the number of subjects in each group. | | | |
| [1] Testing for difference among treatment groups (chi-square test (c) or Fisher's exact test (f)). | | | |

InExample 3, the change in UACR at week 24 according to administration of the test drug and the control drug (dapagliflozin) in the patient group with proteinuria (baseline UACR of 30 to 3500 g/kg) is as follows.

**[Table 68]**

| **Percent change from baseline in UACR at week 24 (per-protocol set) [UACR 30-3500 g/kg baseline]** | | |
|---|---|---|
| | **Enavogliflozin 0.3 mg** | **Dapagliflozin 10 mg** |
| | **(N=30)** | **(N=22)** |
| **Baseline** | | |
| n | 30 | 22 |
| Mean (SD) | 151.87(229.25) | 144.92(198.12) |
| Median | 77.70 | 59.80 |
| Min, Max | 32.40, 1262.90 | 32.10, 816.40 |

| **Week 24** | | |
|---|---|---|
| n | 30 | 22 |
| Mean (SD) | 75.40(90.58) | 80.60(92.63) |
| Median | 46.80 | 43.10 |
| Min, Max | 6.70, 432.40 | 16.40, 416.80 |

| **Percent change from baseline at week 24** | | |
|---|---|---|
| n | 30 | 22 |
| Mean (SD) | -36.03(51.50) | -13.09(105.67) |
| Median | -55.97 | -39.84 |
| Min, Max | -94.58, 116.67 | -85.19, 430.43 |
| P-value [1] | J.0006 (w) | 0.0094 (w) |
| Difference (enavogliflozin 0.3 mg - dapagliflozin 10 mg) | -22.94 | |
| P-value [2] | J.3040 (w) | |
| SD = standard deviation, Min = minimum, Max = maximum. | | |
| [1] Testing for change within-treatment groups (paired t-test (t) or Wilcoxon signed rank test (w)). | | |
| [2] Testing for percent change between DWP16001 and dapagliflozin (two sample t-test (t) or Wilcoxon rank sum test (w)). | | |

Taken together, as shown in FIG. 4, as a result of confirming the changes in renal function-related indicators detected by UACR at week 24, when enavogliflozin was administered alone, UACR at week 24 decreased by 46.06% compared to baseline, and also in dual combination therapy with metformin and triple combination therapy with metformin and gemigliptin, the UACR changes at week 24 decreased by 47.35% and 36.03% on average, respectively, compared to the baseline.

In the dual combination therapy with metformin and the triple combination therapy with metformin and gemigliptin, the UACR reduction in the enavogliflozin-administered patient group less than that of the dapagliflozin-administered patient group, which is a type of SGLT2 inhibitor, was used as a control.

In addition, when the two studies were combined and analyzed, it was confirmed that the UACR change in the enavogliflozin-administered patient group decreased by 20.72% compared to that of the dapagliflozin-administered patient group.

### Example 5: Renal function-related safety

In phase 3 clinical trials of Examples 1 to 3, the changes in eGFR as an indicator of renal function-related safety were confirmed. As a result, as seen from the following table, no significant change in eGFR caused by the administration of the test drug was found, confirming the safety of the test drug.

**[Table 69]**

| | Example 1 (monotherapy) | | Example 2 (combination therapy with metformin) | | Example 3 (combination therapy with metformin/gemigliptin) | |
|---|---|---|---|---|---|---|
| | Enavogliflozin 0.3 mg (N=83) | Placebo (N=84) | Enavogliflozin 0.3 mg (N=101) | Dapagliflozin (N=99) | Enavogliflozin 0.3 mg (N=134) | Dapagliflozin (N=136) |
| Baseline | 89.092 | 88.495 | 88.149 | 94.002 | 91.4522 | 91.7015 |
| Week 6 | 88.464 | 89.574 | 88.574 | 92.123 | 89.3847 | 90.5384 |
| **Change from baseline at week 6** | **-0.845** | **-0.077** | **0.425** | **-1.908** | **-1.9291** | **-1.1439** |
| Week 12 | 90.142 | 89.081 | 90.566 | 92.274 | 92.0995 | 89.8198 |
| **Change from baseline at week 12** | **1.208** | **0.223** | **2.488** | **-1.559** | **0.9308** | **-1.7177** |
| Week 18 | 91.088 | 90.214 | 88.185 | 94.828 | 89.9269 | 90.9546 |
| **Change from baseline at week** | **2.072** | **1.356** | **0.105** | **0.897** | **-1.3048** | **0.0571** |
| **18** | | | | | | |
| Week 24 | 91.387 | 87.248 | 88.056 | 93.739 | 91.9246 | 91.6344 |
| **Change from baseline at week 24** | **2.371** | **-1.092** | **-0.024** | **-0.193** | **1.7726** | **0.5790** |
| P-value (within arm) | 0.1678 | 0.4864 | 0.9817 | 0.8708 | 0.6386 | 0.6287 |
| P-value (between arm) | 0.1701 | | 0.9150 | | 0.9905 | |

### Example 6: Phase 1 clinical trial in patients with type 2 diabetes, whose renal function was impaired

A phase 1 clinical trial was additionally performed to confirm the diabetic nephropathy treatment effect of enavogliflozin.

The subjects were divided into the following four groups according to eGFR values, and urinary glucose excretion (UGE) and eGFR were estimated according to the administration of 0.5 mg of enavogliflozin as the test drug (stages 1 and 3: repeated administration (1 week), stages 2 and 4: single administration).
Stage 1: eGFR≥90 (n=6)
Stage 2: 60≤eGFR<90 (n=7)
Stage 3: 30≤eGFR<60 (n=4)
Stage 4: 15≤eGFR<30 (n=5)

As a result of confirming the urinary glucose excretion (UGE) at weeks 1 and 7 after the administration of the test drug, as shown in FIG. 5, it was confirmed that the urinary glucose excretion increased after the administration of test drug in all renal function groups, but the efficacy tended to decrease in the group with poor renal function.

In addition, as a result of confirming the changes in eGFR with the administration of the test drug, as shown in FIG. 6, eGFR reduction was confirmed in the patient group with 30≤eGFR<60, who tended to recover eGFR with the discontinuation of medication.

Therefore, the therapeutic effect of enavogliflozin on diabetic nephropathy can be confirmed.

### Example 7: Nephropathy-related non-clinical trials

Additional non-clinical trials were conducted to confirm the nephropathy-related effects of enavogliflozin.

SD rats treated with streptozotocin exhibited hyperglycemia, renal dysfunction (proteinuria), and morphologic abnormalities (glomerular mesangial dilatation). Accordingly, 60 mg/kg of streptozotocin was administered intravenously to the SD rats and used as an animal model of diabetic nephropathy.

As shown in Table 70, the SD rats were divided into 8 groups and orally administered the test material once a day for 12 weeks.

**[Table 70]**

| **Group** | **Test material** | **Dose** | **Administration route** | **No. of administration subjects** | **No. of subjects** | **Note** |
|---|---|---|---|---|---|---|
| **G1** | Sham | N/A | PO | QD | 9 | |
| **G2** | Vehicle | N/A | PO | QD | 9 | |
| **G3** | Enavogliflozin | 0.03 | PO | QD | 10 | Corresponding to standard dose of 0.3 mg |
| **G4** | Enavogliflozin | 0.3 | PO | QD | 8 | |
| **G5** | Enavogliflozin | 1 | PO | QD | 9 | |
| **G6** | Dapagliflozin | 1 | PO | QD | 9 | Corresponding to standard dose of 10 mg (Approved dose for CKD indication) |
| **G7** | Empagliflozin | 1 | PO | QD | 10 | Corresponding to standard dose of 10 mg (Permitted dose: 10 mg, 25 mg) |
| **G8** | Losartan | 30 | PO | QD | 9 | |

| | | | | | | |
|---|---|---|---|---|---|---|
| ① **G5:** The difference in efficacy can be dealt with by administering the same doses of Dapa (G6) and Empa (G7) ② **G6:** When converted to human dose, it is equivalent to approximately 10 mg, which is the allowable dose for CKD ③ **G7:** When converted to human dose, it is equivalent to approximately 10 mg, which is the allowable dose for diabetes ④ **G8:** Losartan has been shown to reduce proteinuria in patients with diabetic nephropathy and has indications (ARBs and ACEi were used as first-line therapies.) | | | | | | |

As a result of the experiment, as shown in FIG. 7, when the concentration of urinary albumin was measured at weeks 4, 8, and 12 after administration, a dose-dependent decrease in proteinuria was observed in the enavogliflozin-administered group. In addition, when comparing enavogliflozin with the same dose groups (1 mg/kg) of different SGLT2 inhibitors, enavogliflozin showed the most excellent effect in reducing proteinuria.

At the administration at week 12, the GS score used as a measure of glomerulosclerosis and the TA score used as a measure of tubular atrophy were measured. As a result, as shown in Table 8, in all enavogliflozin-administered groups, a trend toward a decrease in the GS score (significant decrease in the 0.3 mg/kg administration group) and a significant dose-dependent decrease in tubular atrophy were observed.

The fact that enavogliflozin has an effect of improving glomerular sclerosis that has not been found with other drugs in the SGLT2 inhibitor class confirms the excellent pharmacological effect of enavogliflozin on nephropathy. Enavogliflozin has also been shown to improve tubular atrophy, demonstrating that it has an excellent effect in preventing and treating nephropathy at relatively low doses compared to other drugs.

## Claims

1. A pharmaceutical composition for use in preventing or treating nephropathy, comprising enavogliflozin.

2. The pharmaceutical composition for use according to claim 1, wherein the nephropathy includes a disease selected from diabetic nephropathy, renal failure, chronic kidney disease, glomerulonephritis, and proteinuria.

3. The pharmaceutical composition for use according to claim 1, which is for reducing the risk of sustained decline in estimated glomerular filtration rate (eGFR), progression to end-stage kidney disease, cardiovascular death, and kidney-related death in patients with chronic kidney disease.

4. The pharmaceutical composition for use according to claim 1, wherein the nephropathy is diabetic nephropathy.

5. The pharmaceutical composition for use according to claim 1, which is for administration to a patient with diabetes or a pre-diabetic patient.

6. The pharmaceutical composition for use according to any one of claims 1 to 5, which is for monotherapy.

7. The pharmaceutical composition for use according to any one of claims 1 to 5, which is for combination therapy with metformin.

8. The pharmaceutical composition for use according to any one of claims 1 to 5, which is for combination therapy with a DPP4 inhibitor.

9. The pharmaceutical composition for use according to claim 8, wherein the DPP4 inhibitor is gemigliptin.

10. The pharmaceutical composition for use according to any one of claims 1 to 5, which is for combination therapy with metformin and a DPP4 inhibitor.

11. The pharmaceutical composition for use according to any one of claims 1 to 5, which is for combination therapy with sulfonylurea-based insulin secretagogue.

12. The pharmaceutical composition for use according to any one of claims 1 to 5, which is for combination therapy with insulin.

13. A pharmaceutical composition for use in preventing or treating diabetes in nephropathy patients, comprising enavogliflozin.

14. The pharmaceutical composition for use according to claim 13, wherein the nephropathy includes a disease selected from diabetic nephropathy, renal failure, chronic kidney disease, glomerulonephritis, and proteinuria.

15. The pharmaceutical composition for use according to claim 13, wherein the nephropathy is diabetic nephropathy.

16. The pharmaceutical composition for use according to any of claims 13 to 15, which is for monotherapy.

17. The pharmaceutical composition for use according to any of claims 13 to 15, which is for combination therapy with metformin.

18. The pharmaceutical composition for use according to any of claims 13 to 15, which is for combination therapy with a DPP4 inhibitor.

19. The pharmaceutical composition for use according to claim 18, wherein the DPP4 inhibitor is gemigliptin.

20. The pharmaceutical composition for use according to any of claims 13 to 15, which is for combination therapy with metformin and a DPP4 inhibitor.

21. The pharmaceutical composition for use according to any of claims 13 to 15, which is for combination therapy with sulfonylurea-based insulin secretagogue.

22. The pharmaceutical composition for use according to any of claims 13 to 15, which is for combination therapy with insulin.
